(19) Europäisches Patentamt
European Patent Office
Office européen des brevets

(11) **EP 2 370 595 B1**

(12) **EUROPEAN PATENT SPECIFICATION**

(45) Date of publication and mention
of the grant of the patent:
**26.02.2014 Bulletin 2014/09**

(51) Int Cl.:
**C12Q 1/68** (2006.01)

(21) Application number: **09768497.1**

(22) Date of filing: **01.12.2009**

(86) International application number:
**PCT/EP2009/008563**

(87) International publication number:
**WO 2010/063454 (10.06.2010 Gazette 2010/23)**

(54) **METHOD OF STRATIFYING BREAST CANCER PATIENTS BASED ON GENE EXPRESSION**

VERFAHREN ZUR STRATIFIZIERUNG VON BRUSTKREBSPATIENTEN AUF DER GRUNDLAGE VON GENEXPRESSION

PROCÉDÉ DE STRATIFICATION DE PATIENTES ATTEINTES DE CANCER DU SEIN BASÉ SUR L EXPRESSION GÉNIQUE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **01.12.2008 GB 0821787**

(43) Date of publication of application:
**05.10.2011 Bulletin 2011/40**

(73) Proprietor: **University Of Ulster**
**County Londonderry BT52 1SA (GB)**

(72) Inventors:
• **BJOURSON, Anthony**
**Strabane,**
**County Tyrone BT82 9DR (GB)**
• **BERRAR, Daniel**
**Tokyo 162-0851 (JP)**
• **McERLEAN, Seona, Bernadine**
**Toombridge,**
**County Antrim BT41 3SG (GB)**

(74) Representative: **Kelly, Donal Morgan et al**
**FRKelly**
**27 Clyde Road**
**Dublin 4 (IE)**

(56) References cited:
EP-A1- 1 961 825     EP-A2- 1 471 154
WO-A1-2006/016110     WO-A2-2004/065583
WO-A2-2005/033699     WO-A2-2006/015312
WO-A2-2008/048193     WO-A2-2008/094678
US-A1- 2003 190 656     US-A1- 2005 100 933

• **KELTOUMA DRIOUCH ET AL: "Gene arrays for diagnosis, prognosis and treatment of breast cancer metastasis" CLINICAL & EXPERIMENTAL METASTASIS ; OFFICIAL JOURNAL OF THEMETASTASIS RESEARCH SOCIETY, KLUWER ACADEMIC PUBLISHERS, DO, vol. 24, no. 8, 1 November 2007 (2007-11-01), pages 575-585, XP019550026 ISSN: 1573-7276**
• **MARTIN LAUSS ET AL: "Consensus genes of the literature to predict breast cancer recurrence" BREAST CANCER RESEARCH AND TREATMENT, KLUWER ACADEMIC PUBLISHERS, BO, vol. 110, no. 2, 26 September 2007 (2007-09-26), pages 235-244, XP019600682 ISSN: 1573-7217**
• **HAIBE-KAINS BENJAMIN ET AL: "Comparison of prognostic gene expression signatures for breast cancer." BMC GENOMICS 2008, vol. 9, 21 August 2008 (2008-08-21), page 394, XP002567159 ISSN: 1471-2164**
• **NUYTEN DIMITRY S A ET AL: "Combining biological gene expression signatures in predicting outcome in breast cancer: An alternative to supervised classification" EUROPEAN JOURNAL OF CANCER (OXFORD, ENGLAND : 1990) OCT 2008,, vol. 44, no. 15, 18 August 2008 (2008-08-18), pages 2319-2329, XP002565260**
• **XU LEI ET AL: "Merging microarray data from separate breast cancer studies provides a robust prognostic test" BMC BIOINFORMATICS, BIOMED CENTRAL, LONDON, GB, vol. 9, no. 1, 27 February 2008 (2008-02-27), page 125, XP021031690 ISSN: 1471-2105**

**(Cont. next page)**

- **NUYTEN DIMITRY S A ET AL: "Gene expression signatures to predict the development of metastasis in breast cancer" BREAST DISEASE, ELSEVIER SCIENCE PUBLISHING, NEW YORK, NY, US, vol. 26, 1 January 2006 (2006-01-01), pages 149-156, XP009095049 ISSN: 0888-6008**

**Description**

**Introduction**

Despite significant advances in the treatment of breast cancer, the ability to predict the invasive behaviour of tumours remains a significant challenge in clinical oncology. Prognostic assessment for early breast cancer is currently primarily based on clinical and histological parameters, which at present include four biomarkers: estrogen receptor (ER), progesterone receptor (PR) human epidermal growth factor receptor 2 (HER2); and urokinase plasminogen activator (uPA). Also recommended for use by the American Society of Clinical Oncology is the Oncotype DX® assay (by Genomic Health). [Harris et al. American Society of Clinical Oncology 2007 update of recommendations for the use of tumor markers in breast cancer. J. Clin. Oncol. 2007;25:5287-310; Simon R. (2008) The use of genomics in clinical trial design. Clin Cancer Res. 14(19):5984-93.].

Of the conventional prognostic factors, nodal status is consistently held to be the most important parameter for determining prognosis. Other clinical markers can include the age of a patient, tumour size, and number of involved lymph nodes at the time of surgery. However, these clinical and pathological criteria are less than precise for risk group stratification, leading to inconsistency in the results. Hence, a more robust prognostic criterion is needed.

Breast cancer is the most common female malignancy, and similar to other types of malignancy, has an important genetic contribution. The multi-step model of carcinogenesis indicates that breast cancer develops via a series of intermediate hyperplastic lesions, through *in situ,* to invasive carcinoma. However, mutations in genes commonly associated with breast cancer, such as *BRCA1* and *BRCA2*, account for only a small proportion of this hereditary component, suggesting that there exists an important role for other genetic markers, which are as yet undefined. However, the use of any one single genetic marker is in itself limited and does not reflect the multi-step genetic basis of carcinogenesis. In some cases, a point deletion or a duplication of one or several exons in a gene results in large segments of the gene being rearranged. As such, classical methods for detecting mutations, such as nucleotide sequencing, are unable to reveal these types of mutation. Furthermore, classical techniques do not lend themselves to genome-wide or multi-marker analysis, being both time- and financially- consuming, in these situations. Given the complexity of breast cancer prognosis, a more practical strategy is to utilise high-throughput technologies to evaluate a plurality of genetic markers that may contain complementary information. This may lead to a more economical and accurate prognostic system.

Molecular genomic techniques have provided the potential to significantly progress the ability to diagnose disease and classify prognosis. Microarrays provide for the analysis of large amounts of genetic information, thereby providing a genome-wide genetic fingerprint of a patient. Identifying a gene signature using microarray data for breast cancer prognosis has been a central goal in some recent large-scale exploratory studies, which have shown that gene profiling can achieve a much higher specificity than the current clinical systems (50% versus 10%) at the same sensitivity level. Pharmacogenetic techniques can be considered either prognostic or predictive. A prognostic signature is used for classification of tumour subtypes or for risk group stratification. The van't Veer 70-gene signature is such a signature. A predictive signature or a predictive genomic classifier can also find utility as a model for predicting the response to chemotherapy. For example, Hess *et al.* (2006) developed a 30-probe set classifier for the prediction of response to paclitaxel and FAC (fluorouracil, doxorubicin, and cyclophosphamide) in breast cancer patients. [Hess K.R., Anderson K., Symmans W.F., et al. (2006) Pharmacogenomic predictor of sensitivity to preoperative chemotherapy with paclitaxel and fluorouracil, doxorubicin, and cyclophosphamide in breast cancer. J Clin. Oncology 24(26):4236-44.].

However, the ability to assemble the correct information needed to adequately characterise and predict clinical outcome has somewhat hampered the widespread use of genomic-based approaches. The key challenge to deriving a successful prognostic signature from genetic markers is selection of a candidate gene set. A major problem with current gene sets is that they are typically based on broad-ranging biological information. A significant problem with this approach is that the usefulness of a gene set is limited by how representative it is of the particular diseased tissue. For example, if a particular gene set is derived from a single cellular state, the gene set as a whole reveals information relating to that particular state only. Ultimately, each gene in the set relates directly to that particular characteristic only, and so the benefit of utilising a plurality of markers is hampered by all of the markers representing the same single characteristic.

So far, gene expression profiling based on DNA microarrays has revealed sets of genes for the prediction of clinical outcome, but these gene sets are largely non-overlapping, and often contain genes that are involved in broad biological processes, and are not particularly prominent in invasion- and metastasis-related pathways. To our knowledge, only one gene signature has been reported for which each gene has been shown to be functionally linked to metastasis to the lung [Minn, A. J., et al. Genes that mediate breast cancer metastasis to lung. Nature 436(7050):518-524 (2005)]. Here we show a signature of genes that are all functionally linked to invasion and metastases of breast cancer, and of significant prognostic relevance for predicting the clinical outcome of breast cancer patients.

The invention disclosed in European Patent Publication No 1961825 (EP1961825) relates to the prognosis of the progression of breast cancer in a patient and to the prediction of the occurrence of metastasis in one or more tissue or organ of patients affected with a breast cancer. EP1961825 discloses breast cancer metastasis prediction methods comprising a step of determining, in a tumour tissue sample previously collected from a breast cancer patient to be tested the level of expression of one or more biological markers that are indicative of cancer progression towards metastasis, and more precisely, one or more biological markers that are indicative of cancer progression towards metastasis to specific tissues, and especially to bones, brain, lungs and liver.

It is an object of the present invention to assist in prospectively predicting the metastatic likelihood, and thereby, the likely clinical outcome of breast cancer patients, based on the genotype of the patient, in particular, by determining the relative expression level of a set of genes, or subsets thereof.

**Summary of the Invention**

According to a first aspect of the present invention there is provided the use of an expression level of a gene set for the identification of animals, optionally patients, likely to progress to an invasive phenotype, the gene set consisting of the genes selected from SET A.

SET A consists of the genes ABCA1, ADD3, ADFP, ADM, ALDH1A3, AQP3, ARHGAP26, B2M, BAT2D1, BIRC3, BRWD1, C18ORF1, CBLB, CD44, CHKB, CHPT1, CMKOR1, CXCL12, DBN1, EEF1A2, FAS, FLJ11000, FLJ11286, FLRT3, HLA-A, HLA-B, HLA-C, HLA-DMA, HLA-DRB1, HLA-DRB4, HLA-DRB5, HLA-F, HLA-G, HNRPD, IFITM1, IFITM3, INHBB, ISG20, JAG1, JAG2, KITLG, LAMC1, LAP3, LGALS3BP, MYO1B, NME4, PLCB1, PRLR, PSMB9, PXN, RAB14, SEMA3C, SEPP1, SLC6A8, SP100, SP110, STS, TAP1, TMEPAI, TNFSF10, TRAM1, TRIM14, and WSB1.

Preferably, the gene set consists of, all of the genes comprising SET A.

As used herein, the term "patient" is usually intended to refer to human patients.

By the term "expression level" is meant a value representative of the expression of a gene. It is to be appreciated that the value can be representative of at least one functional product of the gene, including but not limited to, evaluating the abundance of RNA transcripts transcribed from the gene, evaluating the abundance of polypeptides translated from said RNA transcripts, or a combination thereof. Evaluation can involve qualitative analysis such as presence or absence of a functional product of the gene, or quantitative analysis such as the measure of the amount of a functional product of the gene. The analysis techniques for evaluation can be those commonly used, and can be selected by one skilled in the art.

A diverse range of protein detection and identification methods are available and can generally be divided into chemical/biological and physical methods. Physical methods can include methods based on, for example, spectroscopy-based techniques that involve light absorption at specific wavelengths, or multidimensional coherent infrared spectroscopic techniques. Alternatively, a diversity of mass spectrometry methods based on mass determination of peptides and their fragments can be used to detect, identify or quantify specific proteins. Chemical/biological methods that are widely used include, for example, two-dimensional electrophoresis, immunological-based methods such as western blotting, immunocytochemistry, ELISA, protein arrays and a diversity of variations of such methods. The proteins encoded by the genes represented by the transcripts of the present invention could be detected by some or all of the methods, or combinations or variations thereof.

It is also understood that the level of gene expression may be altered at the nucleic acid level or protein level, or may be subject to alternative splicing to result in a different polypeptide product. Such differences may be evidenced by a change in mRNA levels, surface expression, secretion or other partitioning of a polypeptide, for example.

According to a second aspect of the present invention there is provided a method of stratifying animals, optionally patients, further optionally human patients, into cohorts, the method comprising the steps of determining an expression level of the genes selected from SET A, identifying animals, optionally patients, further optionally human patients, likely to progress to an invasive phenotype based on the gene expression level of the genes selected from SET A, and stratifying animals, optionally patients, further optionally human patients, into cohorts based on the likelihood to progress to the invasive phenotype.

Optionally, the determining step further comprises the step of comparing the expression level of each gene to a normal control. The comparison of the expression level of each gene represents a deviation from the normal.

As used herein, the term "normal" is defined as a defined expression level of a gene, the defined expression level being associated with a disease-free phenotype. It will be appreciated however that in the case of predicting prognosis in a patient suffering from a disease, the defined expression level of the gene may be associated with a defined stage of disease as opposed to a disease-free phenotype. In an embodiment of the invention, the term "normal" may be the expression level of a gene evaluated at a first time point. Optionally or additionally, the expression level of a gene may be evaluated at a second, or subsequent, time point. Further optionally or additionally, the expression level of a gene may evaluated in a series of more than two subsequent time points. Each or any of the time points may then be used, or referenced as "normal".

The expression level of each gene is used in combination with the expression level of each of the other selected genes of a set to form an expression profile. By the term "expression profile" is meant a simultaneous evaluation comprising the expression levels of all of the genes selected from a given gene set.

Optionally, SET A is divided into at least two subsets. Preferably, the first subset (SET B) comprises at least some of a gene set having an expression level in a disease setting relatively higher to the normal, herein referred to as "up regulated" or "up cassette". Further preferably, the second subset (SET C) comprises at least some of a gene set having an expression level in a disease setting relatively lower to the normal, herein referred to as "down regulated" or "down cassette".

Preferably, the first subset (SET B) comprises, optionally consists of, the genes ABCA1, ADFP, ADM, ALDH1A3, AQP3, BAT2D1, BRWD1, C18ORF1, CBLB, CMKOR1, DBN1, EEF1A2, FLRT3, HNRPD, INHBB, JAG1, JAG2, KITLG, LAMC1, MYO1B, NME4, PLCB1, PXN, SLC6A8, TMEPAI, TRAM1, and WSB1.

By "some of the genes" is meant two or more, optionally five or more, further optionally ten or more, still further optionally at least fifteen, still further optionally at least twenty, still further optionally at least twenty-five, still further optionally all twenty seven, of the genes. The some of the genes may be in any combination or permutation. Preferably, the set of genes comprises, optionally consists of, all of the genes comprising SET B.

Preferably, the second subset (SET C) comprises, optionally consists of, the genes ADD3, ARHGAP26, B2M, BIRC3, CD44, CHKB, CHPT1, CXCL12, FAS, FLJ11000, FLJ11286, HLA-A, HLA-B, HLA-C, HLA-DMA, HLA-DRB1, HLA-DRB4, HLA-DRB5, HLA-F, HLA-G, IFITM1, IFITM3, ISG20, LAP3, LGALS3BP, PRLR, PSMB9, RAB14, SEMA3C, SEPP1, SP100, SP110, STS, TAP1, TNFSF10, and TRIM14.

By "some of the genes" is meant two or more, optionally five or more, further optionally ten or more, still further optionally at least twenty, still further optionally at least thirty, still further optionally at least thirty-five, still further optionally all thirty-six, of the genes. The some of the genes may be in any combination or permutation. Preferably, the set of genes comprises, optionally consists of, all of the genes comprising SET C.

Optionally, the identifying step involves comparing the gene expression profile of at least some or all of the genes selected from the first subset, and the gene expression profile of at least some or all of the genes selected from the second subset. Preferably, the step of identifying patients likely to progress to an invasive phenotype is based on the relative difference between the average expression value of at least some or all of the genes selected from the first subset, and the average expression value of at least some or all of the genes selected from the second subset, referred to herein as a "tandem score".

Without being bound by theory, it is thought that a patient having an average expression value of at least some or all of the genes selected from the second subset (SET C) less than an average expression value of at least some or all of the genes selected from the first subset (SET B) has a relatively bad clinical outcome because the patient's individual profile corresponds to a more aggressive phenotype.

A patient having an average expression value of at least some or all of the genes selected from the second subset (SET C) greater than an average expression value of at least some or all of the genes selected from the first subset (SET B) has a relatively better clinical outcome because the patient's individual profile corresponds to a less aggressive phenotype.

Optionally, patients are sequentially ranked in increasing order based on the value of (average down-cassette) minus (average up-cassette).

Optionally, the stratifying step involves stratifying patients into cohorts based on sequential ranking.

Optionally, patients ranked at or below the 25th percentile, optionally at or below the 20th percentile, further optionally at or below the 10th percentile, are likely to progress to the invasive phenotype. Further optionally, deviation of the expression level of at least some or all of the selected genes from a normal control is indicative of an invasive phenotype. Optionally, positive deviation of the expression level (up regulation) of at least some or all of the genes of the first subset from a normal control is indicative of an invasive phenotype. Optionally, negative deviation of the expression level (down regulation) of at least some or all of the genes of the second subset from a normal control is indicative of an invasive phenotype. Optionally, a combination of positive deviation of the expression level of at least some or all of the genes of the first subset, and negative deviation of the expression level of at least some or all of the genes of the second subset, is indicative of an invasive phenotype.

Optionally, the degree of deviation from the normal is proportional to invasiveness. Optionally, positive deviation of the expression level of more than 1-fold, optionally more than 1.5-fold, further optionally more than 2-fold, further optionally more than 3-fold, further optionally more than 4-fold, of at least some or all of the genes of the first subset from a normal control is indicative of an invasive phenotype. Optionally, negative deviation of the expression level of more than 1-fold, optionally more than 1.5-fold, further optionally more than 2-fold, further optionally more than 3-fold, further optionally more than 4-fold, of at least some or all of the genes of the second subset from a normal control is indicative of an invasive phenotype.

Preferably, the gene set is isolated from a sample from an animal, such as a patient, optionally a human patient.

Preferably, the sample is a fresh tissue sample, such as a fresh tumour tissue sample, optionally a fresh breast tumour tissue sample. Optionally, the sample is a paraffin-embedded tissue sample, such as a paraffin-embedded tumour tissue sample, optionally a paraffin-embedded breast tumour tissue sample. Further optionally, the sample is a frozen tissue sample, such as a frozen tumour tissue sample, optionally a frozen breast at least tissue sample.

Preferably, the expression level of a gene is determined by quantifying a functional RNA transcript.

Preferably, the expression level of each gene is normalised against the quantitative level of all RNA transcripts in the sample.

Optionally, the expression level of each gene is determined using polynucleotides having a nucleic acid sequence capable of hybidising to at least some or all of the nucleic acid sequences selected from SET D, and complementary sequences thereof. Preferably, the polynucleotide is a polyribonucleotide.

What is meant by the term "polynucleotide" is any polyribonucleotide or polydeoxyribonucleotide, which may be unmodified RNA or DNA or modified RNA or DNA, and is intended to include single-and double-stranded DNA, DNA including single-and double- stranded regions, single- and double-stranded RNA, RNA including single-and double-stranded regions, hybrid molecules comprising DNA and RNA that may be single-stranded or, more typically, double-stranded or include single-and double-stranded regions.

SET D consists of the probe sets disclosed in Tables 10 and 11 herein. Optionally, SET D is divided into at least two subsets. Preferably, the first subset (SET E) comprises at least some of the probe sets disclosed in Table 11 and are capable of hybridizing to the respective genes, or complementary sequences thereof, selected from SET B. Further optionally, the second subset (SET F) comprises at least some of the probe sets disclosed in Table 10 and are capable of hybridizing to the respective genes, or complementary sequences thereof, selected from SET C.

By the term "hybridisation" is meant the process of combining complementary single-stranded nucleic acid molecules to form a single double-stranded nucleic acid molecule. It is understood that not all nucleic acids of the single-stranded molecule must be individually combined with a complementary nucleic acid of the complementary single-stranded nucleic acid molecule in order for the double-stranded nucleic acid molecule to be formed. The combination may be achieved through the formation of at least one hydrogen bond between complementary nucleic acids of each of the single-stranded nucleic acid molecules. The term "hybridization" is intended to be used synonymously with the term "annealing".

The conditions for hybridization can be dependent on the specific techniques used to permit annealing of the complementary single-stranded nucleic acid molecules, and may differ depending on the properties of the individual complementary single-stranded nucleic acid molecules, as will be known to those skilled in the art. The conditions for hybridisation, such as salt concentration, temperature, pH, and period of time, are each dependent on the properties of the individual complementary single-stranded nucleic acid molecules, and can each be independently selected by one skilled in the art.

Preferably, the temperature for hybridization is lower than the temperature at which a single double-stranded nucleic acid molecule separates into complementary single-stranded nucleic acid molecules. Optionally, the temperature for hybridization is from about 16°C to about 32°C lower than the temperature at which a single double-stranded nucleic acid molecule separates into complementary single-stranded nucleic acid molecules. The temperature for hybridization can be dependent on the presence of organic solvent, salt concentration, and can be selected by one skilled in the art.

By "some of the nucleic acid sequences" is meant two or more, optionally ten or more, further optionally twenty or more, still further optionally at least forty, still further optionally at least fifty, still further optionally at least sixty, still further optionally at least seventy, still further optionally at least eighty, still further optionally all eighty-seven, of the nucleic acid sequences. The some of the nucleic acid sequences may be in any combination or permutation. Preferably, the set of genes comprises, optionally consists of, all of the probe sets comprising SET D.

The first subset (SET E) can comprise, optionally consist of, the probe sets selected from Probe IDs: 204540_at: 207996_s_at; 202806_at; 202912_at; 211823_s_at; 219250_s_at; 202219_at; 203180_at; 209682_at; 212977_at; 205258_at; 209099_x_at; 216268_s_at; 200771_at; 201398_s_at; 201294_s_at; 209122_at; 211946_s_at; 214820_at; 217025_s_at; 32137_at; 212364_at; 210854_x_at; 212739_s_at; 203505_at; 39248_at; 221480_at; 213222_at; 201296_s_at; 211944_at; 207029_at; and 217875_s_at.

The second subset (SET F) can consist of the probe sets selected from Probe IDs: 217478_s_at; 208306_x_at; 215193_x_at; 204670_x_at; 209312_x_at; 209687_at; 218999_at; 204490_s_at; 209835_x_at; 212014_x_at; 212063_at; 203666_at; 204780_s_at; 216231_s_at; 214459_x_at; 203768_s_at; 221491_x_at; 202687_s_at; 202688_at; 204781_s_at; 216252_x_at; 211799_x_at; 221675_s_at; 211911_x_at; 208812_x_at; 211528_x_at; 211529_x_at; 214022_s_at; 217933_s_at; 206346_at; 209761_s_at; 210070_s_at; 218429_s_at; 215313_x_at; 204806_x_at; 212203_x_at; 201752_s_at; 210538_s_at; 53720_at; 216526_x_at; 221875_x_at; 33304_at; 204279_at; 201427_s_at; 208392_x_at; 203147_s_at; 205068_s_at; 217523_at; 213932_x_at; 221978_at; 200923_at; 203788_s_at; 202863_at; 202307_s_at; and 200927_s_at.

Optionally, when at least some of the nucleic acid sequences or polynucleotides are selected, the nucleic acid sequences or polynucleotides are selected based on a relative weight value. Optionally, the relative weight value is a normed score reflecting the association of the nucleic acid sequence or polynucleotide to a diseased phenotype. Preferably, the nucleic acid sequence has a relative weight value of at least 2.0, optionally at least 1.8, further optionally at least 1.6, still further optionally at least 1.4, still further optionally at least 1.2, still further optionally at least 1.0, still further optionally at least 0.8, still further optionally at least 0.6.

Optionally, the method of stratifying animals, such as patients into cohorts further comprises the step of subjecting the data obtained in the determining step to statistical analysis, in order to determine the deviation of the expression profile of the animal from the normal.

It is understood that the data are subjected to statistical analysis in order to facilitate robust interpretation of the data obtained from the determining step. The statistical analysis provides for means to retrospectively analyse the data to identify those likely to progress to an invasive phenotype, and stratify them based on the likelihood to progress to the invasive phenotype. The statistical analysis may involve any of the steps of background correction, quality control, spot filtering, aggregation and normalisation, identification of significant differential expression, pattern recognition, or a combination thereof, as will be known to those skilled in the art. Optionally, the statistical analysis steps are chosen from the guidelines of established resources such as the Microarray Quality Control project, or MicroArray and Gene Expression (MAGE) group. Although, any statistical analysis well known in the art may be employed to interpret the data.

Preferably, the patient is a mammal. More preferably, the patient is a human.

Preferably, the patient is suffering from a cancer. More preferably, the patient is suffering from breast cancer.

Preferably, the method of stratifying patients into cohorts further comprises the step of determining whether a patient is suffering from breast cancer. Accordingly, the present invention also provides a method for diagnosing a patient with breast cancer by attributing the deviation of the expression profile of a patient from the normal, to a diseased phenotype.

The term "diagnosis" is used herein to refer to the identification of a molecular or pathological state, disease or condition, such as the identification of a molecular subtype of cancer, particularly breast cancer.

Further preferably, the method of stratifying patients into cohorts further comprises the step of evaluating the invasiveness of the breast cancer. Accordingly, the present invention also provides a method for predicting prognosis of a patient with breast cancer by attributing the deviation of the expression profile of a patient from the normal, to an invasive phenotype.

The term "prognosis" is used herein to refer to the prediction of the likelihood of progression, including recurrence, metastatic spread, and drug resistance, of a neoplastic disease, such as breast cancer. For example, a patient having an expression profile, which correlates with an invasive phenotype, may exhibit a high proliferative activity, and therefore may be demonstrative of a favourable response to chemotherapy, as the invasive phenotype can be a histologic characteristic used to indicate a chemotherapy-sensitive neoplastic disease.

Accordingly, it is envisaged that the method of predicting prognosis can also be used to predict if a patient is likely to respond favourably to a treatment regimen, and can hence be used clinically to make treatment decisions by choosing the most appropriate treatment modalities for any particular patient.

Optionally, the prognosis includes prediction of the likelihood of long-term survival of the patient and/or recommendation for a treatment modality of said patient.

Optionally, the method of stratifying animals, optionally patients, into cohorts; the method for diagnosing a patient with breast cancer; or the method for predicting prognosis of a patient with breast cancer; can be used in combination with other methods of prediction.

Optionally, the method of the present invention can be used in combination with each or some of the 70-gene predictor, the wound-response signature, the NIH risk and the St. Gallen criteria, as described herein.

According to a third aspect of the present invention there is provided an array for expression profiling, the array comprising polynucleotides, and complimentary sequences thereof, that specifically hybridise to all, of the genes selected from SET A.

SET A consists of, the genes ABCA1, ADD3, ADFP, ADM, ALDH1A3, AQP3, ARHGAP26, B2M, BAT2D1, BIRC3, BRWD1, C18ORF1, CBLB, CD44, CHKB, CHPT1, CMKOR1, CXCL12, DBN1, EEF1A2, FAS, FLJ11000, FLJ11286, FLRT3, HLA-A, HLA-B, HLA-C, HLA-DMA, HLA-DRB1, HLA-DRB4, HLA-DRB5, HLA-F, HLA-G, HNRPD, IFITM1, IFITM3, INHBB, ISG20, JAG1, JAG2, KITLG, LAMC1, LAP3, LGALS3BP, MYO1B, NME4, PLCB1, PRLR, PSMB9, PXN, RAB14, SEMA3C, SEPP1, SLC6A8, SP100, SP110, STS, TAP1, TMEPAI, TNFSF10, TRAM1, TRIM14, and WSB1.

Preferably, the set of genes consists of, all of the genes comprising SET A.

Optionally, the polynucleotides are selected from SET D.

SET D can consist of the probe sets selected from Probe IDs: 204540_at; 207996_s_at; 202806_at; 202912_at; 211823_s_at; 219250_s_at; 202219_at; 203180_at; 209682_at; 212977_at; 205258_at; 209099_x_at; 216268_s_at; 200771_at; 201398_s_at; 201294_s_at; 209122_at; 211946_s_at; 214820_at; 217025_s_at; 32137_at; 212364_at; 210854_x_at; 212739_s_at; 203505_at; 39248_at; 221480_at; 213222_at; 201296_s_at; 211944_at; 207029_at; 217875_s_at; 217478_s_at; 208306_x_at; 215193_x_at; 204670_x_at; 209312_x_at; 209687_at; 218999_at; 204490_s_at; 209835_x_at; 212014_x_at; 212063_at; 203666_at; 204780_s_at; 216231_s_at; 214459_x_at; 203768_s_at; 221491_x_at; 202687_s_at; 202688_at; 204781_s_at; 216252_x_at; 211799_x_at; 221675_s_at; 211911_x_at; 208812_x_at; 211528_x_at; 211529_x_at; 214022_s_at; 217933_s_at; 206346_at; 209761_s_at;

210070_s_at; 218429_s_at; 215313_x_at; 204806_x_at; 212203_x_at; 201752_s_at; 210538_s_at; 53720_at; 216526_x_at; 221875_x_at; 33304_at; 204279_at; 201427_s_at; 208392_x_at; 203147_s_at; 205068_s_at; 217523_at; 213932_x_at; 221978_at; 200923_at; 203788_s_at; 202863_at; 202307_s_at; and 200927_s_at.

By "complementary sequence" is meant a sequence having a complementary sequence to that of the sequence defined in the respective SET or subset. When the sequence defined in the SET or subset is a nucleic acid sequence, the complementary sequence may be an RNA sequence or a DNA sequence. Similarly, the complementary sequence may be an amino acid sequence encoded by the nucleic acid sequence defined in the SET or subset.

Preferably, the polynucleotides of the array are oligonucleotides. Optionally, the polynucleotides of the array are cDNAs.

Preferably, the array comprises a solid support, and polynucleotide sequences of at least two of the polynucleotides selected from SET D are attached to the support. Optionally ten or more, further optionally twenty or more, still further optionally at least forty, still further optionally at least fifty, still further optionally at least sixty, still further optionally at least seventy, still further optionally at least eighty, still further optionally at least eighty-seven, of the nucleic acid sequences selected from SET D are attached to the support.

Optionally, the array contains other biological molecules, such as polypeptides or antibodies, representative of transcripts of the array. Thus, the arrays provided herein encompass nucleic acid arrays, polypeptide arrays, or antibody arrays. For the purposes of this specification, unless the context demands otherwise, where specific embodiments are described with reference to nucleic acid arrays, it should be understood that corresponding protein arrays and antibody arrays are also contemplated. In such embodiments, the nucleic acids are replaced by polypeptides encoded by the transcripts or antibodies specific for the polypeptides encoded by the transcripts.

According to a further aspect of the invention, there is provided a kit comprising the array of the second aspect of the invention, the kit further comprising one or more of extraction buffer/reagents and protocol, reverse transcription buffer/reagents and protocol and qPCR buffer/reagents and protocol suitable for performing any of the foregoing methods.

## Brief Description of the Invention

An embodiment of the invention will now be described with reference to the accompanying drawings in which:

Figure 1A is a heatmap of tumour gene expression levels in data sets 1, 2, and 3;

Figure 1B is a graphical illustration of distant metastasis-free survival of patients with tumours for which the tandem score is at or below the 75th percentile (upper, darker plot), and patients above the 75th percentile (lower, lighter plot), compared using Kaplan-Meier analysis;

Figure 2 is a graphical illustration of the fold change of the expression values of the probe sets in the down- and up-cassette of the present invention;

Figures 3A-F are graphical illustrations of a Kaplan-Meier analysis of time to distant metastases for patients with tumours for which the tandem score is at or below the 75th percentile (upper, darker plot), and patients above the 75th percentile (lower, lighter plot) from each of data set 1 (A&B); data set 2 (C&D); and data set 3 (E&F);

Figures 4A-D are graphical illustrations of Kaplan-Meier analysis for a test set using (A) 70-gene predictor (van't Veer *et al.*, 2002); (B) Wound-response signature (Chang *et al.,* 2005); (C) NIH risk (based on age, grade, tumour size, lymphnode status, ER status, PR status, and/or intrinsic subtype); and (D) St. Gallen criteria (Goldhirsch *et al.,* 2005);

Figures 5A-C are graphical illustrations of Kaplan-Meier analysis based on the combined predictor consisting of NIH risk, St. Gallen criteria, 70-gene signature and wound-response signature (A); based on the agreement of the combined predictor and the invasiveness gene signature of the present invention (IGS) (B); for patients whom the IGS and the combined predictor do not agree (C);

Figure 6 is a schematic illustration of a Matrigel invasion chamber in which *in vitro* invasion was assessed for each cell line;

Figure 7 is a schematic illustration of a method to isolate invaded subclones from parental MCF-7 cells;

Figure 8 is a graphical representation of the invasion of parental MCF-7 cells and the 3 invaded subclones;

Figure 9 is a graphical representation of the invasion of each subclone after normalisation using the parental MCF-7 (10) cells, and the selection of hyper-invasive cells (shaded) from the primarily weakly-invasive (white) parental population;

Figure 10 is a graphical representation of wound scrape assays for MCF7-10 (0) and MCF7-I6 (■) cells in full medium and serum-free medium;

Figure 11A is a photographical comparison of the MCF7-10 and MCF7-16 cells, showing the more spindle-shaped morphology in the MCF7-16 cells;

Figure 11B is a graphical representation of mRNA expression by qRT-PCR of vimentin, E-cadherin, and N-cadherin in MCF7-10 and MCF7-16 cells;

Figure 11C is a graphical representation of adhesion of MCF7-10 and MCF7-16 cells to extracellular components - laminin, fibronectin and collagen IV - using CytoMatrix screening kit;

Figure 12 illustrates mRNA expression of interferon-induced genes by (a) semiquantitative PCR; (b) quantitative PCR; (c) Western blot analysis of interferon induced genes STAT1, IFITM1 and IRF9, and (d) Western blot analysis of STAT1 activation upon induction by 100 ng/ml IFN-gamma;

Figure 13 is a graphical representation of growth curves for MCF7-10 and MCF7-I6 cells in the presence (dotted curves) and absence (solid curves) of 100 ng/ml IFN-gamma;

Figure 14 is a flow diagram illustrating the filtering process to identify prognostic gene set (tandem signature);

Figure 15 is a heatmap of tumor gene expression levels in the learning sets (a) data set 1 and (b) data set 2;

Figure 16 is a heatmap of tumor gene expression levels in the validation sets (a) data set 3 and (b) data set 4;

Figure 17 is a heatmap of tumor gene expression levels in the validation sets (a) data set 5 and (b) data set 6; and

Figure 18 is a graphical representation of Kaplan-Meier analysis of time to event in the training sets, (a) data set 1 ($n = 286$), (b) data set 2 ($n = 125$), and the validation sets, (c) data set 3 ($n = 141$), (d) data set 4 ($n = 200$), (e) data set 5 ($n = 64$) and (f) data set 6 ($n = 125$).

## Materials and Methods

### Cell Line

MCF-7 cells were purchased from The European Collection of Cell Cultures (ECACC) and maintained at 37°C in a 5% CO2, 95% air humidified atmosphere temperature-controlled incubator (RS Biotech, Galaxy S). All cells were routinely sub-cultured every 2-3 days. MCF-7 cell lines were maintained in Dulbecos Modified Eagles Medium (DMEM) containing 1g/L D-Glucose, L-Glutamine, pyruvate and supplemented with 10% Foetal bovine serum (FBS), 1% Penicillin/ Streptomycin and 1% Non-essential amino acids (all Gibco).

### Matrigel Invasion Assay

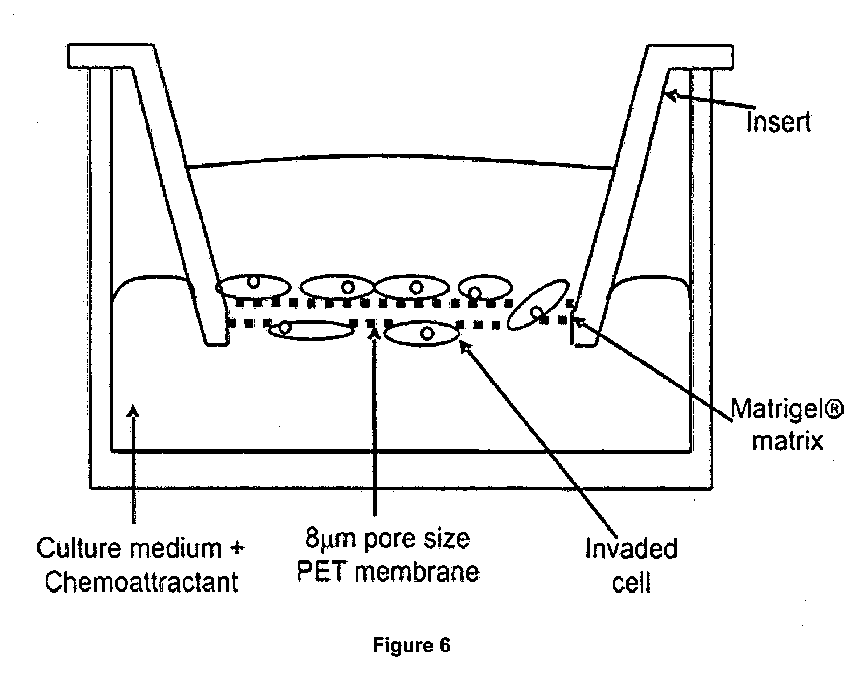

Biocoat 6-well plate Matrigel invasion chambers (BD Biosciences), Figure 6, were allowed to come to room temperature and rehydrated, with growth medium in the companion plate and serum free medium in the inserts, for 2h in a humidified incubator, 37°C. 5% $CO_2$ atmosphere. Cells were harvested as described and resuspended in serum-free medium at a density of $1.25 \times 10^5$ cells per ml. Medium was removed from the companion plate and inserts. Complete growth medium (2.5ml per well) was added to the companion plate. FBS from the same lot was used throughout all the invasion assays. 2ml of cell suspension was placed into the inserts and incubated in a humidified incubator, 37°C, 5% $CO_2$ atmosphere for either 48 or 72h. Following incubation the cells were fixed on both sides of the insert by immersion in 70% ethanol for 30min at room temperature. Cells were stained by immersion in Hematoxylin solution (Sigma) for 5min. The inserts were rinsed in $dH_2O$ and using a cotton bud, half of the non-invaded cells (apical side) were removed as were the opposite half of the invaded cells (basolateral side). Using Nikon Optiphot-2 microscope, 5 random fields of each side were counted and images taken using Kromascan Metero II software. The percentage invasion was calculated using the following formula:

$$\text{Percentage Invasion} = \text{Total invaded cells} / (\text{Total non-invaded} + \text{Invaded cells}) \times 100\%.$$

### Sub-culturing of invaded cells

Invasion assays were performed as described with an incubation time of 72h for the MCF-7 cells and the inserts were removed for fixing and staining. The companion plate from the invasion assay was retained, containing the medium from the assay. These contained cells, which had invaded through the membrane and dropped off. 1ml of growth medium was added to each well and the plates were returned to a humidified incubator, 37°C, 5% $CO_2$ atmosphere overnight to allow any cells within the medium to settle and adhere to the plate. The medium was replaced with fresh growth medium every 2-3 days until sufficient numbers of cells were achieved, approximately sub-confluence in the companion plate. The cells were harvested and cultured to sufficient numbers as described to allow re-introduction into an invasion assay. These cells were named $I^n$ where n = number of times the cells have passed through the invasion chamber.

### Growth and isolation in presence of artificial basement membrane matrix

In order to mimic the microenvironment during the invasion assay, cells were grown in the presence of Matrigel basement membrane matrix (BD Biosciences), and subsequently recovered from the matrix prior to RNA extraction. Matrigel basement membrane matrix was allowed to thaw overnight at 4°C. All pipettes, plates and tubes were kept cool to prevent premature gelling of Matrigel. Matrigel basement membrane matrix was diluted 1:10 with cold serum free medium then 2.5ml was added to cover 900 mm dish and incubated for 1h at room temperature. Any unbound material was aspirated and the dishes rinsed gently with serum free medium. Cells were harvested, seeded and allowed to reach subconfluence before being recovered from the matrix. Cells were washed 3 times with PBS and 3ml Cell Recovery Solution (BD Biosciences) added per dish. The cell/gel layer was scraped into a cold 15ml centrifuge tube along with 3ml of additional recovery solution after rinsing the dish. This mixture was left on ice for 1h and then centrifuged at 200-300 x g for 5min. The pellets were washed by gentle resuspension in ice cold PBS and centrifugation, twice.

**Matrigel invasion chamber**

Matrigel invasion assays were performed. Initially all cell lines were incubated in the invasion chamber for 48hr and percentage invasion was calculated. This provided a baseline percentage invasion for each of the cell lines. All subsequent invasion assays involving MCF-7 cells were incubated for 72hrs.

**Isolation of invaded subclones**

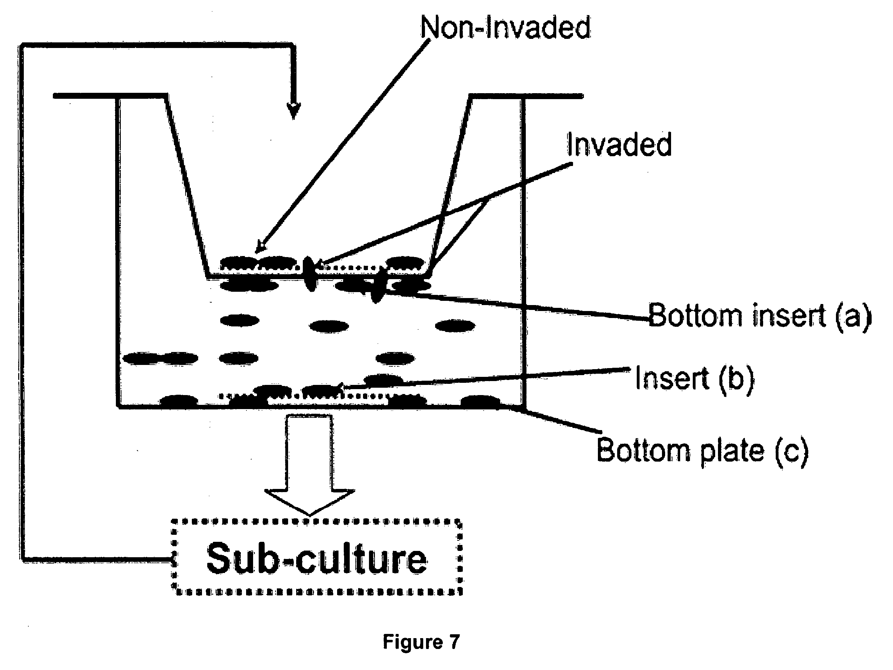

The Matrigel-coated membranes from the invasion assay inserts were aseptically removed and placed in the bottom of a companion plate. MCF-7 cells ($2.5 \times 10^5$) were loaded into the top well of the Matrigel invasion assay and incubated for 72h. On completion of the assay the invading cells were collected as follows; (a) Cells that had degraded the Matrigel matrix and migrated to the underside of the membrane were scraped off using a cell scraper (Corning, Netherlands) and transferred to a single well of a 6-well plate containing 1ml of complete culture medium; (b) Cells that had degraded the Matrigel matrix and migrated into the bottom well and adhered to the inserts in the bottom of the companion plate were also collected. These inserts in the bottom of the companion plate were aseptically transferred to a 6-well plate and 1ml culture medium placed in the companion plate of the invasion assay; (c) MCF-7 cells were loaded into the top well of an additional Matrigel invasion assay and incubated for 72h. Cells that had degraded the Matrigel matrix and migrated into the bottom well and adhered to the bottom of the companion plate were collected. These invaded subclones were cultured by replacing the culture medium every 2-3 days to give rise to 3 MCF-7 subclones (see Figure 7).

Once sufficient numbers of these subclones were achieved, they were introduced into another Matrigel invasion assay with the parental MCF-7 cells as a control. The invaded subclones were isolated and re-introduced into invasion assay to give rise to $I^n$ where n = number of times through invasion assay.

**Wound scrape assays**

Cells were seeded in 12 well plates (Iwaki, Sterilin Limited, United Kingdom) and allowed to grow to form a confluent monolayer. Cells were scraped away using a 10$\mu$l tip to form a channel and the medium replaced. The medium was changed again after 48 hours. The motility of the cells was assessed by measuring the rate of closure of the channel both by distance and area at several time points. All images were taken using a phase contrast inverted microscope (Nikon Eclipse TS100) at X4 magnification in conjunction with Nikon DS1 imaging software and measured using NIS Elements software. The assay was also performed with serum-free medium, added 24 hours before forming a channel using a 10$\mu$l tip, and replaced after 48 hours to assess motility without proliferation.

**Total RNA extraction from cell lines**

Total RNA was isolated using RNeasy mini kit (Qiagen). Cells were trypsinised as described and collected as a cell pellet of 1-2 $\times 10^6$ cells per pellet prior to extraction. The pellet was disrupted by flicking the tube and addition of 350$\mu$l RLT lysis buffer containing $\beta$-Mercaptoethanol and vortexing. An aliquot of 350$\mu$l of 70% ethanol was added and the mixture transferred to a silica-gel membrane column and centrifuged for 15sec at 10,000 rpm. The column was washed with 350$\mu$l RW1 washing buffer. DNase digestion was performed by addition of 80$\mu$l of DNase I in buffer RDD (Qiagen) and incubation at room temperature for 15min. The column was washed twice with 500$\mu$l buffer RPE containing 70% Ethanol and the RNA eluted in 40$\mu$l RNase-free water and stored at -80°C.

**Quantification of total RNA**

Total RNA was quantified using either NanoDrop ND-1000 spectrophotometer (Labtech) or 2100 Bioanalyser (Agilent). Using the NanoDrop method, 1.5$\mu$l of RNase free water was loaded to zero the absorbance of the instrument then using the RNA setting 1.5$\mu$l of total RNA was loaded and quantified. The NanoDrop gave concentrations in ng/ul and the ratio of $A_{260}/A_{280}$ gave an indication of the purity of the RNA sample, which should be in the range 1.8-2.0 for RNA. The Bioanalyser was used in conjunction with RNA 6000 Nano assay chips. Total RNA samples were diluted 1: 5 with RNase free water prior to analysis. The data was presented as concentration in ng/$\mu$l and giving a RNA Integrity Number (RIN) for each sample. The RIN gives an indication of the quality and purity of the RNA sample and is a value between 1 and 10 with 10 being the highest quality. Only samples with a RIN of 8.0 or above were used.

**Sample Preparation and Microarray Analysis**

MCF-7 I0 and MCF-7 I6 cells were grown in the presence of basement membrane matrix and recovered as described. Total RNA was extracted and quantified using the Bioanalyser as described to give triplicate samples for both. Microarray gene expression analysis was performed by Almac Diagnostics N. Ireland using Affymetrix Human GeneChip U133 Plus 2.0 array.

The samples were supplied to Almac Diagnostics as total RNA of 3x MCF-7 I0 - Control and 3x MCF-7 I6 - Treated. The microarray data was presented as 3 separate lists; present absent, stringent and less stringent.

Microarray experiments were carried out by Almac Diagnostics (http://www.almacgroup.com/diagnostics). All Eukaryotic Target Preparations using the One-Cycle and Two-Cycle labelling assays were carried out in accordance with the Affymetrix GeneChip® Expression Analysis Technical Manual. 2$\mu$g of total RNA was converted to cDNA via first and second strand synthesis using the GeneChip® Expression 3'-Amplification One-Cycle cDNA Synthesis kit, in conjunction with the GeneChip® Eukaryotic PolyA RNA Control Kit. Cleanup of the double-stranded cDNA was carried out using the GeneChip® Sample Cleanup Module. Biotin labeled cRNA was synthesized from the double-stranded cDNA using the GeneChip® Expression 3'-Amplification IVT Labeling Kit. To determine an accurate concentration and purity for the newly synthesized biotin labeled cRNA, a cleanup step was carried out to remove unincorporated NTPs using the GeneChip® Sample Cleanup Module. The cRNA quality was assessed using an Eppendorf Biophotometer and an Agilent 2100 bioanalyzer. 25$\mu$g of cRNA generated in the *in vitro* transcription (IVT) reaction was fragmented using 5X Fragmentation buffer and RNase-free water contained within the GeneChip® Sample Cleanup Module. The fragmentation reaction was carried out at 94°C for 35 min to generate 35-200 base fragments for hybridization. The fragmented cRNA quality was assessed using an Agilent 2100 bioanalyzer. Prior to hybridization, the adjusted cRNA yield in the fragmentation reaction was calculated to account for carryover of total RNA in the IVT reaction. 15$\mu$g of fragmented cRNA was made up into a hybridization cocktail in accordance with the Affymetrix technical manual corresponding to a 49 format (standard)/64 format array. The hybridization cocktail was added to the appropriate array and hybridized for 16 h at 45°C. The array was washed and stained on the GeneChip® fluidics station 450 using the appropriate fluidics script. Once completed, the array was inserted into the Affymetrix autoloader carousel and scanned using the GeneChip® Scanner 3000.

For all gene lists the treated samples (MCF-7 I6) were used as variables and data was normalised where values below 0.01 were set to 0.01. Data normalization was performed using GeneSpring software. All six (3 for MCF7-I0 and 3 for MCF7-16) unscaled Affymetrix CHP "chip" data files were used for the analysis. Values below 0.01 were set to 0.01. Each measurement was divided by the 50th percentile of all measurements in that sample. A per-gene normalization to specific samples (control samples) was applied. The control value was the mean of the three control replicates. The Cross Gene Error Model (CGEM) was established based on replicates. The average base /proportional value was 15.59. This analysis was carried out by ALMAC Diagnostics (http://www.almacgroup.com/diagnostics).

The stringent and less stringent gene lists were generated using a per-gene normalisation to specific samples (controls) was applied. The control value was a mean of the 3 replicates. The present absent gene list was generated by dividing each gene by the median of its measurements in all samples. If the median of the raw values was below 10 then each measurement for that gene was divided by 10 if the numerator was above 10, otherwise the measurement was thrown out. All genes were extracted to MS Excel. The Affymetrix probe ID's were then re-imported into GeneSpring. The selected present-absent genes were assessed based on raw data using fold change and p-values based on univariate *t*-statistics. Raw and pre-processed microarray data for the MCF7-I0 and MCF7-6 cells were submitted to the Gene Expression Omnibus (GSE17889).

**Microarray data pre-processing and probe selection**

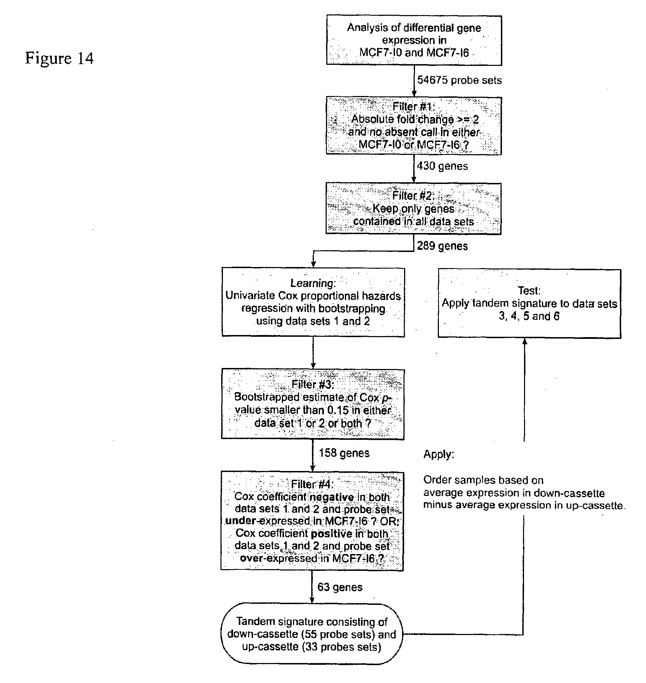

Expression profiling of the 10 and 16 cell lines was based on triplicate micorarray experiments. Univarite t-statistics with Benjamini and Hochberg's method for controlling the false discovery rate (FDR) at 0.05 revealed the probes for inclusion in the further analysis. In addition, probes with an absolute fold change of at least 2.0 were included, termed Filter #1 herein. Control probes were removed prior to analysis. Flagged expression values were treated as missing values and not included in further analysis. The remaining expression values were log$_2$-transformed. The values were median-centered first by array and then by probe. With reference to Figure 14, the differential expression in I0 and I6 was analysed using the Affymetrix Human U133 Plus 2 GeneChip. Triplicate micorarray experiments using Affymetrix Human U133 Plus 2 GeneChips revealed that 546 probe sets referring to 430 genes are differentially expressed between MCF7-10 and MCF7-16.

**Public microarray and patient data sets**

Three publicly available microarray data sets, derived from frozen primary breast tumor samples obtained by surgery, and clinical patient data, were analysed. Data set 1 contained only lymph-node negative tumors (at the time of diagnosis) obtained from patients who had not received chemotherapy or hormonal therapy. Therefore, patients of this type were only selected from data sets 2 and 3. Table 1 shows a synopsis of the data set properties.

Table 1. Synopsis of the publicly available data sets.

|  | Data set 1 | Data set 2 | Data set 3 |
|---|---|---|---|
| # of patients | 286 | 125 | 141 |
| Age |  |  |  |
| Mean (SD) | 54 (12) | 52 (10) | 43 (6) |
| ≤ 40 | 36 (13%) | 16 (13%) | 44 (31%) |
| 41-55 | 129 (45%) | 57 (46%) | 97 (69%) |
| 56-70 | 89 (31%) | 49 (39%) | - |
| >70 | 32 (11%) | 3 (2%) | - |
| Grade |  |  |  |
| Poor | 148 (52%) | 28 (22%) | 66 (47%) |
| Moderate | 42 (15%) | 48 (38%) | 42 (30%) |
| Good | 7 (2%) | 32 (26%) | 33 (23%) |
| Unknown | 89 (31%) | 17 (14%) | - |
| ER status |  |  |  |
| Positive | 209 (73%) | 85 (68%) | 104 (74%) |
| Negative | 77 (27%) | 34 (27%) | 37 (26%) |
| Unknown | - | 6 (5%) | - |
| Metastases within 5 years |  |  |  |
| Yes | 93 (33%) | 21 (17%) | 39 (28%) |
| No | 183 (64%) | 86 (69%) | 97 (69%) |
| Censored | 10 (3%) | 18 (14%) | 5 (4%) |
| Other |  |  |  |
| Platform | Affymetrix Human U133A | Affymetrix Human U133A | Hu25k microarray |
| Reference(s ) | Wang *et al.* (2006) - | Sotiriou *et al.* (2005) | van't Veer *et al.* (2002); Chang *et al.* (2005) |
| URL | http: //www.ncbi.nlm.nih.gov /geo/ query/acc.cgi? acc=GSE20 34 | http: //www.ncbi.nlm.nih.go v/geo/ query/acc.cgi? acc=GSE29 90 | http://microarray-pubs. stanford.edu/wound_ NKI/ |

Data set 3 is based on cDNA arrays, hence a matching to probe set identifiers is not possible. Therefore, the names of the differentially expressed genes in I0 vs. I6 were defined as canonical names and all their synonyms and NCBI reference IDs were retrieved using iHop [http://www.ihop-net.org/UniPub/iHOP]. Then, for each of the differentially expressed genes, its name or one of its synonyms was checked for inclusion in data set 3, and whether the corresponding NCBI RefSeq is in accordance. The corresponding genes in data set 3 were then selected, and the gene name replaced by the canonical name, if necessary. Finally, all genes that are contained in all three data sets were also selected. Control probes were removed prior to analysis. Flagged expression values were treated as missing values and not included in further analysis. The remaining expression values were log2-transformed. The values were median-centered first by array and then by probe. As the transcriptional analysis involved different microarray platforms, analysis was focussed on genes that were contained on all arrays, termed Filter #2 herein, leaving 289 genes for further analysis.

**Selection of prognostic genes**

Referring to Figure 14, using univariate Cox proportional hazards regression and bootstrapping in combination with the filtering technique described below, probe sets were selected that correlate significantly with time to distant metastases in two cohorts of breast cancer patients (referred to as data set 1 and 2, respectively) termed Filter #3 herein. In total, these two training sets comprise 411 lymph-node negative (at time of diagnosis) patients who did not receive chemotherapy or hormonal treatment. We identified a cassette of down- and up-regulated genes in MCF7-16 whose expression correlates significantly with time to distant metastases. Next, we assessed the prognostic power of the signature using an independent test set (data set 3) comprising a comparable cohort of 141 breast cancer patients. Univariate Cox proportional hazards regression was carried out on data set 1 and 2 using R 2.5.1 [R 2.5.1; The R Foundation for Statistical Computing, 2007] to identify probes that correlate with the time endpoint (i.e., distant metastases-free survival or last time to follow-up). To address the problem of multiple testing, the analysis was embedded in a bootstrapping approach as follows. One thousand bootstrap samples were created by repeatedly sampling the patients with replacement from data set 1. Then, for each bootstrap sample, the Cox regression p-value for each probe was calculated, leading to 1 000 bootstrapped p-values per probe. To derive a robust estimate of the p-value for a probe, the average of all its corresponding bootstrapped p-values in the interval of the mean $\pm$ 1 standard deviation was computed. An analogous procedure was followed for data set 2 and obtained the estimates for the p-values, $\hat{p}_{i,1}$ and $\hat{p}_{i,2}$, for the $i$-th probe in data set 1 and data set 2, respectively. Only those probes with $\hat{p} < 0.15$ in either data set 1 or data set 2 or in both were selected. These probes could be strongly or moderately associated with distant metastases-free survival.

In Cox proportional hazards model, the exponentiated Cox regression coefficients are interpretable as multiplicative effects on the hazard. An exponentiated coefficient smaller than 1 can be interpreted as having a reducing effect on the hazard, whereas an exponentiated coefficient larger than 1 as having an increasing effect. Thus, only probes that have the same effect in both data set 1 and 2 were selected, i.e., only probes for which the exponentiated coefficient has the same sign in both data sets were kept.

All probes referring to a gene that is underexpressed in I6 (compared to I0) must have an exponentiated coefficient smaller than 1. This reflects the expected effect that an increase in this gene's expression should be associated with a decrease of the hazard. Further, all probes referring to a gene that is overexpressed in I6 (compared to I0) must have an exponentiated coefficient larger than 1. This reflects the expected effect that an increase in this gene's expression should be associated with an increase of the hazard.

**Relative weights of the predictive probes**

The association between the predictive importance (or relative weight) of a probe, and its association with distant metastases-free survival is captured by the Cox p-value. Hence, the smaller the bootstrapped-estimated p-value $p_{bi}$ for probe $i$ is in a data set, the higher is the relative importance of this gene. The inverses of the bootstrapped-estimated p-values would express this relationship; however, the relative weights would be dominated by very small $\hat{p}$. To alleviate this bias, a log-transformed value was used, $-\log(\hat{p}_{i,j})$. Let $\hat{p}_{i,1}$ and $\hat{p}_{i,2}$ be the bootstrap-estimated p-value for the $i$-th probe in data set 1 and data set 2, respectively, and $i = 1..n$, with $n = 89$. The average weight $\overline{w}_j$ of all probes in data set $j$ is then defined as follows.

$$\overline{w}_j = n^{-1}\sum_i -\log(\hat{p}_{i,j}) \qquad (1)$$

The weight $w_i$ for probe $i$ is then defined as a relative score, expressed in %, and averaged over the two data sets as shown in Equation (2).

$$w_i = 0.5\left(\frac{-\log(\hat{p}_{i,1})}{\overline{w}_1} + \frac{-\log(\hat{p}_{i,2})}{\overline{w}_2}\right)100\% \qquad (2)$$

The weight $w_i$ is a simple measure for assessing the relative importance of probe $i$ and has an obvious interpretation. This weight can be easily refined as more evidence is accumulated from additional data sets.

Filter #4 identifies prognostic genes of critical importance for this analysis. In Cox proportional hazards regression, the exponentiated Cox coefficients are interpretable as multiplicative effects on the hazard (here, risk of developing distant metastases). Therefore, to enforce consistency between the *in vitro* results and observations in the genomic profiles of patients, all probe sets referring to a gene that is *under*-expressed in MCF7-I6 (compared to MCF7-I0) were required to have a Cox coefficient *smaller* than 0. This reflects the expected effect that an *increase* in this probe's expression should be associated with a *decrease* of the hazard. In contrast, all probe sets referring to a gene that is *over*-expressed in MCF7-16 (compared to MCF7-10) were required to have a coefficient *greater* than 0. This reflects the expected effect that an *increase* in this gene's expression should be associated with an *increase* of the hazard.

This sequential filtering process resulted in 87 probe sets referring to 63 genes (36 under- and 27 over-expressed). See Tables 2 and 3. This *tandem* signature comprises a down-cassette of 55 probe sets (35 genes) and an up-cassette of 32 probe sets (27 genes).

## Examples

### Example 1: Selection of MCF-7 Invaded subclones

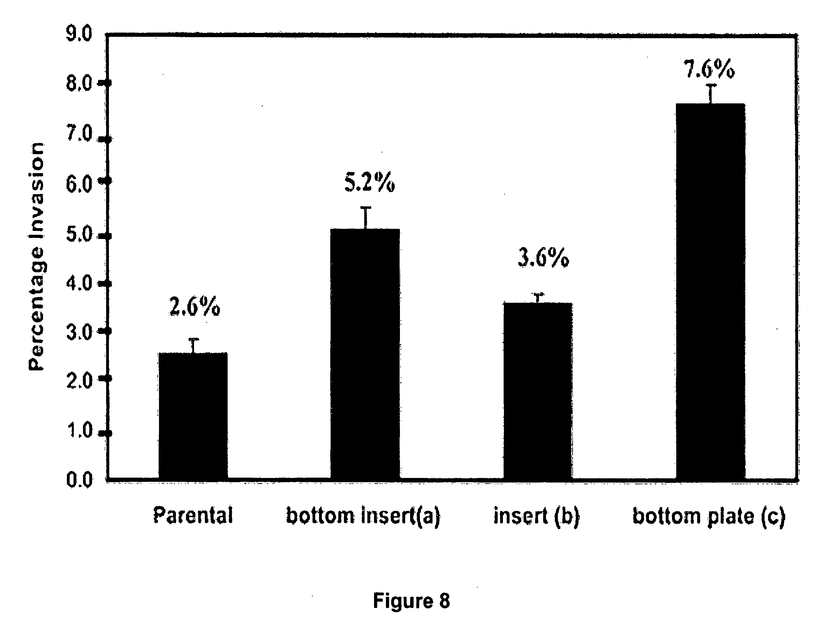

The hyper-invasive subclones were selected using Matrigel® invasion chambers as a model for the invasion process *in vivo*. MCF-7 cells had a percentage invasion of just 1.5% after 48h incubation in the invasion assay, so the incubation time was increased to 72h to enable more cells to invade. Referring to Figure 7, the cells which had invaded were isolated from (a) the basolateral side of the insert, (b) those which had adhered to the Matrigel insert in the bottom of the companion plate and (c) the bottom of the plate. These sub-populations were cultured to sufficient numbers to enable reintroduction into a second invasion cycle and the percentage invasion was again calculated. All 3 invaded subclones displayed a percentage invasion greater than that of the parental MCF-7 cells, termed MCF-7 I0, which were used as a control. The subclones isolated from the bottom of the plate, Figure 7(c), displayed the greatest increase in percentage invasion at 7.6% compared to the MCF-7 I0 cells at 2.6% (see Figure 8).

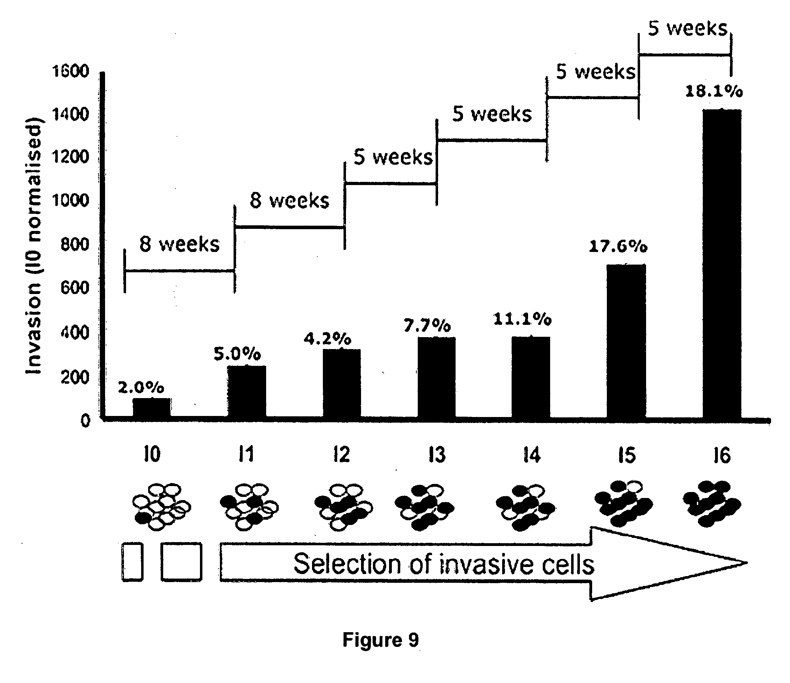

Of the percentage invasion results from the MCF-7 I1 invasion assays of the 3 invaded subclones, the "bottom of the plate subclone" (c), Figure 8, was the most interesting with a percentage invasion greater than the parental MCF-7 cells and greater than the other MCF-7 invaded subclones. Following this invasion assay the cells in the companion plate were cultured and re-introduced into the invasion assay again, these were now denoted as MCF-7 I2 cells. Again, the percentage invasion was higher than that of the MCF-7 I0 cells of the same passage number, which were used as a control. When the percentage invasion of the MCF-7 I1 and I2 subclones were normalised to the MCF-7 10 of the same experiment, it was found that the MCF-7 I2 subclone was more invasive than the MCF-7 I1 subclone (see Figure 9).

This process of culturing the invaded subclones and re-introducing them into the Matrigel invasion assay was repeated until MCF-7 I6 cells were isolated; these correspond to a subpopulation that had been selected through the invasion chamber 6 times. Following each invasion selection cycle the percentage invasion was calculated and normalised with the MCF-7 10 control in the same plate. Each successive invaded subclone population displayed an increase in invasion compared to the MCF-7 10 control and also compared to the preceding invaded subclone (see Figure 9). The MCF-7 I6 subclone displayed a percentage invasion of 18.1 % compared to 2.0% for the parental MCF-7 I0 cells, within the invasion assay. When normalised with the MCF-7 I0 invasion, the MCF-7 I6 cells had an invasion capacity 14 times the average MCF-7 10 control across the whole experiment, Figure 9.

### Example 2: Probes that are significantly differentially expressed in I6 vs. 10 and associated with distant metastases-free survival in data set 1 and 2

In total, 87 probes are significantly associated with distant metastases-free survival, with 55 probes being under- and 32 probes being overexpressed in I6 vs. I0. These probes refer to 63 unique, annotated genes, with 36 being under- and 27 being overexpressed in I6 vs. I0. The set of downregulated probes is referred to as "down-cassette" (Table 2) and the set of upregulated probes as "up-cassette" (Table 3). Using the bootstrapped p-values for the predictive power of the probes, a weighting scheme was devised that assigns a normed score to each probe. This score reflects the relative importance (in percent) of the probe with respect to distant metastases-free survival. For example, B2M is twice as important as ARHGAP26.

Table 2. Down-cassette of probes that are significantly differentially expressed in I6 vs. I0 and associated with distant metastases-free survival in data set 1 and 2.

| Probe set ID | Gene Symbol | Fold change (16 vs 10) | Weight | Description |
|---|---|---|---|---|
| 201752_s_at | ADD3 | -3.93 | 0.82 | adducin 3 (gamma) |
| 205068_s_at | ARHGAP26 | -1.78 | 0.7 | Rho GTPase activating protein 26 |
| 216231_s_at | B2M | -2.09 | 1.4 | beta-2-microglobulin |
| 210538_s_at | BIRC3 | -2.27 | 0.84 | baculoviral IAP repeat-containing 3 |
| 209835_x_at | CD44 | -1.71 | 1.66 | CD44 antigen (homing function and Indian blood group system) |
| 212014_x_at | CD44 | -1.71 | 1.65 | CD44 antigen (homing function and Indian blood group system) |
| 204490_s_at | CD44 | -1.48 | 1.64 | CD44 antigen (homing function and Indian blood group system) |
| 212063_at | CD44 | -2.21 | 1.63 | CD44 antigen (homing function and Indian blood group system) |
| 217523_at | CD44 | -2.1 | 0.68 | CD44 antigen (homing function and Indian blood group system) |
| 210070_s_at | CHKB | -1.64 | 0.9 | choline kinase beta ; carnitine palmitoyltransferase 1B (muscle) |
| 221675_s_at | CHPT1 | -1.94 | 0.97 | choline phosphotransferase 1 |
| 209687_at | CXCL12 | -1.6 | 1.71 | chemokine (C-X-C motif) ligand 12 (stromal cell-derived factor 1) |
| 203666_at | CXCL12 | -2.15 | 1.62 | chemokine (C-X-C motif) ligand 12 (stromal cell-derived factor 1) |
| 204780_s_at | FAS | -2.08 | 1.7 | Fas (TNF receptor superfamily, member 6) |
| 204781_s_at | FAS | -1.92 | 1.13 | Fas (TNF receptor superfamily, member 6) |
| 216252_x_at | FAS | -1.57 | 1.13 | Fas (TNF receptor superfamily, member 6) |
| 218999_at | FLJ11000 | -2.9 | 1.7 | hypothetical protein FLJ11000 |
| 218429_s_at | FLJ11286 | -4.16 | 0.94 | hypothetical protein FLJ11286 |
| 53720_at | FLJ11286 | -3.99 | 0.83 | hypothetical protein FLJ11286 |
| 215313_x_at | HLA-A | -3.17 | 0.9 | major histocompatibility complex, class I, A |

(continued)

| Probe set ID | Gene Symbol | Fold change (16 vs 10) | Weight | Description |
|---|---|---|---|---|
| 213932_x_at | HLA-A | -2.38 | 0.75 | major histocompatibility complex, class I, A |
| 211911_x_at | HLA-B | -2.8 | 1.02 | major histocompatibility complex, class I, B |
| 214459_x_at | HLA-C | -3.3 | 1.32 | major histocompatibility complex, class I, C |
| 211799_x_at | HLA-C | -3.87 | 1.07 | major histocompatibility complex, class I, C |
| 208812_x_at | HLA-C | -3.49 | 1.04 | major histocompatibility complex, class I, C |
| 216526_x_at | HLA-C | -4.12 | 0.84 | major histocompatibility complex, class I, C |
| 217478_s_at | HLA-DMA | -2.05 | 2.3 | major histocompatibility complex, class II, DM alpha |
| 215193_x_at | HLA-DRB1 | -1.79 | 2.01 | major histocompatibility complex, class II, DR beta 1 |
| 209312_x_at | HLA-DRB1 | -1.82 | 1.95 | major histocompatibility complex, class II, DR beta 1 |
| 221491_x_at | HLA-DRB1 | -1.68 | 1.22 | major histocompatibility complex, class II, DR beta 1 |
| 208306_x_at | HLA-DRB4 | -5.62 | 2.22 | major histocompatibility complex, class II, DR beta 4 |
| 204670_x_at | HLA-DRB5 | -1.77 | 1.91 | major histocompatibility complex, class II, DR beta 5 |
| 204806_x_at | HLA-F | -1.83 | 0.92 | major histocompatibility complex, class I, F |
| 221875_x_at | HLA-F | -2.52 | 0.86 | major histocompatibility complex, class I, F |
| 221978_at | HLA-F | -1.87 | 0.72 | major histocompatibility complex, class I, F |
| 211529_x_at | HLA-G | -3.07 | 1.04 | HLA-G histocompatibility antigen, class I, G |
| 211528_x_at | HLA-G | -2.41 | 0.97 | HLA-G histocompatibility antigen, class I, G |
| 214022_s_at | IFITM1 | -10.24 | 1.06 | interferon induced transmembrane protein 1 (9-27) |
| 212203_x_at | IFITM3 | -2.79 | 0.94 | interferon induced transmembrane protein 3 (1-8U) |
| 33304_at | ISG20 | -2.47 | 0.81 | interferon stimulated exonuclease gene 20kDa |

(continued)

| Probe set ID | Gene Symbol | Fold change (16 vs 10) | Weight | Description |
|---|---|---|---|---|
| 217933_s_at | LAP3 | -3.11 | 1.07 | leucine aminopeptidase 3 |
| 200923_at | LGALS3BP | -5.02 | 0.7 | lectin, galactoside-binding, soluble, 3 binding protein |
| 206346_at | PRLR | -1.61 | 0.98 | prolactin receptor |
| 204279_at | PSMB9 | -4.37 | 0.84 | proteasome (prosome, macropain) subunit, beta type, 9 |
| 200927_s_at | RAB14 | -1.27 | 0.66 | RAB14, member RAS oncogene family |
| 203788_s_at | SEMA3C | -1.35 | 0.67 | semaphorin-3C precursor |
| 201427_s_at | SEPP1 | -4.08 | 0.84 | selenoprotein P, plasma, 1 |
| 202863_at | SP100 | -3.71 | 0.69 | nuclear antigen Sp100 |
| 209761_s_at | SP110 | -6.04 | 0.97 | SP110 nuclear body protein |
| 208392_x_at | SP110 | -4.22 | 0.85 | SP110 nuclear body protein |
| 203768_s_at | STS | -5.07 | 1.32 | steroid sulfatase (microsomal), arylsulfatase C, isozyme S |
| 202307_s_at | TAP1 | -4.22 | 0.69 | transporter 1, ATP-binding cassette, sub-family B (MDR/TAP) |
| 202687_s_at | TNFSF10 | -2.15 | 1.23 | tumor necrosis factor (ligand) superfamily, member 10 |
| 202688_at | TNFSF10 | -2.02 | 1.21 | tumor necrosis factor (ligand) superfamily, member 10; |
| 203147_s_at | TRIM14 | -2.79 | 0.84 | tripartite motif-containing 14 |

Table 3. Up-cassette of probes that are significantly differentially expressed in I6 vs. I0 and associated with distant metastases-free survival in data set 1 and 2.

| Probe set ID | Gene Symbol | Fold change (16 vs 10) | Weight | Description |
|---|---|---|---|---|
| 209122_at | ADFP | 2.11 | 0.99 | adipose differentiation-related protein |
| 202912_at | ADM | 4.07 | 1.29 | adrenomedullin |
| 203180_at | ALDH1A3 | 2.91 | 1.17 | aldehyde dehydrogenase 1 family, member A3 |
| 39248_at | AQP3 | 1.53 | 0.79 | aquaporin 3 |
| 211946_s_at | BAT2D1 | 1.63 | 0.98 | BAT2 domain containing 1 |

(continued)

| Probe set ID | Gene Symbol | Fold change (16 vs 10) | Weight | Description |
|---|---|---|---|---|
| 211944_at | BAT2D1 | 1.09 | 0.64 | BAT2 domain containing 1 |
| 214820_at | BRWD1 | 1.22 | 1.01 | bromodomain and WD repeat domain containing 1 |
| 207996_s_at | C180RF1 | 1.23 | 1.84 | chromosome 18 open reading frame 1 |
| 209574_s_at | C180RF1 | 2.09 | 1.69 | chromosome 18 open reading frame 1 |
| 209682_at | CBLB | 1.97 | 1.17 | Cas-Br-M (murine) ecotropic retroviral transforming sequence b |
| 212977_at | CMKOR1 | 1.92 | 1.15 | chemokine orphan receptor 1 |
| 202806_at | DBN1 | 1.78 | 1.82 | drebrin 1 |
| 217025_s_at | DBN1 | 2.72 | 1.02 | drebrin 1 |
| 204540_at | EEF1A2 | 2.26 | 2.62 | eukaryotic translation elongation factor 1 alpha 2 |
| 219250_s_at | FLRT3 | 2.44 | 1.23 | fibronectin leucine rich transmembrane protein 3 |
| 221480_at | HNRPD | 1.91 | 0.84 | heterogeneous nuclear ribonucleoprotein D |
| 205258_at | INHBB | 2.33 | 1.13 | inhibin, beta B (activin AB beta polypeptide) |
| 216268_s_at | JAG1 | 3.26 | 1.13 | jagged 1 (Alagille syndrome) |
| 209099_x_at | JAG1 | 2.48 | 1.08 | jagged 1 (Alagille syndrome) |
| 32137_at | JAG2 | 1.93 | 0.99 | jagged 2 |
| 207029_at | KITLG | 1.82 | 0.66 | KIT ligand |
| 200771_at | LAMC1 | 1.97 | 1.08 | laminin, gamma 1 (formerly LAMB2) |
| 212364_at | MYO1B | 2.26 | 0.99 | myosin IB |
| 212739_s_at | NME4 | 1.97 | 0.96 | non-metastatic cells 4, protein expressed in |
| 213222_at | PLCB1 | 2.21 | 0.72 | phospholipase C, beta 1 (phosphoinositide-specific) |
| 211823_s_at | PXN | 1.59 | 1.34 | paxillin |

(continued)

| Probe set ID | Gene Symbol | Fold change (16 vs 10) | Weight | Description |
|---|---|---|---|---|
| 202219_at | SLC6A8 | 2.39 | 1.24 | solute carrier family 6 (neurotransmitter transporter, creatine), member 8 |
| 210854_x_at | SLC6A8 | 1.81 | 1.06 | solute carrier family 6 (neurotransmitter transporter, creatine), member 8 |
| 217875_s_at | TMEPAI | 2.11 | 0.62 | transmembrane, prostate androgen induced RNA |
| 201398_s_at | TRAM1 | 1.28 | 1.08 | translocation associated membrane protein 1 |
| 201294_s_at | WSB1 | 1.19 | 1.07 | WD repeat and SOCS box-containing 1 |
| 201296_s_at | WSB1 | 1.22 | 0.76 | WD repeat and SOCS box-containing 1 |

**Example 3: Analysis of Gene Ontology annotations**

BiNGO (Maere *et al.*, 2005) was used to detect groups of genes with a significantly overrepresented Gene Ontology (GO) annotation of biological process, molecular function, and cellular component. Significance analysis is based on the hypergeometric distribution; *p*-values are corrected based on Benjamini & Hochberg's method at a FDR of 0.05. For example, 18 of 36 (50%) down-regulated genes are annotated with the Gene Ontology (GO) function *immune response* (GO Id 6955), whereas only 654 of 13 953 (4.7%) genes have this annotation. The corrected *p*-value is $3.94 \times 10^{-14}$ ; hence, the process *immune response* is significantly overrepresented in the down-cassette. Similarly, the down-cassette contains a substantial amount of genes involved in antigen processing and presentation ($P = 6.85 \times 10^{-14}$), antigen processing and presentation of peptide antigen via MHC class I ($P = 4.41 \times 10^{-9}$), and cellular defense response ($P = 1.80 \times 10^{-2}$). Many genes in the down-cassette are located in the plasma membrane ($P = 1.45 \times 10^{-5}$), and notably in the MHC protein complex ($P = 7.00 \times 10^{-13}$). Genes in the up-cassette are involved, among others, in cell signaling, hemopoiesis, and regulation of cell migration. Interestingly, the up-cassette contains a significant ($P = 1.48 \times 10^{-3}$) number of growth factor related genes: JAG1, KITLG, INHBB, JAG2, and PXN.

Table 4: Significantly overrepresented biological processes in the down-cassette. GO, Gene Ontology; *p*-values are corrected based on Benjamini & Hochberg's FDR of 0.05.

| GO-ID | Description | Genes in down-cassette | P-value |
|---|---|---|---|
| 6955 | immune response | CXCL12, HLA-DMA, IFITM3, PSMB9, TNFSF10, HLA-F, HLA-B, IFITM1, HLA-G, HLA-DRB5, FAS, HLA-C, HLA-A, SEMA3C, TAP1, HLA-DRB1, LAP3, B2M | $3.94 \times 10^{-14}$ |

(continued)

| GO-ID | Description | Genes in down-cassette | P-value |
|---|---|---|---|
| 2376 | immune system process | CXCL12, HLA-DMA, IFITM3, PSMB9, TNFSF10, HLA-F, IFITM1, HLA-B, HLA-G, PRLR, HLA-DRB5, FAS, HLA-C, HLA-A, SEMA3C, TAP1, HLA-DRB1, LAP3, B2M | $6.85 \times 10^{-14}$ |
| 19882 | antigen processing and presentation | HLA-DRB5, HLA-DMA, HLA-C, PSMB9, HLA-A, HLA-F, HLA-DRB1, HLA-B, B2M, HLA-G | $6.85 \times 10^{-14}$ |
| 48002 | antigen processing and presentation of peptide antigen | HLA-DMA, HLA-C, HLA-A, HLA-F, HLA-B, B2M, HLA-G | $2.04 \times 10^{-10}$ |
| 51869 | physiological response to stimulus | CXCL12, HLA-DMA, IFITM3, PSMB9, TNFSF10, HLA-F, IFITM1, HLA-B, HLA-G, SP110, HLA-DRB5, FAS, HLA-C, HLA-A, SEMA3C, LGALS3BP, TAP1, HLA-DRB1, LAP3, B2M | $3.46 \times 10^{-10}$ |
| 2474 | antigen processing and presentation of peptide antigen via MHC class I | HLA-C, HLA-A, HLA-F, HLA-B, B2M, HLA-G | $4.41 \times 10^{-9}$ |
| 50874 | organismal physiological process | STS, CXCL12, HLA-DMA, IFITM3, PSMB9, HLA-F, TNFSF10, IFITM1, HLA-B, HLA-G, PRLR, SP110, HLA-DRB5, FAS, HLA-C, HLA-A, SEMA3C, TAP1, RAB14, HLA-DRB1, LAP3, B2M | $4.41 \times 10^{-9}$ |
| 2504 | antigen processing and presentation of peptide or polysaccharide antigen via MHC class II | HLA-DRB5, HLA-DMA, HLA-DRB1 | $4.91 \times 10^{-4}$ |
| 6952 | defense response | CXCL12, LGALS3BP, TAP1, LAP3, HLA-B, B2M, HLA-G | $5.21 \times 10^{-3}$ |
| 50896 | response to stimulus | CXCL12, IFITM3, HLA-B, IFITM1, HLA-G, ISG20, SP110, LGALS3BP, SEPP1, SEMA3C, TAP1, LAP3, B2M | $9.28 \times 10^{-3}$ |
| 16067 | cellular defense response | LGALS3BP, B2M, HLA-G | $1.80 \times 10^{-2}$ |
| 9607 | response to biotic stimulus | CXCL12, ISG20, IFITM3, IFITM1, LAP3 | $2.24 \times 10^{-2}$ |
| 9615 | response to virus | CXCL12, ISG20, LAP3 | $2.54 \times 10^{-2}$ |
| 42110 | T cell activation | HLA-DMA, LAP3, PRLR | $3.95 \times 10^{-2}$ |

(continued)

| GO-ID | Description | Genes in down-cassette | P-value |
|---|---|---|---|
| 42976 | activation of JAK protein | PRLR | $3.96 \times 10^{-2}$ |
| 1887 | selenium metabolism | SEPP1 | $3.96 \times 10^{-2}$ |
| 6657 | GDP-choline pathway | CHPT1 | $3.96 \times 10^{-2}$ |
| 738 | DNA catabolism, exonucleolytic | ISG20 | $3.96 \times 10^{-2}$ |
| 42977 | tyrosine phosphorylation of JAK2 protein | PRLR | $3.96 \times 10^{-2}$ |
| 8610 | lipid biosynthesis | FAS, CHPT1, TAP1, PRLR | $3.96 \times 10^{-2}$ |
| 42829 | physiological defense response | CXCL12, LGALS3BP, LAP3, B2M, HLA-G | $4.15 \times 10^{-2}$ |
| 48754 | branching morphogenesis of a tube | CD44, LAP3 | $4.15 \times 10^{-2}$ |
| 1763 | morphogenesis of a branching structure | CD44, LAP3 | $4.69 \times 10^{-2}$ |

Table 5: Significantly overrepresented molecular functions in the down-cassette. GO, Gene Ontology; p-values are corrected based on Benjamini & Hochberg's FDR of 0.05.

| GO-ID | Description | Genes in down-cassette | P-value |
|---|---|---|---|
| 4317 | 3-hydroxypalmitoyl-[acyl-carrier-protein] dehydratase activity | FAS | $2.96 \times 10^{-2}$ |
| 16631 | enoyl-[acyl-carrier-protein] reductase activity | FAS | $2.96 \times 10^{-2}$ |
| 4316 | 3-oxoacyl-[acyl-carrier-protein] reductase activity | FAS | $2.96 \times 10^{-2}$ |
| 4925 | prolactin receptor activity | PRLR | $2.96 \times 10^{-2}$ |
| 4773 | steryl-sulfatase activity | STS | $2.96 \times 10^{-2}$ |
| 19171 | 3-hydroxyacyl-[acyl-carrier-protein] dehydratase activity | FAS | $2.96 \times 10^{-2}$ |
| 16804 | prolyl aminopeptidase activity | LAP3 | $2.96 \times 10^{-2}$ |
| 32027 | myosin light chain binding | LAP3 | $2.96 \times 10^{-2}$ |
| 4313 | [acyl-carrier-protein] S-acetyltransferase activity | FAS | $2.96 \times 10^{-2}$ |
| 8310 | single-stranded DNA specific 3'-5' exodeoxyribonuclease activity | ISG20 | $2.96 \times 10^{-2}$ |
| 4319 | enoyl-[acyl-carrier-protein] reductase (NADPH, B-specific) activity | FAS | $2.96 \times 10^{-2}$ |
| 8859 | exoribonuclease II activity | ISG20 | $2.96 \times 10^{-2}$ |
| 30215 | semaphorin receptor binding | SEMA3C | $3.09 \times 10^{-2}$ |
| 8431 | vitamin E binding | TAP1 | $3.09 \times 10^{-2}$ |

(continued)

| GO-ID | Description | Genes in down-cassette | P-value |
|---|---|---|---|
| 4142 | diacylglycerol cholinephosphotransferase activity | CHPT1 | $3.09 \times 10^{-2}$ |
| 16418 | S-acetyltransferase activity | FAS | $3.09 \times 10^{-2}$ |
| 4178 | leucyl aminopeptidase activity | LAP3 | $3.09 \times 10^{-2}$ |
| 16419 | S-malonyltransferase activity | FAS | $3.09 \times 10^{-2}$ |
| 16420 | malonyltransferase activity | FAS | $3.09 \times 10^{-2}$ |
| 42978 | ornithine decarboxylase activator activity | PRLR | $3.09 \times 10^{-2}$ |
| 4314 | [acyl-carrier-protein] S-malonyltransferase activity | FAS | $3.09 \times 10^{-2}$ |
| 4315 | 3-oxoacyl-[acyl-carrier-protein] synthase activity | FAS | $3.09 \times 10^{-2}$ |
| 8297 | single-stranded DNA specific exodeoxyribonuclease activity | ISG20 | $3.09 \times 10^{-2}$ |
| 8296 | 3'-5'-exodeoxyribonuclease activity | ISG20 | $3.67 \times 10^{-2}$ |
| 10281 | acyl-ACP thioesterase activity | FAS | $3.67 \times 10^{-2}$ |
| 4305 | ethanolamine kinase activity | CHKB | $3.67 \times 10^{-2}$ |
| 16297 | acyl-[acyl-carrier-protein] hydrolase activity | FAS | $3.67 \times 10^{-2}$ |
| 4320 | oleoyl-[acyl-carrier-protein] hydrolase activity | FAS | $3.67 \times 10^{-2}$ |
| 4103 | choline kinase activity | CHKB | $3.67 \times 10^{-2}$ |
| 5515 | protein binding | CXCL12, BIRC3, HLA-DMA, CD44, TNFSF10, TRIM14, IFITM1, HLA-G, PRLR, SP110, FAS, HLA-A, SEMA3C, LGALS3BP, TAP1, ADD3, LAP3, B2M | $4.58 \times 10^{-2}$ |
| 5062 | hematopoietin/interferon-class (D200-domain) cytokine receptor signal transducer activity | SP110 | $4.58 \times 10^{-2}$ |

Table 6: Significantly overrepresented cellular components in the down-cassette. GO, Gene Ontology; p-values are corrected based on Benjamini & Hochberg's FDR of 0.05.

| GO-ID | Description | Genes in down-cassette | P-value |
|---|---|---|---|
| 42611 | MHC protein complex | HLA-DRB5, HLA-DMA, HLA-C, HLA-A, HLA-F, HLA-DRB1, HLA-B, B2M, HLA-G | $7.00 \times 10^{-13}$ |
| 42612 | MHC class I protein complex | HLA-C, HLA-A, HLA-F, HLA-B, B2M, HLA-G | $2.32 \times 10^{-8}$ |

(continued)

| GO-ID | Description | Genes in down-cassette | P-value |
|---|---|---|---|
| 5886 | plasma membrane | STS, HLA-DMA, IFITM3, CD44, TNFSF10, HLA-F, HLA-B, IFITM1, HLA-G, SP110, HLA-DRB5, HLA-C, HLA-A, RAB14, HLA-DRB1, LAP3, B2M | $1.45 \times 10^{-5}$ |
| 42613 | MHC class II protein complex | HLA-DRB5, HLA-DMA, HLA-DRB1 | $2.03 \times 10^{-4}$ |
| 44459 | plasma membrane part | HLA-DMA, CD44, TNFSF10, HLA-F, HLA-B, HLA-G, HLA-DRB5, SP110, HLA-A, HLA-C, HLA-DRB1, LAP3, B2M | $8.56 \times 10^{-4}$ |
| 16605 | PML body | ISG20, SP100 | $4.96 \times 10^{-3}$ |
| 16020 | membrane | STS, IFITM3, HLA-DMA, CD44, HLA-F, TNFSF10, IFITM1, HLA-B, HLA-G, PRLR, SP110, HLA-DRB5, FAS, HLA-C, HLA-A, LGALS3BP, CHPT1, TAP1, RAB14, ADD3, HLA-DRB1, LAP3, B2M, FLJ11000 | $5.50 \times 10^{-3}$ |
| 44425 | membrane part | STS, HLA-DMA, IFITM3, CD44, HLA-F, TNFSF10, IFITM1, HLA-B, HLA-G, PRLR, SP110, HLA-DRB5, FAS, HLA-C, HLA-A, TAP1, RAB14, HLA-DRB1, LAP3, B2M, FLJ11000 | $6.65 \times 10^{-3}$ |
| 16021 | integral to membrane | STS, HLA-DMA, IFITM3, CD44, HLA-F, TNFSF10, IFITM1, HLA-B, HLA-G, PRLR, SP110, HLA-DRB5, FAS, HLA-C, HLA-A, TAP1, HLA-DRB1, LAP3, B2M, FLJ11000 | $6.65 \times 10^{-3}$ |
| 31224 | intrinsic to membrane | STS, HLA-DMA, IFITM3, CD44, HLA-F, TNFSF10, IFITM1, HLA-B, HLA-G, PRLR, SP110, HLA-DRB5, FAS, HLA-C, HLA-A, TAP1, HLA-DRB1, LAP3, B2M, FLJ11000 | $6.65 \times 10^{-3}$ |
| 5770 | late endosome | HLA-DMA, RAB14 | $1.45 \times 10^{-2}$ |
| 5887 | integral to plasma membrane | SP110, HLA-DRB5, HLA-C, HLA-A, CD44, TNFSF10, LAP3, HLA-B, B2M | $1.59 \times 10^{-2}$ |
| 5768 | endosome | STS, HLA-DMA, RAB14 | $1.59 \times 10^{-2}$ |

(continued)

| GO-ID | Description | Genes in down-cassette | P-value |
|---|---|---|---|
| 31226 | intrinsic to plasma membrane | SP110, HLA-DRB5, HLA-C, HLA-A, CD44, TNFSF10, LAP3, HLA-B, B2M | $1.59 \times 10^{-2}$ |
| 16604 | nuclear body | ISG20, SP100 | $2.07 \times 10^{-2}$ |
| 267 | cell fraction | STS, SP110, FAS, CHPT1, TNFSF10, RAB14, HLA-B | $2.51 \times 10^{-2}$ |
| 5764 | lysosome | STS, HLA-DMA, RAB14 | $2.51 \times 10^{-2}$ |
| 323 | lytic vacuole | STS, HLA-DMA, RAB14 | $2.51 \times 10^{-2}$ |
| 42587 | glycogen granule | FAS | $2.51 \times 10^{-2}$ |
| 5773 | vacuole | STS, HLA-DMA, RAB14 | $3.23 \times 10^{-2}$ |

An analogous procedure was followed for the genes in the up-cassette. Note, that the corrected p-values are smaller than 0.10 but exceed 0.05; the up-cassette does not contain any genes involved in a biological process that is significantly overrepresented at FDR 0.05.

Table 7: Significantly overrepresented biological processes in the up-cassette. GO, Gene Ontology; p-values are corrected based on Benjamini & Hochberg's FDR of 0.10.

| GO-ID | Description | Genes in up-cassette | P-value |
|---|---|---|---|
| 7154 | cell communication | WSB1, CBLB, JAG1, KITLG, DBN1, SLC6A8, INHBB, PXN, PLCB1, ADM, TMEPAI, CMKOR1, ARRB1, JAG2 | $7.94 \times 10^{-2}$ |
| 50874 | organismal physiological process | JAG1, CBLB, AQP3, KITLG, ARRB1, DBN1, SLC6A8, INHBB, JAG2, PXN, ADM | $7.94 \times 10^{-2}$ |
| 9887 | organ morphogenesis | JAG1, KITLG, INHBB, JAG2, ALDH1A3 | $7.94 \times 10^{-2}$ |
| 7267 | cell-cell signaling | DBN1, SLC6A8, INHBB, JAG2, ADM | $7.94 \times 10^{-2}$ |
| 19952 | reproduction | KITLG, INHBB, JAG2, ADM | $7.94 \times 10^{-2}$ |
| 30097 | hemopoiesis | JAG1, KITLG, JAG2 | $7.94 \times 10^{-2}$ |
| 48534 | hemopoietic or lymphoid organ development | JAG1, KITLG, JAG2 | $7.94 \times 10^{-2}$ |
| 2520 | immune system development | JAG1, KITLG, JAG2 | $7.94 \times 10^{-2}$ |
| 1709 | cell fate determination | JAG1, JAG2 | $7.94 \times 10^{-2}$ |
| 7219 | Notch signaling pathway | JAG1, JAG2 | $7.94 \times 10^{-2}$ |
| 30334 | regulation of cell migration | JAG1, JAG2 | $7.94 \times 10^{-2}$ |
| 7588 | excretion | AQP3, ADM | $7.94 \times 10^{-2}$ |
| 51270 | regulation of cell motility | JAG1, JAG2 | $7.94 \times 10^{-2}$ |
| 40012 | regulation of locomotion | JAG1, JAG2 | $7.94 \times 10^{-2}$ |

(continued)

| GO-ID | Description | Genes in up-cassette | P-value |
|---|---|---|---|
| 45165 | cell fate commitment | JAG1, JAG2 | $7.94 \times 10^{-2}$ |
| 48176 | regulation of hepatocyte growth factor biosynthesis | INHBB | $7.94 \times 10^{-2}$ |
| 32605 | hepatocyte growth factor production | INHBB | $7.94 \times 10^{-2}$ |
| 48178 | negative regulation of hepatocyte growth factor biosynthesis | INHBB | $7.94 \times 10^{-2}$ |
| 48175 | hepatocyte growth factor biosynthesis | INHBB | $7.94 \times 10^{-2}$ |
| 6701 | progesterone biosynthesis | ADM | $7.94 \times 10^{-2}$ |
| 15914 | phospholipid transport | ABCA1 | $7.94 \times 10^{-2}$ |
| 42492 | gamma-delta T cell differentiation | JAG2 | $7.94 \times 10^{-2}$ |
| 46629 | gamma-delta T cell activation | JAG2 | $7.94 \times 10^{-2}$ |
| 45747 | positive regulation of Notch signaling pathway | JAG1 | $7.94 \times 10^{-2}$ |
| 9912 | auditory receptor cell fate commitment | JAG2 | $7.94 \times 10^{-2}$ |
| 45332 | phospholipid translocation | ABCA1 | $7.94 \times 10^{-2}$ |
| 46881 | positive regulation of follicle-stimulating hormone secretion | INHBB | $7.94 \times 10^{-2}$ |
| 32278 | positive regulation of gonadotropin secretion | INHBB | $7.94 \times 10^{-2}$ |
| 46887 | positive regulation of hormone secretion | INHBB | $7.94 \times 10^{-2}$ |
| 46884 | follicle-stimulating hormone secretion | INHBB | $7.94 \times 10^{-2}$ |
| 32276 | regulation of gonadotropin secretion | INHBB | $7.94 \times 10^{-2}$ |
| 32274 | gonadotropin secretion | INHBB | $7.94 \times 10^{-2}$ |
| 42448 | progesterone metabolism | ADM | $7.94 \times 10^{-2}$ |
| 46882 | negative regulation of follicle-stimulating hormone secretion | INHBB | $7.94 \times 10^{-2}$ |
| 32277 | negative regulation of gonadotropin secretion | INHBB | $7.94 \times 10^{-2}$ |
| 2011 | morphogenesis of an epithelial sheet | JAG1 | $7.94 \times 10^{-2}$ |
| 50773 | regulation of dendrite development | DBN1 | $7.94 \times 10^{-2}$ |

(continued)

| GO-ID | Description | Genes in up-cassette | P-value |
|---|---|---|---|
| 46880 | regulation of follicle-stimulating hormone secretion | INHBB | $7.94 \times 10^{-2}$ |
| 9653 | morphogenesis | JAG1, KITLG, DBN1, INHBB, JAG2, ALDH1A3 | $8.11 \times 10^{-2}$ |
| 48518 | positive regulation of biological process | LAMC1, JAG1, CBLB, KITLG, INHBB, ALDH1A3 | $8.19 \times 10^{-2}$ |
| 42445 | hormone metabolism | ALDH1A3, ADM | $8.19 \times 10^{-2}$ |
| 50858 | negative regulation of antigen receptor-mediated signaling pathway | CBLB | $8.19 \times 10^{-2}$ |
| 50860 | negative regulation of T cell receptor signaling pathway | CBLB | $8.19 \times 10^{-2}$ |
| 46888 | negative regulation of hormone secretion | INHBB | $8.19 \times 10^{-2}$ |
| 42491 | auditory receptor cell differentiation | JAG2 | $8.19 \times 10^{-2}$ |
| 8593 | regulation of Notch signaling pathway | JAG1 | $8.19 \times 10^{-2}$ |
| 6911 | phagocytosis, engulfment | ABCA1 | $9.61 \times 10^{-2}$ |

Table 8: Significantly overrepresented molecular functions in the up-cassette. GO, Gene Ontology; p-values are corrected based on Benjamini & Hochberg's FDR of 0.05.

| GO-ID | Description | Genes in up-cassette | P-value |
|---|---|---|---|
| 8083 | growth factor activity | JAG1, KITLG, INHBB, JAG2, PXN | $1.48 \times 10^{-3}$ |
| 5112 | Notch binding | JAG1, JAG2 | $5.04 \times 10^{-3}$ |
| 5102 | receptor binding | JAG1, KITLG, INHBB, JAG2, PXN, ADM | $3.03 \times 10^{-2}$ |
| 46812 | host cell surface binding | INHBB | $3.03 \times 10^{-2}$ |
| 5309 | creatine:sodium symporter activity | SLC6A8 | $3.03 \times 10^{-2}$ |
| 46789 | host cell surface receptor binding | INHBB | $3.03 \times 10^{-2}$ |
| 5308 | creatine transporter activity | SLC6A8 | $3.03 \times 10^{-2}$ |

Table 9: Overrepresented cellular components in the up-cassette. GO, Gene Ontology; p-values are corrected based on Benjamini & Hochberg's FDR of 0.05.

| GO-ID | Description | Genes in up-cassette | P-value |
|---|---|---|---|
| 42641 | actomyosin | DBN1 | $1.10 \times 10^{-1}$ |
| 5886 | plasma membrane | FLRT3, JAG1, AQP3, KITLG, ARRB1, DBN1, SLC6A8, JAG2, ABCA1 | $1.71 \times 10^{-1}$ |

(continued)

| GO-ID | Description | Genes in up-cassette | P-value |
|---|---|---|---|
| 5811 | lipid particle | ADFP | $1.71 \times 10^{-1}$ |
| 5606 | laminin-1 complex | LAMC1 | $1.71 \times 10^{-1}$ |
| 43256 | laminin complex | LAMC1 | $1.71 \times 10^{-1}$ |
| 5576 | extracellular region | FLRT3, LAMC1, JAG1, KITLG, INHBB, JAG2, ADFP, ADM | $1.71 \times 10^{-1}$ |
| 5853 | eukaryotic translation elongation factor 1 complex | EEF1A2 | $1.71 \times 10^{-1}$ |
| 5887 | integral to plasma membrane | FLRT3, JAG1, AQP3, SLC6A8, JAG2, ABCA1 | $1.76 \times 410^{-1}$ |
| 31226 | intrinsic to plasma membrane | FLRT3, JAG1, AQP3, SLC6A8, JAG2, ABCA1 | $1.76 \times 10^{-1}$ |

**Example 4: Clinical relevance of the cassettes of differentially expressed genes**

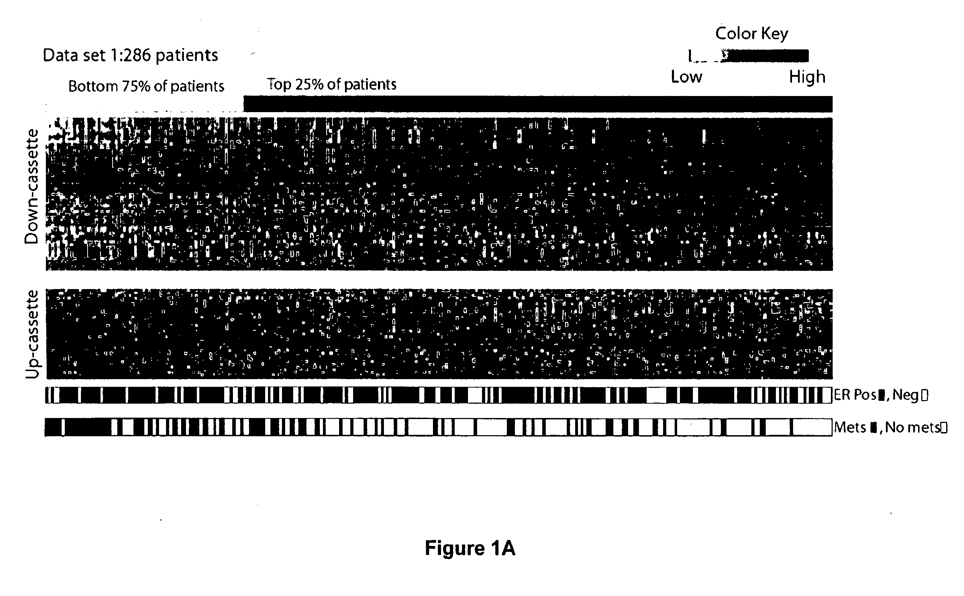

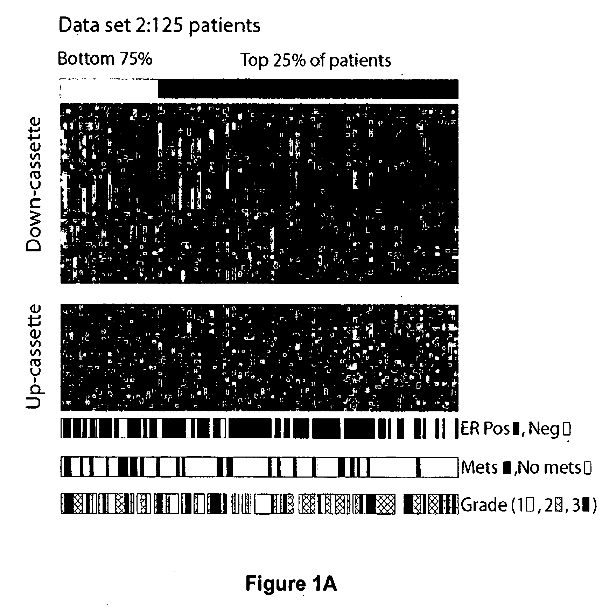

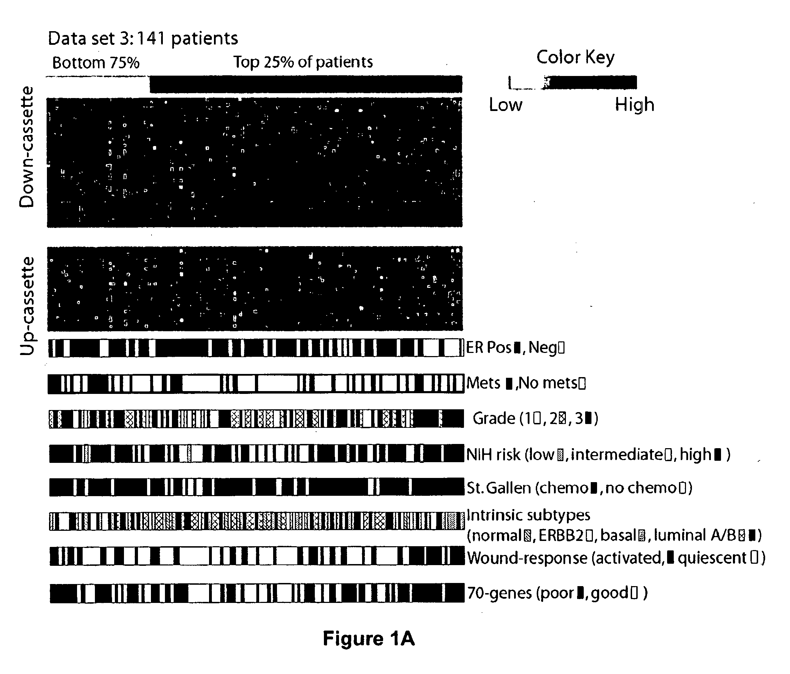

Consider a patient's down-cassette with a very *small* average expression value, while the corresponding up-cassette has a very *large* average expression value. It can be expected that this patient has a relatively bad clinical outcome because her individual profile corresponds to an aggressive phenotype. In contrast, another patient whose down-cassette has a *large* average expression value and the up-cassette has a *small* average expression value can be expected to have a relatively better prognosis. Hence, it can be speculated that the smaller the difference of *(average down-cassette) minus (average up-cassette),* the worse the prognosis. To test this hypothesis, Kaplan-Meier analyses were performed as follows. Figure 1A depicts heatmaps of tumor gene expression levels in data set 1 (Wang *et al*., 2005), data set 2 (Sotiriou *et al.,* 2006), and data set 3 (Chang *et al.,* 2005). The patients are ranked in increasing order based on the value of (average down-cassette) minus (average up-cassette).

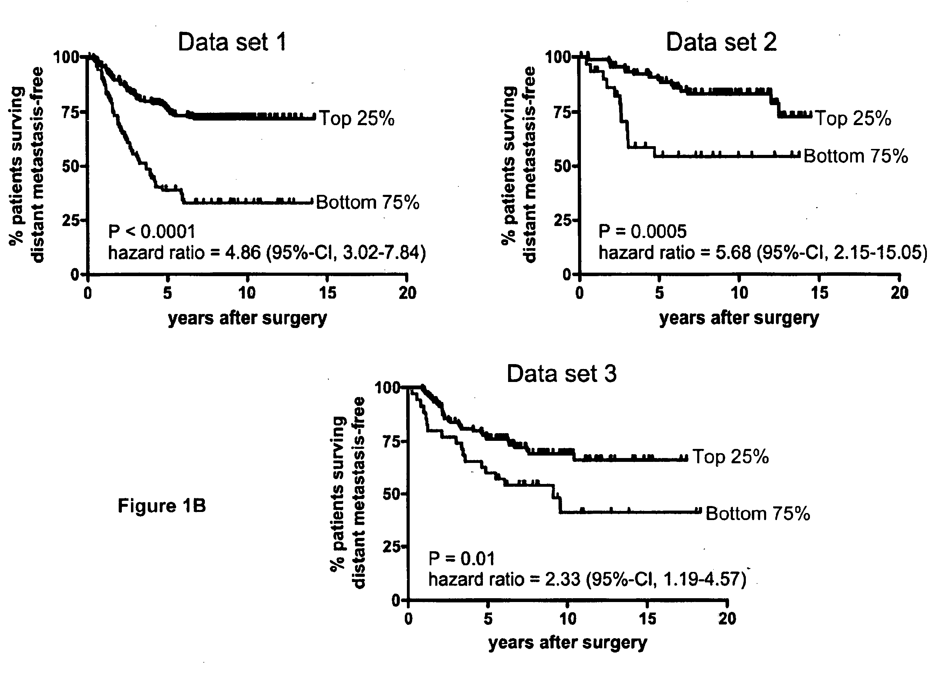

The clinical outcome of the patients at or above the 75th percentile was compared (i.e., the top 25% of patients, marked by the overhead darker, right hand side bar in Figure 1A) with the remaining patients (marked by the overhead lighter, left ahnd side bar) in each data set. Expression values are shaded, with lighter shading indicating lower and darker shading indicating higher values (see inset shading key, Figure 1A). Rows represent probe sets corresponding to down- or up-regulated genes in MCF7-I6 vs. MCF7-I0 (rows clustered based on complete hierarchical linkage). Columns represent tumours, ranked from left to right in increasing order based on (average expression value in the cassette of down-regulated genes) minus (average expression value in the cassette of up-regulated genes), short: avg(Down) - avg(Up). The bar termed Mets/No Mets, indicates the absence (light) or presence (dark) of distant metastases in the patients from which the tumours were obtained. The ER status of the patients is shown in the bar termed ER pos/neg (dark: ER+; light: ER-). For data sets 2 and 3, the tumor grade (1: well differentiated, 2: intermediate, 3: poorly differentiated) is shown in the bar termed Grade (1, 2, 3). Patients with tumors for which avg(Down) - avg(Up) is at or below the 75th percentile are one group, while patients above the 75th percentile are considered another group. The distant metastasis-free survival of patients in both groups is compared using Kaplan-Meier analysis (Figure 1 B).

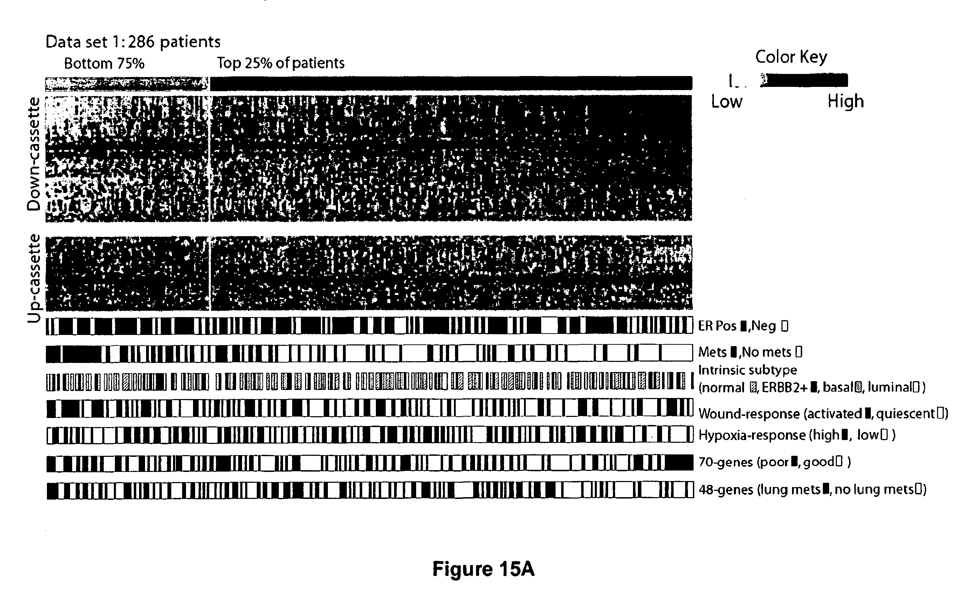

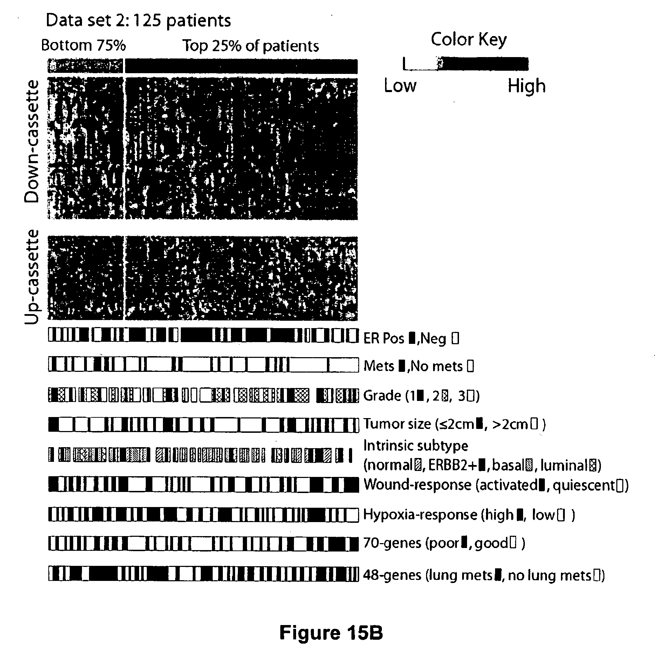

Figure 15 shows this ranking for the two learning sets (data sets 1 and 2 respectively). The clinical outcome of the patients at or below the 25th percentile (i.e., the 25% of patients with the smallest tandem score) was compared with the remaining patients. Predictions resulting from previously reported prognostic/predictive gene signatures were included: the 70-gene signature (referred to as 70-gene) by van't Veer *et al.,* the wound-response signature (referred to as wound-response) by Chang *et al.,* the hypoxia-response signature (referred to as hypoxia-response) by Chi *et al.,* the prognostic signature for lung metastases (referred to as 48-genes) by Minn *et al.,* and the genes of the intrinsic subtypes by Sørlie *et al.*

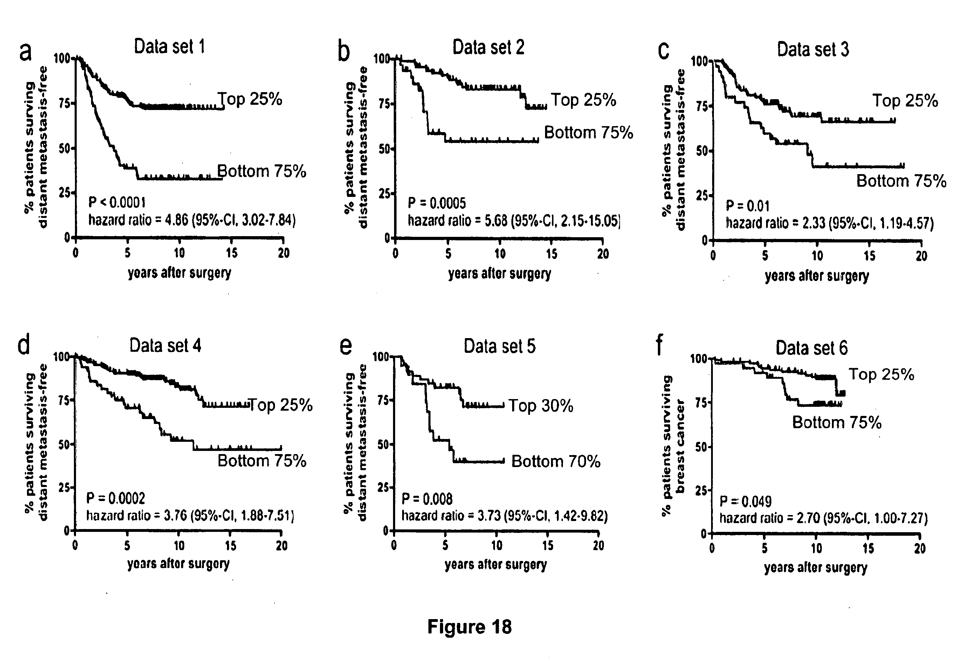

Referring to Figure 15, there is shown heatmaps of tumor gene expression levels in the learning sets. (Figure 15a) Data set 1 and (Figure 15b) data set 2. Expression values are shaded, with lighter shading indicating lower and darker shading indicating higher values (see inset shading key). Rows represent probe sets corresponding to down- or up-regulated genes in MCF7-I6 vs. MCF7-I0 (probe sets were clustered based on complete hierarchical linkage). Columns represent tumors, ranked from left to right in increasing order based on the tandem score. The bar termed Mets/No Mets indicates the absence (light) or presence (dark) of distant metastases in the patients from which the tumors were obtained. Established prognostic factors are shown as bar plots. Patients with tumors for which the tandem score is at or below the 25th percentile are one group (overhead lighter, left hand side bar), while patients above the 25th percentile are considered another group (overhead darker, right hand side bar). The distant metastasis-free survival of patients in both groups is compared using Kaplan-Meier analysis (see Figure 18a and b).

In all three data sets, a higher concentration of patients with metastases above the 75th percentile is observed. Kaplan-Meier analysis reveals a significantly different clinical outcome in all three data sets. Note that the patients in data set 1 with low expression values of the down-cassette and high expression values in the up-cassette have nearly a five-fold increased hazard of developing metastases than the remaining patients.

The MCF7 cell line is derived from a patient with positive estrogen receptor status, which could impact on the set of differently expressed genes. However, as can be seen in bar termed ER pos/neg below the heatmaps, there is no apparent association between the Estrogen Receptor (ER) status and the clinical outcome. The distribution of the ER+ and ER- patients in the respective groups in all three data sets was compared. In data set 1, the top 25% of patients with significantly worse clinical outcome comprise 58 ER+ and 14 ER- patients, while the remaining 75% of patients comprise 151 ER+ and 63 ER- patients. Based on Fisher's exact test, this is not a significant difference ($P = 0.54$). Similarly, there is no significant difference in the distribution of ER+ and ER- patients in data set 2 ($P = 0.74$) and data set 3 ($P = 0.88$). Therefore, the clinical outcome is independent of the ER status and the expression signature based on the down- and up-cassette is a predictor for both ER+ and ER-patients.

Due to the ranking based on avg(Down) - avg(Up), we observe that the heatmaps corresponding to the down-cassette are 'lighter' on the left and 'darker' on the right, whereas the heatmaps corresponding to the up-cassette are 'darker' on the left and 'lighter' on the right. Cases at the lefthand side correspond - with respect to the expression profile - to a more aggressive phenotype, as represented by 16, whereas cases at the right-hand side correspond to a less aggressive phenotype, as represented by 10.

In data set 1, a significant concentration of patients with distant metastases at or below the 25th percentile was observed, as compared to the remaining patients ($P = 7.35'10-9$, Fisher's exact test). In fact, when we consider the distribution of metastases across the data set, the correlation between the expression profiles and the presence/absence of distant metastases is highly significant ($P < 0.0001$, Wilcoxon rank-sum test). Across the entire data set, ER positive tumors tend to be concentrated towards the left ($P = 0.04$, Wilcoxon rank-sum test), but the lower 25th percentile does not contain significantly more ER positive tumors than the upper 75th percentile ($P = 0.12$, Fisher's exact test). In data set 1, patients at or below the 25th percentile have a significantly worse clinical outcome ($P < 0.0001$; log-rank test) with a nearly five-fold increased risk of developing distant metastases (hazard ratio 4.86; 95%-CI, 3.02-7.84). See Figure 1B.

In data set 2, we also observe a concentration of distant metastases towards the left ($P = 0.01$, Wilcoxon rank-sum test). The lower 25th percentile contains marginally more cases with distant metastases than the upper 75th percentile ($P = 0.05$, Fisher's exact test). There exists no significant correlation between the expression profiles and the distribution of ER positive and negative tumors across the entire data set ($P = 0.74$, Wilcoxon rank-sum test). The distribution of ER positive and negative tumors is not significantly different in the lower 25th and upper 75th percentile ($P = 0.38$, Fisher's exact test). Furthermore, there is no significant difference between the distribution of age or tumor size in the lower 25th and upper 75th percentile ($P = 0.34$ and $P = 0.55$, respectively, both based on Welch's $t$-statistics). Finally, there is no significant correlation between the tumor grade and the expression profiles ($P = 0.13$, Kruskal-Wallis test). In data set 2, the risk is nearly six-fold in patients at or below the 25th percentile ($P = 0.0005$, hazard ratio 5.68; 95%-CI, 2.15-15.05). See Figure 1B.

It was then investigated whether the gene set of the present invention, referred to as the tandem signature, could provide a prognostic tool for lymph node-negative breast cancer patients. The distribution of risk factors in the high- and low-risk groups was compared. The overall distribution of risk factors across the entire spectrum of samples was also compared. Tables 12 and 13 show the results for data set 1 and 2, respectively. As mentioned above, in data set 1, a marginally significant concentration of ER+ samples towards the left was observed ($P = 0.045$, Wilcoxon rank-sum test), i.e., a weak correlation with the tandem score. However, this could not be confirmed in data set 2. In data set 1, but not 2, the tandem score correlates positively ($P= 0.003$, Wilcoxon rank-sum test) with the predictions of the wound-response signature. In data set 2, but not 1, basal-like subtypes tend to be concentrated towards the left ($P = 0.001$, Wilcoxon rank-sum test), implying a correlation with the tandem score. Further, in data set 2, but not 1, the tandem score correlates ($P = 0.01$, Wilcoxon rank-sum test) with the hypoxia-response signature.

**Table 12.** Correlation with clinical risk factors and genomic signatures in data set 1 (Wang *et al.,* 2005) (*n* = 286). The *P*-value for the comparison between the lower 25th and the upper 75th percentile (72 vs. 214 patients) is based on Fisher's exact test; the P-value for the overall distribution is based on Wilcoxon rank sum test for binary covariates and Kruskal-Wallis test for covariates with more than two values in the lower 25th percentile and upper 75th percentile. All tests are two-sided and without adjustments for multiple testing, *p* < 0.05 is considered statistically significant and shown in bold face. Median time to follow-up refers only to patients without metastases.

| Covariate | At or below 25% | Above 25% | *P*-value (lower 25% vs. upper 75%) | *P*-value (overall distribution) |
|---|---|---|---|---|
| Metastases | 48 mets vs 24 no mets (median time to follow up of 9.1 years, range, 4.9-14.1) | 59 mets vs 155 no mets (median time to follow up of 8.7 years, range, 4.2-14.3) | $7.35 \times 10^{-9}$ (Fisher's) (P < 0.0001, log-rank) * | $2.67 \times 10^{-11}$ |
| ER (positive vs. negative) | S8 ER+ vs. 14 ER- | 151 ER+ vs. 63 ER- | 0.122 | 0.045 |
| Intrinsic subtypes (normal, ERBB2+, basal-like, luminal, unknown) | 9 basal-like, 16 ERBB2+, 9 luminal, 13 normal, 25 unknown | 37 basal-like, 35 ERBB2+, 22 luminal, 52 normal, 68 unknown | - | 0.635 |
| ERBB2 (positive vs. others) | 16 ERBB2+ vs. 56 others- | 35 ERBB2+ vs. 179 others | 0.287 | 0.731 |
| Basal subtype (basal-like vs. others) | 9 basal-like vs. 63 others | 37 basal-like vs. 177 others | 0.458 | 0.780 |
| Wound-response (activated vs. quiescent) | 44 activated vs. 28 quiescent | 96 activated vs. 118 quiescent | 0.020 | 0.003 |
| Hypoxia-response (high vs. low) | 37 high vs. 35 low | 108 high vs. 106 low | 1.00 | 0.221 |
| 70-gene signature (poor vs. good) | 40 poor vs. 32 good | 100 poor vs. 114 good | 0.221 | 0.521 |
| 48-gene signature (lung mets. vs. no lung mets.) | 38 LM vs. 34 no LM | 94 LM vs. 120 no LM | 0.219 | 0.008 |

* Log-rank *p*-values from Kaplan-Meier analysis are also reported, cf. Fig. 8a, main manuscript.

**Table 13.** Correlation with clinical risk factors and genomic signatures in data set 2 (Sotiriou *et al.*, 2006) (*n* = 125). The *P*-value for the comparison between the lower 25th and the upper 75th percentile (31 vs. 94 patients) is based on Fisher's exact test; the *P*-value for the overall distribution is based on Wilcoxon rank sum test for binary covariates and Kruskal-Wallis test for covariates with more than two values, and Welch's *t*-test for comparing the distributions of continuous values (age and tumor size) in the lower 25th percentile and upper 75th percentile. All tests are two-sided and without adjustments for multiple testing, *p* < 0.05 is considered statistically significant and shown in bold face. Median time to follow-up refers only to patients without metastases.

| Covariate | At or below 25% | Above 25% | *P*-value (lower 25% vs. upper 75%) | *P*-value (overall distribution) |
|---|---|---|---|---|
| Metastases | 12 mets vs. 19 no mets (median time to follow up of 7.3 years, range, 0.8-13.8) | 16 mets vs. 78 no mets (median time to follow up of 9.1 years, range, 0.2-14.5) | **0.023 (*P* = 0.0005, log-rank)*** | 0.014 |

(continued)

| Covariate | | At or below 25% | Above 25% | P-value (lower 25% vs. upper 75%) | P-value (overall distribution) |
|---|---|---|---|---|---|
| Tumor size (≤ 2cm vs. > 2cm) | | 20 tumors ≤ 2cm vs. 11 tumors > 2cm | 56 tumors ≤ 2cm vs. 38 tumors > 2cm | 0.676 | 0.775 |
| | Tumor size (diameter in cm) | - | - | 0.552 | - |
| Age (≤ 40 years vs. > 40 years) | | 7 patients S 40 years vs. 24 > 40 years | 9 patients ≤ 40 years vs. 85> 40 years | 0.070 | 0.292 |
| | Age (in years) | - | - | 0.338 | - |
| Grade (1, 2, 3 J | | - | - | - | 0.133 |
| | Grade (3 vs. 1 or 2) | 7 tumors grade 3 vs. 21 tumors grade 1 or 2 (grade of 3 tumors is NA) | 21 tumors grade 3 vs. 59 tumors grade 1 or 2 (grade of 14 tumors is NA) | 1.00 | 0.692 |
| ER (positive vs. negative) | | 19 ER+ vs. 11 ER- (1 NA) | 66 ER+ vs. 23 ER-{5 NA) | 0.380 | 0.740 |
| Intrinsic subtypes (normal, ERBB2+, basal, luminal, unknown) | | - | - | - | 0.003 |
| | ERBB2 (positive vs. others) | 6 ERBB2+ vs. 25 others | 15 ERBB2+ vs. 79 others | 0.782 | 0.257 |
| | Basal subtype (basal-like vs. others) | 11 basal-like vs. 20 others | 18 basal-like vs. 76 others | 0.085 | 0.001 |
| Wound-response (activated vs. quiescent) | | 19 activated vs. 12 quiescent | 40 activated vs. 54 others | 0.097 | 0.742 |
| Hypoxia-response (high vs. low) | | 20 high vs. 11 low | 51 high vs. 43 low | 0.404 | 0.010 |
| 70-gene signature (poor vs. good) | | 17 poor vs. 14 good | 39 poor vs, 55 good | 0.217 | 0.210 |
| 48-gene signature (lung mets. vs. no lung mets.) | | 24 LM vs. 7 no LM | 55 LM vs. 39 no LM | 0.085 | 0.213 |
| * Log-rank p-values from Kaplan-Meier analysis are also reported, cf. Fig. 8b, main manuscript. | | | | | |

Consider the patients at or above the 90% percentile (i.e., the 29 cases at the far right side of Figure 15a and the 13 patients at the far right of Figure 15b) - The expression profiles of these patients resemble more the weakly invasive phenotype MCF7-I0; thus, these patients are expected to have a relatively better clinical outcome. Interestingly, this is the case (see Tables 14 and 15) - in data set 1, only four (14%) patients developed metastases whereas 25 (86%) did not (median time to follow up of 8.3 years, range, 4.2-13.4). In contrast, of the remaining 257 patients below the 90% percentile, 103 (40%) developed metastases (median time to follow up of 8.8 years, range, 4.3-14.3). Thus, we observed a significantly ($P = 0.005$, two-sided Fisher's exact test) smaller proportion of metastatic tumors at or above the 90% percentile. The overall better clinical outcome is confirmed by Kaplan-Meier analysis ($P = 0.012$, log-rank test; hazard ratio 2.16; 95%-CI, 1.12-3.92). This observation is surprising, because the conventional risk factors for these patients might lead to a different prognosis: 14 (48%) of 29 are ER-, compared to 49 (19%) of the remaining 257 patients ($P = 0.002$; two-sided Fisher's exact test); 10 (34%) of 29 are ERBB2+, compared to 41 (16%) of the remaining 257 patients ($P = 0.020$; two-sided Fisher's exact test); 7 (24%) of 29 express a high hypoxia response, compared to 138 (54%) of the remaining 257 patients ($P = 0.003$; two-sided Fisher's exact test), and perhaps most surprisingly, 23 (79%) of 29 patients have a poor prognosis based on the 70-gene signature, compared to 117 (46%) of the remaining 257 patients ($P = 6.6$ ; two-sided Fisher's exact test).

**Table 14.** Risk factors for patients at or above the 90% vs. below 90% percentile in data set 1 (29 vs. 257 patients). *P*-values are based on two-sided Fisher's exact test without corrections for multiple testing. For the time to distant metastases, an additional *P*-value is reported based on Kaplan-Meier analysis (log-rank test). Median time to follow-up refers only to patients without metastases.

| Covariate | At or above 90% | Below 90% | *P*-value |
|---|---|---|---|
| Metastases | 4 mets vs 25 no mets (median time to follow up of 8.3 years, range, 4.2-13.4) | 103 mets vs. 154 no mets (median time to follow up of 8.8 years, range, 4.3-14.3) | 0.005 (Fisher's 0.012 (log-rank) |
| ER | 14 ER- vs. 15 ER+ | 49 ER- vs. 194 ER+ | 0.002 |
| ERBB2 | 10 ERBB2+ vs. 19 ERBB2 | 41 ERBB2+ vs. 216 ERBB2 | 0.020 |
| Basal-like | 5 basal-like vs. 24 non-basal-like | 41 basal-like vs. 216 non-basal-like | 0.793 |
| Wound-response | 14 activated vs. 15 quiescent | 126 activated vs.131 quiescent | 1.00 |
| Hypoxia-response | 7 high vs. 22 low | 138 high vs. 119 low | 0.003 |
| 70-gene signature | 23 poor vs. 6 good | 117 poor vs. 140 good | $6.6 \times 10^{-4}$ |
| 48-gene signature | 8 LM vs. 21 no LM | 124 LM vs. 133 no LM | 0.048 |

**Table 15.** Risk factors for patients at or above the 90% vs. below 90% percentile in data set 2 (13 vs. 112 patients). *P*-values are based on two-sided Fisher's exact test without corrections for multiple testing. For the time to distant metastases, an additional *P*-value is reported based on Kaplan-Meier analysis (log-rank test). Median time to follow-up refers only to patients without metastases.

| Covariate | At or above 90% | Below 90% | *P*-value |
|---|---|---|---|
| Metastases | 1 mets vs 12 no mets (median time to follow-up of 9.6 years; range, 2.0-12.8) | 27 mets vs. 85 no mets (median time to follow-up of 8.8 years, range, 0.17-14.5) | 0.294 (Fisher's) |
| | | | 0.172 (log-rank) |
| Tumor size | 5 tumors > 2cm vs. 8 tumors ≤ 2cm | 44 tumors > 2cm vs. 68 ≤ 2cm | 1.00 |
| Age | 3 ≤ 40 years vs. 10 > 40 years | 13 ≤ 40 years vs. 99 > 40 years | 0.372 |
| Grade | 4 tumors of grade 3 vs. 9 tumors not grade 3 | 24 tumors of grade 3 vs. 88 tumors not grade 3 | 0.485 |
| ER | 5 ER- vs. 7 ER+ | 27 ER- vs. 81 ER+ | 0.300 |
| ERBB2 | 3 ERBB2+vs. 10 ERBB2- | 18 ERBB2+ vs. 94 ERBB2- | 0.457 |
| Basal-like | 0 basal-like vs. 13 non-basal-like | 29 basal-like vs. 83 non-basal-like | 0.038 |
| Wound-response | 9 activated vs. 4 quiescent | 50 activated vs. 62 quiescent | 0.141 |
| Hypoxia-response | 3 high vs. 10 low | 68 high vs. 44 low | 0.016 |
| 70-gene signature | 12 poor vs. 1 good | 44 poor vs. 68 good | 0.0003 |
| 48-gene signature | 8 LM vs. 5 no LM | 71 LM vs. 41 no LM | 1.00 |

In data set 2 (Table 15), a similarly surprising observation was made. Of the 13 patients at or above the 90% percentile, 12 (92%) patients did not develop metastases (median time to follow-up of 9.6 years; range, 2.0-12.8). Again, a substantial proportion of these patients have high risk factors; specifically, the 70-gene signature predicts a poor prognosis for 12 of 13 (92%) patients ($P$ = 0.0003; two-sided Fisher's exact test).

In the independent test data set 3, we do not observe a strong concentration of cases with metastases towards the left (*P* = 0.09, Wilcoxon rank-sum test), but the lower 25th percentile contains significantly more metastases than the upper 75th percentile (*P* = 0.02, Fisher's exact test). Overall, ER positive cases tend to be concentrated towards the left (*P* = 0.02, Wilcoxon rank-sum test), but the distribution of ER positive and negative cases is not significantly different in the lower 25th and the upper 75th percentile (*P* = 1.0, Fisher's exact test). Furthermore, there is no significant difference between the distribution of age or tumor size in the lower 25th and the upper 75th percentile (P = 0.93 and P = 0.27, respectively; both based on Welch's t-statistics). Similarly, we failed to see any significant association between the tumors' differentiation and the expression profiles (*P* = 0.36, Kruskal-Wallis test).

In all three data sets, the expression profiles correlate significantly with the time-to-event (i.e., time to distant metastases, see Figure 1 B). Specifically, patients with a tumor whose expression profile corresponds to the aggressive phenotype I6 have a significantly poorer clinical outcome, with an increased hazard of developing metastases of 4.86 (95%-CI, 3.02-7.84) in data set 1, 5.68 (95%-CI, 2.15-15.05) in data set 2 and 2.33 (95%-CI, 1.19-4.57) in data set 3.

**Example 5. Comparison with genomic and clinical predictors of relative risk**

There exist several genomic signatures to assess a breast cancer patient's relative risk for developing distant metastases and to predict clinical outcome, and 'classic' clinical criteria such as the St. Gallen criteria or NIH risk. To address the question of whether our signature adds additional information, we focus on the test set because these results represent an independent validation. Based on clinical features, each patient's NIH risk is either *low, intermediate* or *high*. We do not observe any significant association between the expression profiles and the NIH risk (*P* = 0.81, Kruskal-Wallis test). Hence, the signature provides additional information beyond the NIH risk. Based on the St. Gallen criteria, each patient is recommended to either receive chemotherapy or not to receive chemotherapy. There exists no significant association between the expression profiles and the recommendation for chemotherapy (*P* = 0.31, Wilcoxon rank-sum test). Sørlie *et al* reported five intrinsic subtypes of breast cancer that are marked by different clinical outcomes, with a poor prognosis for patients with a luminal subtype. There exists no strong correlation between the Sørlie subtypes and the expression profiles (*P* = 0.11, Kruskal-Wallis test). Similarly, there is no association between the risk predicted by the wound-response signature (activated vs. quiescent) and the expression profiles (*P*= 0.10, Wilcoxon rank sum test). Specifically, there is no difference between the lower 25th percentile and the upper 75th percentile (*P* = 0.84, Fisher's exact test). Finally, there is no significant association (*P* = 0.59, Wilcoxon rank-sum test) between the expression profiles and the prediction (poor/good) based on the 70-gene predictor. Specifically, there is no difference in the distribution of good and poor prognosis cases in the lower 25th and the upper 75th percentile (*P* = 0.11, Fisher's exact test). Thus, our signature provides additional information beyond what can be inferred from the investigated predictors.

**Example 6. Predicting clinical outcome using the level of differential expression in MCF7-I0 and MCF7-I6**

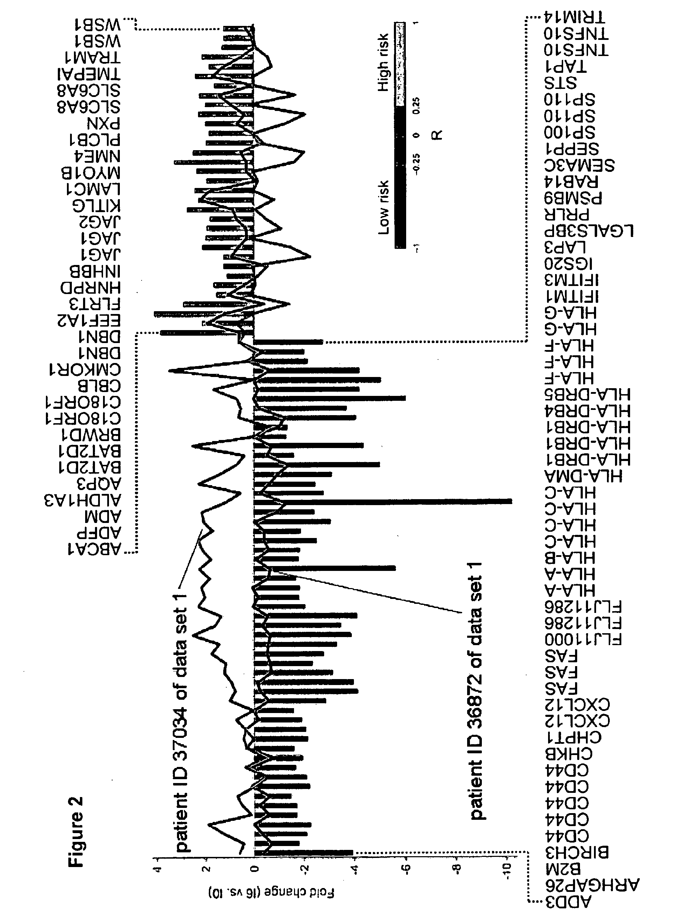

We speculated that the level of differential expression between MCF7-I0 and MCF7-I6 as reflected by the fold change contains additional information about the relative risk of developing distant metastases. To assess this hypothesis, we correlated the expression profiles of the patients with the vector of fold changes of our identified genes (Figure 2). To illustrate the idea, we superimposed the expression profile of two patients from data set 1. Following a similar approach described by van't Veer *et al.,* we decided to use the Pearson correlation coefficient to assess a patient's association with the aggressive phenotype MCF7-16. As a cut-off threshold value, we selected R = 0.25. This value corresponds to the upper 25th percentile of the patients in the largest data set; values of R >= 0.25 reflect a moderate to strong association with the aggressive phenotype, whereas values of R <= -0.25 reflect a moderate to strong association with the less aggressive phenotype.

Referring now to Figure 2, the black curve shows the normalized expression values of the corresponding probe sets of patient ID 36872 of data set 1. The Pearson correlation coefficient with the fold change is R = 0.71. This patient developed metastases after 7 months. The grey curve shows the normalized expression profile of patient ID 37034 of data set 1. The Pearson correlation coefficient with the fold change is R = -0.67. This patient did not develop metastases (last time to follow-up: 88 months).

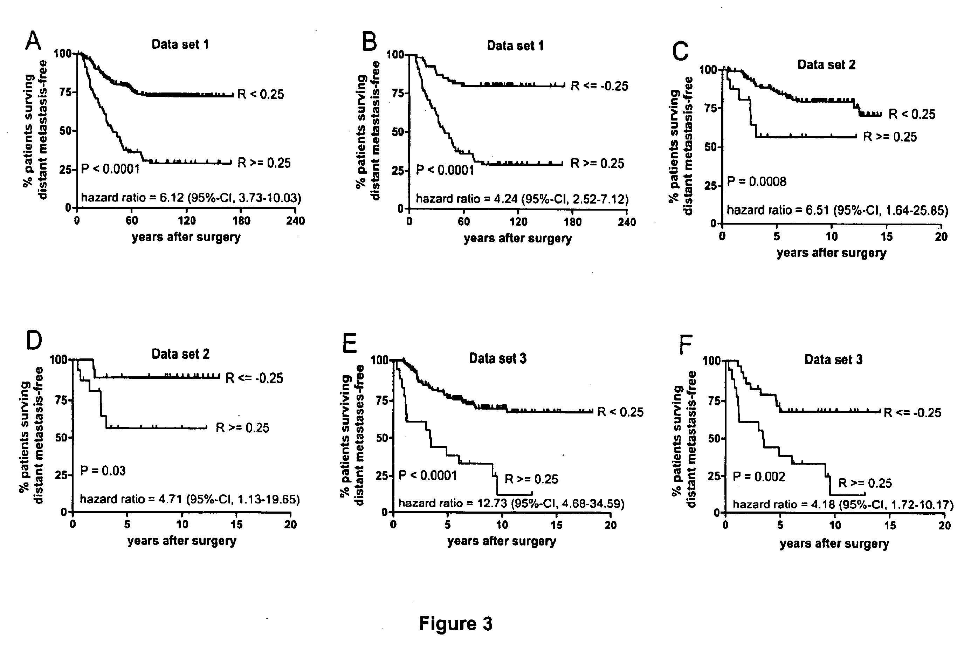

Figure 3 shows the resulting risk groups. Kaplan-Meier analysis for (A) data set 1; time to distant metastases is compared between patients whose expression profile correlates moderately or strongly with the fold-change signature (R $\geq$ 0.25), and the remaining patients whose expression profile correlates poorly (R < 0.25); (B) Data set 1; time to distant metastases is compared between patients whose expression profile correlates moderately or strongly with the fold-change signature (R $\geq$ 0.25), and the patients whose expression profile anti-correlates moderately or strongly with the fold=change signature (R $\leq$ -0.25); (B-F) analogous for data sets 2 and 3. Particularly for the test set (data set 3), we observe a remarkably high hazard ratio of almost 13 (Figure 3E). Consequently, our signature has a high predictive power with respect to the clinical outcome.

**Example 7. Combining predictors of clinical outcome**

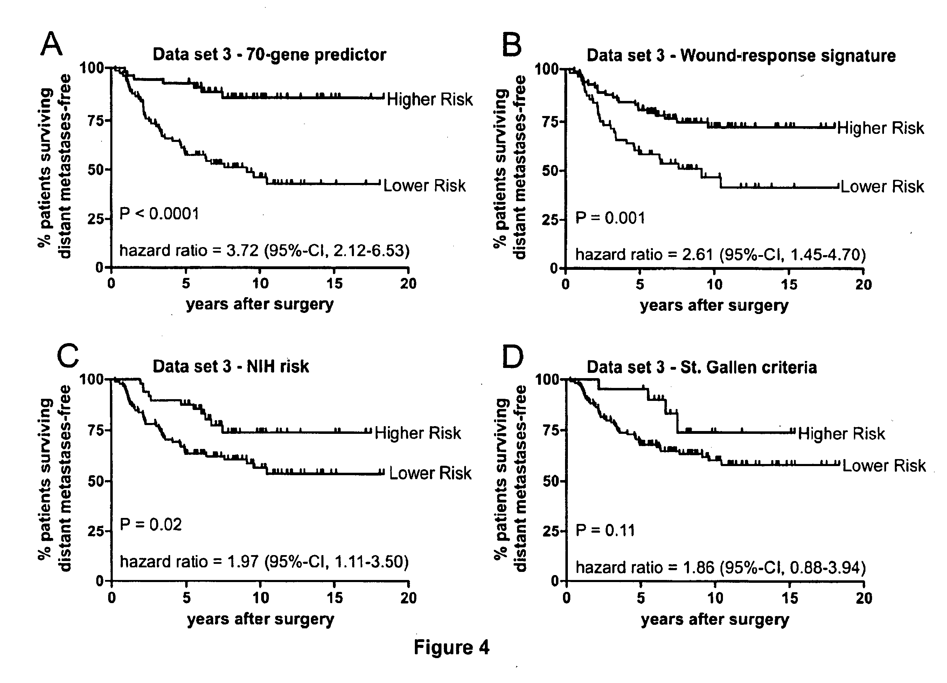

We compared the performance of our signature with the 70-gene predictor (Figure 4A), the wound-response signature (Figure 4B), the NIH risk (Figure 4C) and the St. Gallen criteria (Figure 4C). Figure 4 shows the resulting Kaplan-Meier curves that are obtained from the individual predictors for the test set.

Among the investigated predictors, the 70-gene predictor provides for the best risk group stratification with a hazard ratio of 3.72 (95%-CI, 2.12-6.53), which, however, is more than three times lower that the ratio obtained by our signature (hazard ratio 12.73, 95%-CI, 4.68-34.59), see Figures 3E and 4A. The gene signature of the present invention provides complementary information to the investigated predictors, and therefore, we might be able to derive an even more powerful tool by a fusion of the individual predictions.

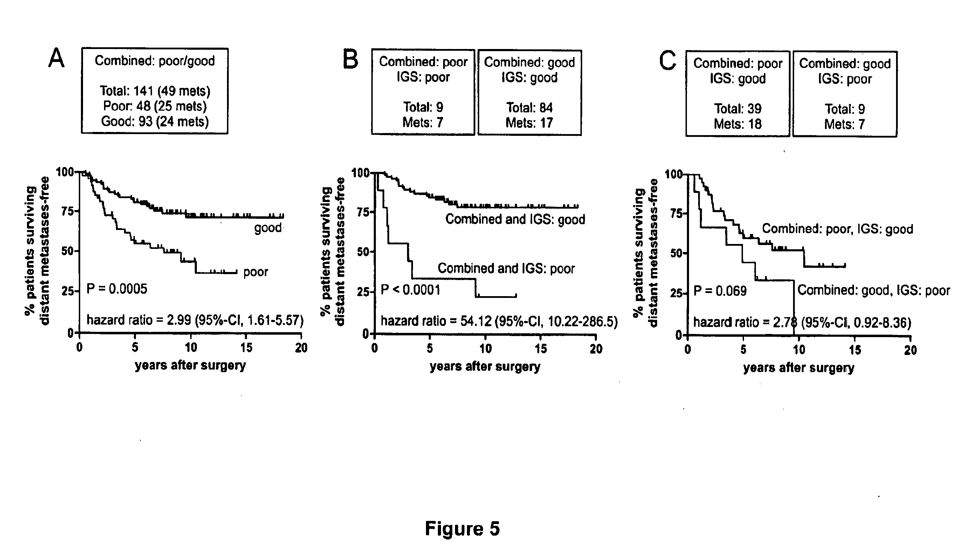

Referring to Figure 5, a simple combined predictor was constructed as follows: If a patient's risk is high based on NIH risk and St. Gallen criteria, and if the 70-gene predictor predicts a poor outcome and if a patient's wound-response signature is activated, then this patient's clinical outcome is considered to be poor, otherwise the patient's clinical outcome is considered to be *good.*

Figure 5A illustrates Kaplan-Meier curve for the patients predicted to have poor and good clinical outcome based on the combined predictor consisting of NIH risk, St. Gallen criteria, 70-gene signature and wound-response signature. Figure 5B illustrates the Kaplan-Meier curves for the patients predicted to have *poor* and *good* clinical outcome based on the agreement of the combined predictor and the invasiveness gene signature of the present invention (IGS). Agreement is achieved for 93 of 141 patients (9 poor and 84 good). Figure 5C shows Kaplan-Meier curves for the patients for whom the IGS and the combined predictor do not agree (48 patients). Based on this classification, 93 patients of the test set are predicted to have a good outcome and 48 are predicted to have a poor outcome. In total, there are 93 patients for whom this combined predictor agrees with our invasiveness signature (Figure 5B).

For the remaining 48 patients, the predictions based on our invasiveness gene signature (short, IGS) *disagree* with the combined predictor (Figure 5C). In Figure 5B, the hazard ratio is 54.12 (95%-CI, 10.22-286.5), indicating that, by integrating our signature IGS with the NIH risk, St. Gallen criteria, 70-gene signature and the wound-response signature, we can derive an even more powerful prognostic tool. Here, all individual predictors agree for 93 patients; 9 patients are predicted to have a poor outcome and 7 of these develop metastases relatively early (median, 1.22 years; range, 0.27-9.12). Of the remaining 84 patients for whom the predictors agree (outcome: good), only 17 develop distant metastases. More interestingly, perhaps, are the results depicted in Figure 5C. Thirty-nine patients are predicted to have a poor prognosis based on the combined predictor, whereas our signature predicts a good outcome for these patients. Of these 39 patients, only 18 developed metastases, whereas the remaining 21 did not (median time to follow-up, 8.17 years; range, 1.78-14.13). For nine patients, the combined predictor predicts a good outcome, whereas our signature disagrees. Seven of these patients developed metastases, and relatively early, with a median time to metastases of 3.47 years (range, 0.57-9.57).

It was further investigated whether there exist significant differences in the distribution of age, node size, and tumor grade in the top 25% patients, compared to the remaining 75% patients. No significant differences were seen. Therefore, it was concluded that the observed differences in clinical outcome are associated with the different expression profiles.

The signature provided by the down- and up-cassette is of clinical prognostic relevance for risk group stratification of breast cancer patients, regardless of estrogen receptor status or histopathological parameters. Liu *et al.* (2007) recently reported an invasiveness gene signature (IGS) with prognostic relevance in various types of cancer. This 186-gene signature, however, is derived from a comparison of tumorigenic breast cancer cells, with normal breast epithelial cells, and thus may not reflect key regulators of invasion and metastases. The IGS does not contain a substantial number of genes known to be involved in invasiveness. Accordingly, the present invention provides robust means for prospectively predicting the metastatic likelihood, and thereby, the likely clinical outcome of breast cancer patients, based on the genotype of the patient, in particular, by determining the relative expression level of a set of genes associated with invasiveness.

**Example 8. MCF7-I6 cells are more motile than parental MCF7-I0 cells *in vitro***

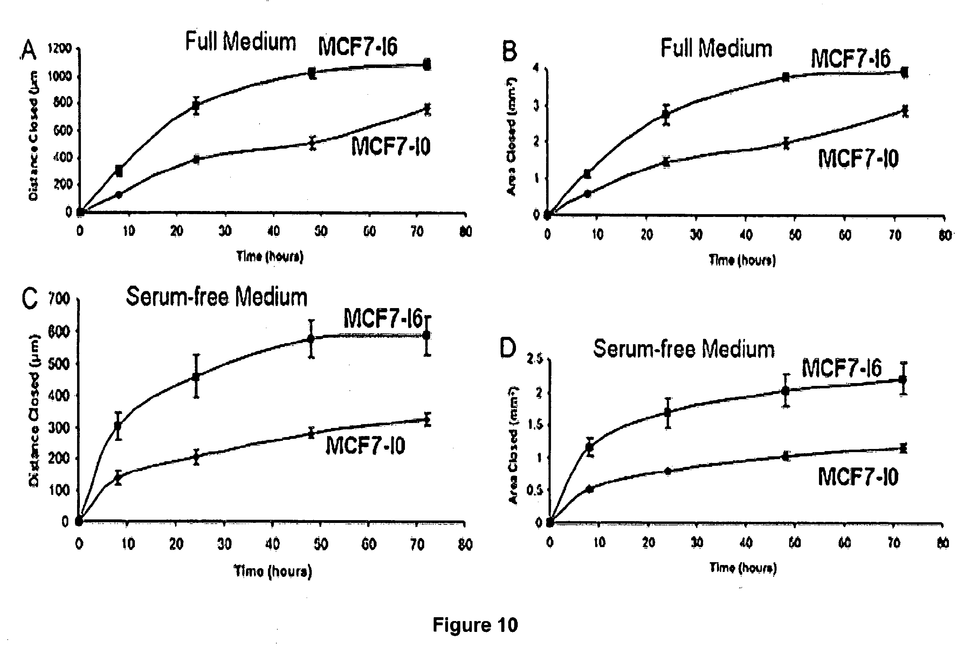

The motility of the parental MCF7-10 and the daughter MCF7-I6 cell populations was assessed using wound scrape assays. The experiments were performed both with and without serum in the medium to confirm that the difference in rate of closure is due to motility rather than cell proliferation. The rate of closure was assessed by measuring the distance at five points per field of view and also by measuring the overall area using NIS Elements software. Referring to Figure 10, at each time point, MCF7-I6 cells closed the wound significantly faster than the parental MCF7-I0 cells. Wound scrape assays for MCF7-I0 and MCF7-I6 cells were conducted in full medium (Figures 10a and b) and serum-free medium (Figures 10c and d). The wound was measured both by distance closed (10a and 10c) and area closed (10b and 10d). At each time point, five measurements were taken, and three replicates were used. The assays were performed in triplicate. Shown are mean values with 84%-confidence intervals indicated by vertical bars. Non-overlapping intervals correspond to approximate pairwise significance tests at alpha = 0.05 for differences between mean values at each time point. Statistical significance was confirmed by ANOVA ($P < 0.001$) for both full medium and serum-free conditions.

**Example 9. MCF7-I6 cells have undergone a partial epithelial to mesenchymal transition and are less adhesive to extracellular matrix components**

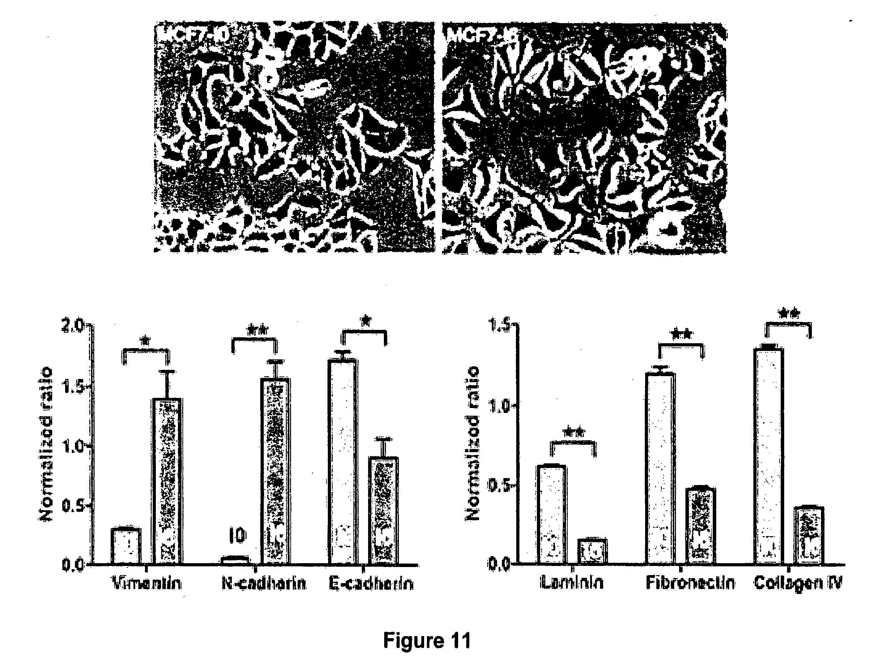

As seen in Figure 11A, morphologically, the MCF7-I6 cells appeared more mesenchymal-like, exhibiting spindle-shaped morphology with visible filopodia extending from the surface of the cells, compared to the parental MCF7-I0 cells grown under the same conditions. Figure 11A shows the comparison of the MCF7-I0 and MCF7-I6, showing the more spindle-shaped morphology in the MCF7-I6 cells.

E-cadherin and vimentin mRNA expression was assessed and relatively quantified by qRT-PCR and revealed a significant difference between the MCF7-I0 and MCF7-I6 cell lines. Referring to Figure 11B, the mesenchymal markers vimentin and N-cadherin were up-regulated 4.7-fold and 27.5-fold, respectively, in the MCF7-I6 cells. In contrast, the epithelial marker E-cadherin was down-regulated 1.9-fold in the MCF7-I6 cells. mRNA expression by qRT-PCR revealed a significant overexpression of vimentin ($P$ = 0.04; two-sided, unequal variance *t*-test) and N-cadherin ($P$ = 0.009) in MCF7-I6, and a significant under-expression of E-cadherin ($P$ = 0.02) in MCF7-I6, see Figure 11B. Adhesion to extracellular components - laminin, fibronectin and collagen IV - were assessed using CytoMatrix screening kit (Chemicon). MCF7-I6 cells show significantly less adhesion to laminin ($P$ = 0.0008), fibronectin ($P$ = 0.0012) and collagen IV ($P$ = 0.0006), see Figure 11C. p-values were corrected for multiple testing using Holm's method. All data are mean $\pm$ s.e.m. for three experiments. *, $P < 0.05$; **, $P < 0.01$. MCF7-I6 cells exhibited significantly ($P < 0.0001$, ANOVA) less adhesion to all three extracellular matrix components tested compared to the parental MCF7-I0 cells (Figure 11C). The adhesion to collagen IV was 3.7-fold lower in MCF7-16, adhesion to laminin was 4-fold lower, and adhesion to fibronectin was 2.5-fold lower.

**Example 10. MCF7-I6 cells have a diminished interferon-gamma response**

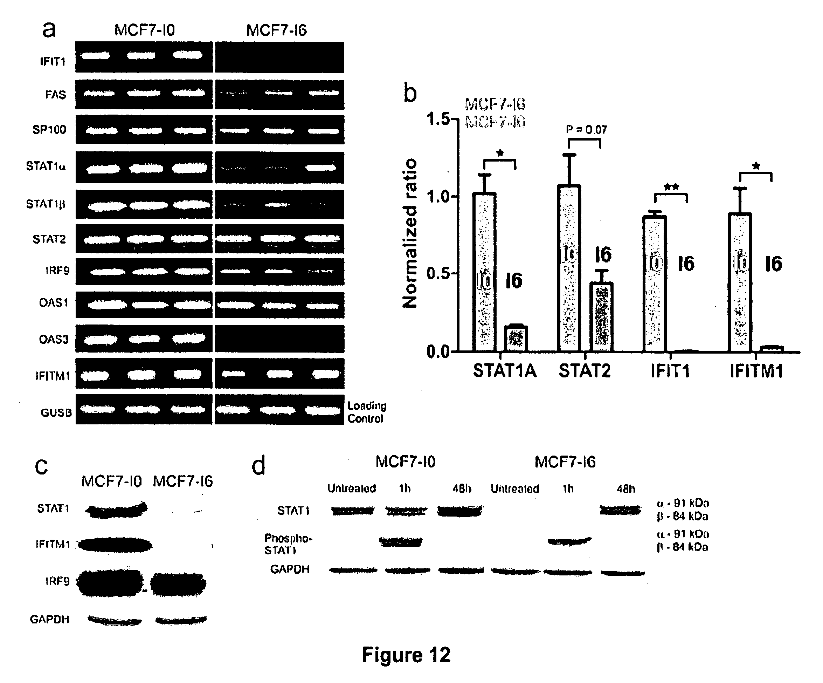

Figure 12A illustrates a significant down-regulation of interferon-induced and immune-response genes ($P$ = $2.52\times10$) in the MCF7-I6 cells.

mRNA expression of interferon-induced genes was investigated by (Figure 12a) semiquantitative PCR and (Figure 12b) quantitative PCR, validating the microarray results and showing a down-regulation in many IFN regulated genes (STAT1A, $P$ = 0.02; STAT2, $P$ = 0.07; IFIT1, $P$ = 0.001; IFITM1, $P$ = 0.03. Two-sided, unequal variance t-tests for individual 5 comparisons). Figure 12c shows Western blot analysis of interferon induced genes STAT1, IFITM1 and IRF9 showing these are also down-regulated at the protein level in the hyper-invasive MCF7-I6 cells compared to the parental MCF7-I0 cells. Figure 12d shows Western blot analysis of STAT1 upon induction by 100 ng/ml IFN-gamma after 1hr and 48hr. Active Phospho-STAT1 is induced 1hr after treatment in both the MCF7-I0 and MCF7-I6 cells but to a lesser extent in the MCF7-I6 cells. Expression of STAT1 protein is induced 48hr after treatment in both the MCF7-I0 and MCF7-I6 cells, but again to a lesser extent in the MCF7-I6 cells. Referring to Figures 12a and 12b, STAT1-alpha, STAT2, IFIT1, and IFITM1 mRNA expression were subsequently quantified by qRT-PCR corroborating the RT-PCR and microarray results, showing significant down-regulation of these genes in the MCF7-I6 cells ($P< 0.0001$, ANOVA). The down-regulation of the interferon-induced genes STAT1, IFITM1, and IRF9 were also assessed at the protein level by Western blotting and were all down-regulated in the MCF7-I6 cells compared to the parental MCF7-I0 cells (Figure 12c). Protein expression of STAT1 and phospho-STAT1 following IFN-gamma treatment was further assessed by Western blot analysis (Figure 12d). Both STAT1 alpha and beta isoforms are down-regulated in the MCF7-16 cells in the untreated samples. Phospho-STAT1 is induced after 1h treatment in both populations but to a lesser extent in the MCF7-16 cells. Similarly, after 48h exposure to IFN-gamma, both STAT1 alpha and beta isoforms are upregulated in both populations, but again to a lesser extent in the MCF7-I6 cells.

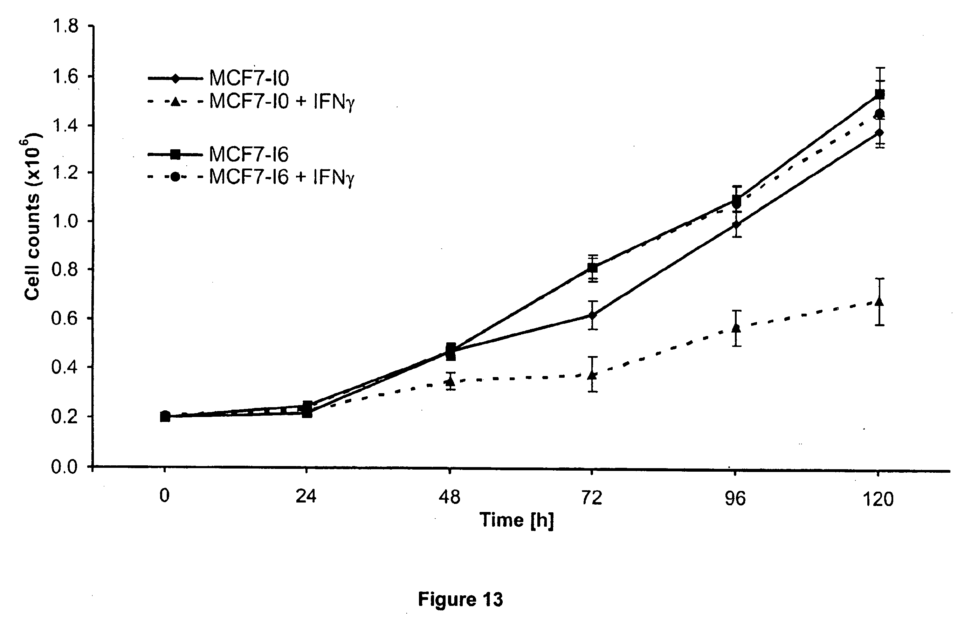

Figure 13 shows growth curves for MCF7-I0 and MCF7-I6 cells in the presence (dotted curves) and absence (solid curves) of 100 ng/ml IFN -gamma over a period of 6 days. IFN-gamma has a significant effect on the growth curve of MCF7-10 ($P < 0.0001$, two-way ANOVA with 15 repeated measures); after 72h, the effect becomes significant ($P < 0.01$; Bonferroni post-hoc test). In contrast, IFN-gamma has no effect on the growth of MCF7-I6 cells ($P$ = 0.96, two-way ANOVA with repeated measurements). Data shown are mean for eight replicates per day $\pm$ s.e.m. Referring to Figure 13, the effect of IFN-gamma on growth of MCF7-I0 and MCF-7-I6 cells was assessed over a six-day period. IFN-gamma inhibited growth of the MCF7-I0 cells significantly ($P < 0.0001$, ANOVA), extending their doubling time from 36h to 66h. However, IFN-gamma did not have any significant ($P$ = 0.96, ANOVA) effect on the growth of the MCF7-I6 cells with doubling time of 26h for cells under normal growth conditions and 27h in the presence of IFN-gamma. This suggests that the weakly-invasive parental MCF7-I0 cells are sensitive to IFN-gamma induced apoptosis whereas the hyper-invasive MCF7-I6 cells are resistant.

**Example 11. Prognostic power of the tandem signature in multi-center validation sets**

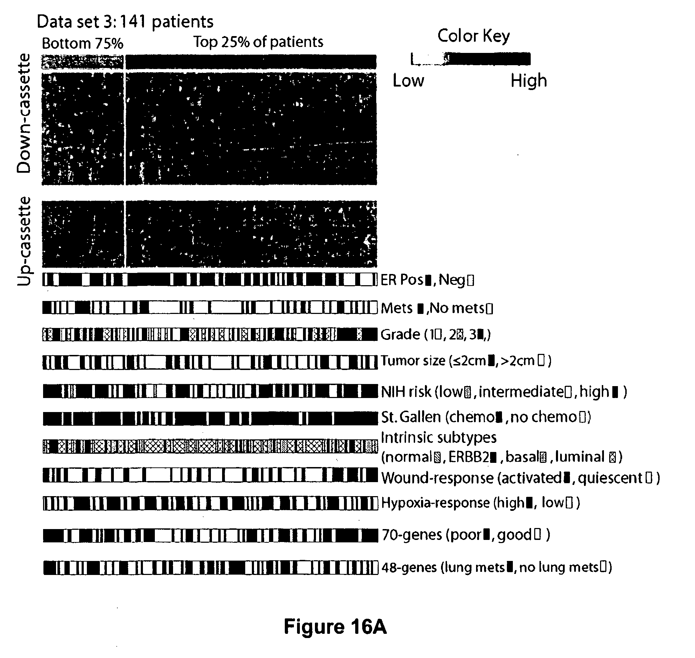

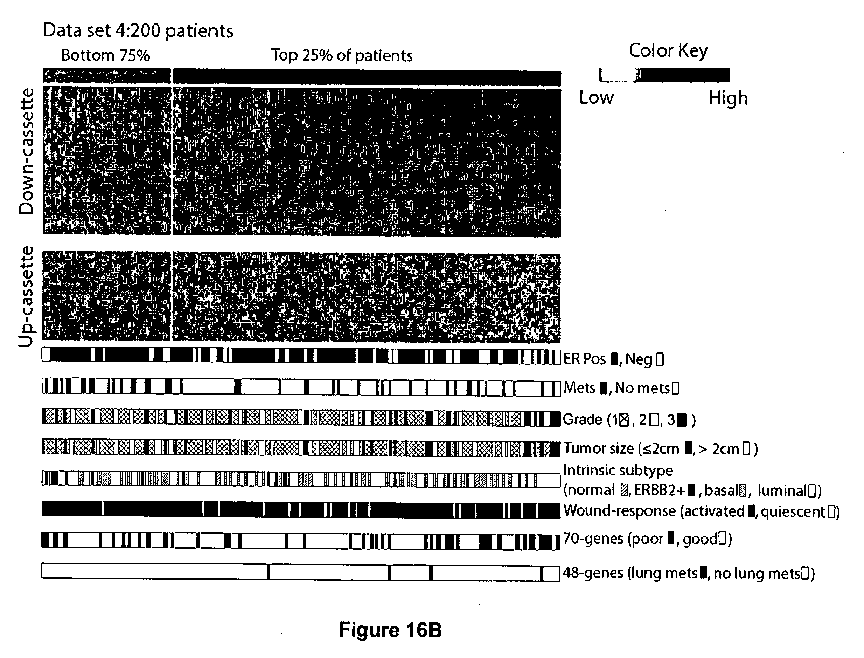

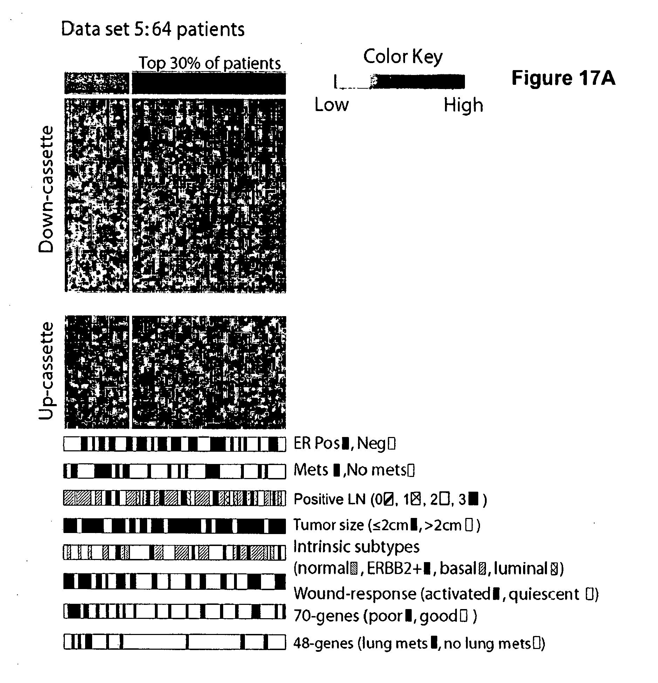

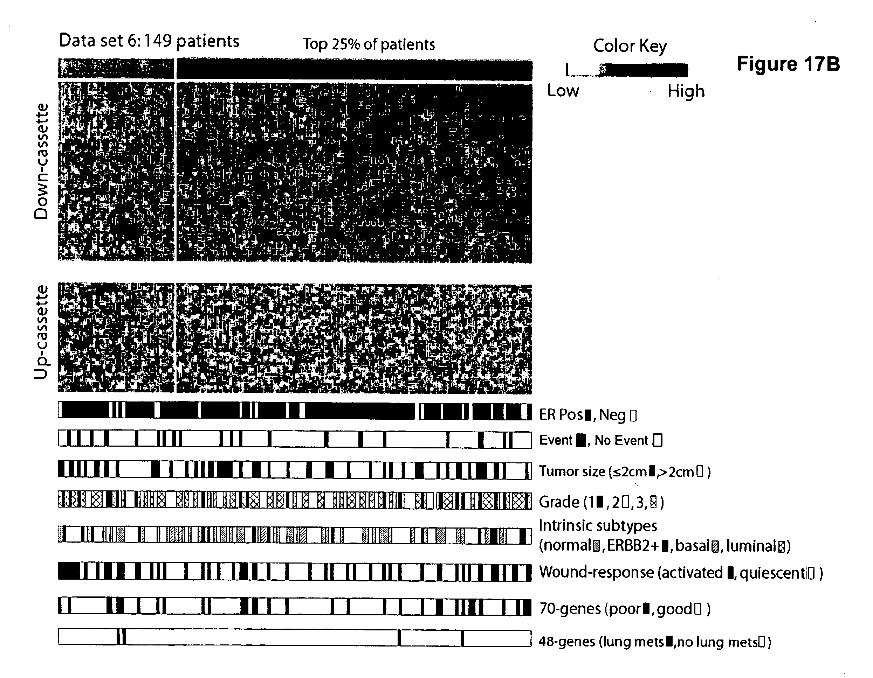

The gene set of the present invention ("tandem signature") was validated using four independent, multi-center data sets (Table 16). The patient cohorts of data sets 3 and 4 contain only lymph node-negative (LNN) samples for patients who did not receive hormonal or chemotherapy. To investigate the prognostic power of the tandem signature for cases with early lymph node involvement, we included data set 5 (64 samples, 28 LNN, 15 LN1+, 9 LN2+, 12 LN3+). To investigate whether the tandem signature is not only prognostic for time to distant metastases, we included data set 6 (149 LNN cases) and considered time to death from breast cancer as endpoint. We analyzed the validation sets as described for the learning sets. Figures 16a and b show the resulting heatmaps for data sets 3 and 4, respectively; and Figures 17a and b show the resulting heatmaps for data sets 5 and 6, respectively.

**Table 16.** Synopsis of the publicly available data sets. Data in italics were not available from the indicated URL and therefore estimated from gene expression data (as described below).

| | Learning sets | | Test sets | | | |
|---|---|---|---|---|---|---|
| | Data set 1 | Data set 2 | Data set 3 | Data set 4 | Data set 5 | Data set 6 |
| # of patients | 286 | 125 | 141 | 200 | 64 | 149 |
| **Age** | | | | | | |
| Mean (SD) | 54 (12) | 52 (10) | 43 (6) | - | 56 (14) | 63 (13) |
| ≤ 40 | 36 (13%) | 16 (13%) | 44 (31%) | - | 9(14%) | 11 (7%) |
| 41-55 | 129 (45%) | 57 (46%) | 97 (69%) | - | 23 (36%) | 31 (21%) |
| 56-70 | 89 (31%) | 49 (39%) | - | - | 19 (30%) | 59 (40%) |
| > 70 | 32 (11%) | 3 (2%) | - | - | 13 (20%) | 48 (32%) |
| **Grade** | | | | | | |
| 3 (poor) | 148 (52%) | 28 (22%) | 66 (47%) | 35 (18%) | - | 22 (15%) |
| 2 (moderate) | 42 (15%) | 48 (38%) | 42 (30%) | 136 (68%) | - | 75 (50%) |
| 1 (good) | 7 (2%) | 32 (26%) | 33 (23%) | 29 (14%) | - | 51 (34%) |
| Unknown | 89 (31%) | 17 (14%) | - | - | - | 1 (1%) |
| **Tumor size** | | | | | | |
| ≤ 2cm | - | - | 79 (56%) | 112 (56%) | 11 (17%) | 92 (62%) |
| > 2cm | - | - | 62 (44%) | 88 (4496) | 53 (83%) | 57 (38%) |
| Unknown | - | - | - | - | - | - |
| **Lymph node status (at start of census)** | | | | | | |
| Positive | 0 (0%) | 0 (0%) | 0 (0%) | 0 (0%) | 36 * (56%) | 0 (0%) |
| Negative | 286 (100%) | 125 (100%) | 141 (100%) | 200 (100%) | 28 (44%) | 149 (100%) |
| **ER status** | | | | | | |
| Positive | 209 (73%) | 85 (68%) | 104 (74%) | *156 (78%)* | 34 (53%) | 127 (85%) |
| Negative | 77 (27%) | 34 (27%) | 37 (26%) | *44 (22%)* | 30 (47%) | 19 (13%) |
| Unknown | -11 | 6 (5%) | - | - | - | 3 (2%) |
| **PR status** | | | | | | |
| Positive | - | - | - | | 40 (63%) | 31 (21%) |
| Negative | - | - | - | | 24 (37%) | 118 (79%) |

(continued)

| Metastases within 5 years (data sets 1-5) or death from breast cancer within 5 years (data set 6) | | | | | | |
|---|---|---|---|---|---|
| Yes | 93 (33%) | 21(17%) | 39 (28%) | 28 (14%) | 17 (27%) | 9 (6%) |
| No | 183 (64%) | 86 (69%) | 97 (69%) | 153 (77%) | 42 (66%) | 133 (89%) |
| Censored | 10 (3%) | 18 (14%) | 5 (4%) | 19 (9%) | 5 (7%) | 7 (5%) |
| **Intrinsic subtype (Sørlie *et al.*, 2001)** | | | | | | |
| Normal | *65 (23%)* | *30 (24%)* | 10 (7%) | *36 (18%)* | *3 (5%)* | *22 (15%)* |
| ERBB2+ | *51 (18%)* | *21 (17%)* | 25 (18%) | *16 (8%)* | *8 (13%)* | *15 (10%)* |
| Basal-like | *46 (16%)* | *29 (23%)* | 23 (16%) | *48 (24%)* | *23 (36%)* | *35 (24%)* |
| Luminal | *31 (11%)* | *17 (14%)* | 83 (59%) | *0 (0%)* | *0 (0%)* | *0 (0%)* |
| Unknown | *93 (32%)* | *28 (22%)* | - | *100 (500%)* | *30 (46%)* | *77 (51%)* |
| **Wound-response signature (Chang *et al.*, 2005)[1]** | | | | | | |
| Activated | 140 (49%) | 59 (47%) | 58 (41%) | 182 (91%) | 32 (50%) | 57 (38%) |
| Quiescent | *146 (51%)* | *66 (53%)* | 83 (59%) | *18 (9%)* | *32 (50%)* | *92 (62%)* |
| **Hypoxia-response (Chi *et al.*, 2005)** | | | | | | |
| High | *145 (51%)* | *71 (57%)* | *84 (60%)* | *200 (100%)* | *64 (100%)* | *149 (100%)* |
| Low | *141 (49%)* | *54 (43%)* | *57 (40%)* | *0 (0%)* | *0 (0%)* | *0 (0%)* |
| **70-gene signature (van't Veer *et al.*, 2002)** | | | | | | |
| Poor | *140 (49%)* | *56 (45%)* | *84 (60%)* | *142 (71%)* | *22 (34%)* | *37(25%)* |
| Good | *146 (51%)* | *69 (55%)* | *57 (40%)* | *58 (29%)* | *42 (66%)* | *112 (75%)* |
| **Lung metastases signature (Minn *et al.*, 2005)** | | | | | | |
| Lung mets | *132 (46%)* | *79 (63%)* | *75 (53%)* | *4 (2%)* | *9 (14%)* | *4 (396)* |
| No long mets | *154 (54%)* | *46 (37%)* | *66 (47%)* | *196 (98%)* | *55 (86%)* | *145 (97%)* |
| **Other** | | | | | | |
| Platform Reference(s) <br><br> Available at | HG-U133A Wang *et al.* (2006) GEO: GSE2034 | HG-U133A Sotiriou *et al.* (2005) GEO: GSE2990 | Rosetta Hu25k van't Veer *et al.* (2002); Chang *et al.* (2005) http://microarray-pubs.stanford.edu/wound_NKI/explore.ht ml | HG-U133A Schmidt et *al.* (2008) GEO: GSE11121 | HG-U133A Minn *et al.* (2005) GEO: GSE2603 | HG-U 133A Miller *et. al.* (2005) GEO: GSE1379 |
| * Of 36 lymph node-positive cases, 15 cases have 1 positive node, 9 cases have 2 positive nodes, and 12 have 3 positive nodes. On average, 20 lymph nodes were assessed per patient (range, 2-37).<br>† The ER status of the patients in data set 4 was not available; therefore, it was derived based on gene expression analysis as described below. | | | | | | |

We analyzed six publicly available microarray data sets of predominantly lymph node negative (LNN) patients. As the largest data set (data set 1, 286 patients) contains only LNN patients who did not receive hormonal or chemotherapy, we selected a similar cohort of patients from data sets 2 and 3. Data set 4 contains exclusively LNN patients. Data set 5 contains samples from LNN patients and patients with a maximum of three positive lymph nodes. In data sets 1-5, time to distant metastases is the primary clinical endpoint. In data set 6, 'time to death from breast cancer' is the endpoint. Table 16 shows a synopsis. We used data sets 1 and 2 as learning sets. Data sets 3, 4, 5 and 6 were used as test sets for independent, cross-platform and multi-center validation. From the publicly available repositories, the microarray data sets were downloaded in the normalized formats as described in the original studies (e.g., series files with normalized signal values based on Affymetrix MAS 5.0 or Robust Multichip Average, RMA). We performed only minor additional pre-processing such as log2-transformation and median-centering of arrays. For data set 5, we downloaded the raw data and performed RMA normalization using the function rma of the R package affy (R Development Core Team, 2008). Note, that some data sets have incomplete clinical data because this information was not available from the public repositories. Some of the missing information was derived from the gene expression data, such as the estrogen receptor status for data set 4 and the intrinsic subtypes for data sets 1, 2, 4, 5 and 6 (data in italics in Table 16). However, no additional wet lab experiments were performed to confirm these results. Furthermore, note the differences in tumor grade and patient age between the cohorts.

Figure 18 shows Kaplan-Meier analysis of time to event in the training sets, (Figure 18a) data set 1 (n = 286) and (Figure 18b) data set 2 (*n* = 125), and in the validation sets, (Figure 18c) data set 3 (*n* = 141), (Figure 18d) data set 4 (*n* = 200), (Figure 18e) data set 5 (*n* = 64) and (f) data set 6 (*n* = 125). Compared are patients at or below the 25th percentile of the tandem score (upper, darker curve) and patients above the 25th percentile (lower, lighter curve) in data sets 1, 2, 3, 4 and 6. Due to the small number of samples in data set 5, patients at or below the 30th percentile of the tandem score (i.e., 19 patients, green curve) are compared with patients above the 30th percentile (i.e., 45 patients, red curve). All *p*-values are based on logrank test. In data sets 1, 2, 3, 4, and 5, the event is distant metastases (any site). In data set 6, the event is death from breast cancer.

For all validation sets, we observed that the risk group stratification based on the tandem score is statistically significant. The different clinical outcome is most pronounced in data sets 3 and 4 (Figures 18c and d). Here, a 2.3-fold and 3.8-fold increased risk, respectively, of developing distant metastases for tumors that express the tandem signature, is observed. The results are confirmed in data set 5 (Fig. 18e), which contains tumors with a small number of positive lymph nodes. In contrast, in data set 6, we observed only a marginally significant ($P$ = 0.049, log-rank test) difference between the two risk groups. Here, the endpoint is time to death from breast cancer, not time to distant metastases. The tandem signature therefore seems to be a prognostic factor for time to distant metastases.

## Example 12. Correlation of the tandem signature with other risk factors in the validation sets

In data set 3, we observed a statistically significant correlation between the tandem score and the intrinsic subtypes (Table 17). Tumors expressing ERBB2 are more prevalent in the lower 25th percentile ($P$ = 0.005, Fisher's exact test). Interestingly, however, basal-like tumors tend to be concentrated towards 5 the right side of the heatmap in Figure 16a ($P$ = 0.011; Wilcoxon rank sum test). Above the 90% percentile (i.e., 14 patients at the right-hand side of Figure 6a), we even see a significant ($P$ = 0.012, Fisher's exact test) concentration of basal-like tumors (6 of 14 vs. 17 of 127 below the 90% percentile - see Table 18).

Figure 16 shows heatmaps of tumor gene expression levels in the validation sets, (Figure 16a) data set 3 and (Figure 16b) data set 4. Expression values are shaded, with lighter shading indicating lower and darker shading indicating higher values (see inset shading key). Rows represent probe sets corresponding to down- or upregulated genes in MCF7-I6 vs. MCF7-I0 (probe sets were clustered based on complete hierarchical linkage). Columns represent tumors, ranked from left to right in increasing order based on the tandem score. The bar termed Mets/NoMets indicates the absence (light) or presence (dark) of distant metastases in the patients from which the tumors were obtained. Established prognostic factors are shown as bar plots. Patients with tumors for which the tandem score is at or below the 25th percentile are one group (overhead, left hand bar), while patients above the 25th percentile are considered another group (overhead, right hand bar). The distant metastasis-free survival of patients in both groups is compared using Kaplan-Meier analysis (see Figure 18c and d).

**Table 17.** Risk factors for patients at or above the 90% vs. below 90% percentile in data set 3 (14 vs. 127 patients). *P*-values are based on two-sided Fisher's exact test without corrections for multiple testing. For the time to distant metastases, an additional *P*-value is reported based on Kaplan-Meier analysis (log-rank test). Median time to follow-up refers only to patients without metastases.

| Covariate | At or above 90% | Below 90% | *P*-value |
|---|---|---|---|
| Metastases | 4 mets vs 10 no mets (median time to follow-up of 9.5 years; range, 3.0-14.1) | 45 mets vs. 85 no mets (median time to follow-up of 8.4 years, range, 1.8-18.3) | 0.772 (Fisher's) |
| | | | 0.664 (log-rank) |
| Tumor size | 8 tumors > 2cm vs. 6 $\leq$ 2cm | 54 tumors > 2cm vs. 73 $\leq$ 2cm | 0.397 |
| Age | 6 $\leq$ 40 years vs. 8 > 40 years | 38 $\leq$ 40 vs. 89 > 40 years | 0.367 |
| Grade | 13 poorly diff. vs. 1 intermediate | 53 poorly diff. vs. 74 intermediate/well diff. | 0.0003 |

(continued)

| Covariate | At or above 90% | Below 90% | P-value |
|---|---|---|---|
| St. Gallen | 14 chemo vs. 0 no chemo | 106 chemo vs. 21 no chemo | 0.129 |
| NIH risk | 13 high vs. 1 intermediate | 79 high vs. 48 intermediate or low | 0.035 |
| ER | 11 ER-vs. 3 ER+ | 26 ER-vs. 101 ER+ | $2.3 \times 10^{-5}$ |
| ERBB2 | 4 ERBB2+ vs. 10 ERBB2- | 21 ERBB2+vs. 107 ERBB2- | 0.271 |
| Basal-like | 6 basal-like vs. 8 non-basal-like | 17 basal-like vs. 110 non-basal-like | 0.012 |
| Wound-response | 11 activated vs. 3 quiescent | 47 activated vs. 80 quiescent | 0.0037 |
| Hypoxia-response | 7 high vs. 7 low | 77 high vs. 50 low | 0.568 |
| 70-gene signature | 13 poor vs. 1 good | 71 poor vs. 56 good | 0.0082 |
| 48-gene signature | 6 LM vs. 8 no LM | 69 LM vs. 58 no LM | 0.574 |

Although basal-like tumors have been shown to be associated with a rather aggressive clinical behavior, five of these six patients did *not* develop metastases (median time to follow-up of 8.8 years, range 3.0-14.1), which supports the hypothesis that basal-like cancers are a molecularly heterogeneous group with different clinical outcomes. Further, for the tumors at or above the 90% percentile, we made again a surprising observation: 13 of 14 are poorly differentiated ($P = 0.0003$; Fisher's exact test), inviting pessimistic prognoses. However, 10 of 14 patients did not develop metastases, with a median time to follow-up of 9.5 years (range, 3.0-14.1). Note, that the standard risk factors for these 14 patients would also lead to pessimistic prognoses: based on the St. Gallen criteria, all 14 patients are recommended for chemotherapy ($P = 0.13$); the NIH risk is high for 13 of 14 patients ($P = 0.04$), 11 are ER- ($P = 2.3 \times 10$-5), 11 have an activated wound-response signature ($P = 0.004$), and 13 of 14 have a poor prognosis based on the 70-gene signature ($P = 0.008$).

**Table 19.** Correlation with clinical risk factors and genomic signatures in data set 4 (Schmidt *et al.,* 2008) ($n = 200$). The *P*-value for the comparison between the lower 25th and the upper 75th percentile (50 vs. 150 patients) is based on Fisher's exact test; the *P*-value for the overall distribution is based on Wilcoxon rank sum test for binary covariates and Kruskal-Wallis test for covariates with more than two values, and Welch's t-test for comparing the distributions of continuous values (age and tumor size) in the lower 25th percentile and upper 75th percentile. All tests are two-sided and without adjustments for multiple testing, $p < 0.05$ is considered statistically significant and shown in bold face. Median time to follow-up refers only to patients without metastases.

| Covariate | At or below 25% | Above 25% | P-value (lower 25% vs. upper 75%) | P-value (overall distribution) |
|---|---|---|---|---|
| Metastases | 21 mets vs. 29 no mets (median time to follow up of 8.6 years, range, 0.1-20.0) | 25 mets vs. 125 no mets (median time to follow up of 7.9 years, range, 0.1-16.9) | 0.0004 (P = 0.0002, log-rank) * | 0.037 |
| Grade (1, 2, or 3) | - | | - | 0.019 |
| Grade 3 vs. 1 or 2 | 7 grade 3 vs. 43 grade 1 or 2 | 28 grade 3 vs. 122 grade 1 or 2 | 0.525 | 0.009 |
| Tumor size ($\leq$ 2cm vs. > 2cm) | 30 tumors $\leq$ 2cm vs. 20 tumors > 2cm | 82 tumors $\leq$ 2cm vs. 68 tumors > 2cm | 0.622 | 0.028 |
| Tumor size (diameter in cm) | - | - | 0.553 | - |
| ER (positive vs. negative) | 41 ER+ vs. 9 ER- | 115 ER+vs. 35 ER- | 0.555 | 0.014 |

(continued)

| Covariate | | At or below 25% | Above 25% | *P*-value (lower 25% vs. upper 75%) | *P*-value (overall distribution) |
|---|---|---|---|---|---|
| Intrinsic subtypes (normal, ERBB2+, basal, luminal) | | - | | - | 0.462 |
| | ERBB2 (positive vs. others) | 6 ERBB2+ vs. 44 others | 10 ERBB2+ vs. 140 others | 0.370 | 0.273 |
| | Basal subtype (basal-like vs. others) | 16 basal-like vs. 34 others | 32 basal-like vs. 118 others | 0.131 | 0.350 |
| Wound-response (activated vs. quiescent) | | 49 activated vs 1 quiescent | 133 activated vs. 17 quiescent | 0.048 | 0.057 |
| Hypoxia-response (high vs. low) | | 50 high vs. 0 low | 150 high vs. 0 low | 1.0 | 1.0 |
| 70-gene signature (poor vs. good) | | 16 poor vs. 34 good | 42 poor vs. 108 good | 0.593 | 0.019 |
| 48-gene signature (lung mets. vs. no lung mets.) | | 0 LM vs. 50 no LM | 4 LM vs. 146 no LM | 0.574 | 0.149 |
| * Log-rank p-values from Kaplan-Meier analysis are also reported, cf. Fig. 8d, main manuscript. | | | | | |

For data set 4 (Table 19), we made similar observations. Patients whose tumor is of a higher grade or over 2 cm tend to be concentrated towards the right hand side (*P* = 0.009 and *P* = 0.028, respectively; Wilcoxon rank sum test). ER- tumors are also concentrated towards the right (*P* = 0.014; Wilcoxon rank sum test). Interestingly, we also observed that patients with a poor prognosis prediction based on the 70-gene signature tend to be concentrated towards the right. In fact, for patients at or above the 90% percentile (i.e., the 20 patients 5 at the far right of Fig. 6b), 13 are predicted as 'poor outcome' whereas for the remaining 180 patients below the 90% percentile, only 45 are predicted as 'poor outcome' (*P* = 0.0005; Fisher's exact test - see Table 20).

**Table 20.** Risk factors for patients at or above the 90% vs. below 90% percentile in data set 4 (20 vs. 180 patients). *P*-values are based on two-sided Fisher's exact test without corrections for multiple testing. For the time to distant metastases, an additional *P*-value is reported based on Kaplan-Meier analysis (log-rank test). Median time to follow-up refers only to patients without metastases.

| Covariate | At or above 90% | Below 90% | *P*-value |
|---|---|---|---|
| Metastases | 3 mets vs 17 no mets (median time to follow-up of 9.9 years; range, 0.3-16.8) | 43 mets vs 137 no mets (median time to follow-up of 7.4 years; range, 0.1-20.0) | 0.575 (Fisher's) |
| | | | 0.317 (log-rank) |
| Grade | 10 grade 3 vs. 10 grade 1 or 2 | 25 grade 3 vs. 155 grade 1 or 2 | 0.0004 |
| Tumor size | 13 tumors > 2 cm vs. 7 $\leq$ 2 cm | 75 tumors > 2 cm vs. 105 tumors $\leq$ 2 cm | 0.058 |
| ER | 9 ER-vs. 11 ER+ | 35 ER-vs. 145 ER+ | 0.019 |
| ERBB2 | 2 ERBB2+ vs. 18 ERBB2- | 14 ERBB2+ vs. 166 ERBB2- | 0.169 |
| Basal-like | 2 basal-like vs.18 non-basal-like | 46 basal-like vs. 134 non-basal-like | 0.665 |
| Wound-response | 17 activated vs. 3 quiescent | 165 activated vs. 15 quiescent | 0.399 |
| Hypoxia-response | 20 high vs. 0 low | 180 high vs. 0 low | 1.0 |
| 70-gene signature | 13 poor vs. 7 good | 45 poor vs. 135 good | 0.0005 |

(continued)

| Covariate | At or above 90% | Below 90% | *P*-value |
|---|---|---|---|
| 48-gene signature | 1 LM vs.19 no LM | 3 LM vs. 177 no LM | 0.346 |

Other risk factors would also lead to a pessimistic prognosis: 10 of 20 patients have a tumor of grade 3, compared to 25 of the remaining 180 patients ($P$ = 0.0004; Fisher's exact test). Nine of 20 tumors are ER-, compared to 35 of the remaining 180 tumors ($P$ = 0.02; Fisher's exact test). However, of the 20 patients at or above the 90% percentile, 17 did not develop any metastastes (median time to follow-up of 9.9 years; range, 0.3-16.8). Figure 17 shows heatmaps of tumor gene expression levels in the validation sets, (Figure 17a) data set 5 and (Figure 17b) data set 6. Expression values are shaded, with lighter shading indicating lower and darker shading indicating higher values (see inset shading key). Rows represent probe sets corresponding to down- or upregulated genes in MCF7-I6 vs. MCF7-I0 (probe sets were clustered based on complete hierarchical linkage). Columns represent tumors, ranked from left to right in increasing order based on the tandem score. The bar termed Mets/NoMets indicates the absence (light) or presence (dark) of distant metastases in the patients from which the tumors were obtained. Established prognostic factors are shown as bar plots. In data set 5, patients with tumors for which the tandem score is at or below the 30th percentile are one group (overhead, left hand bar), while patients above the 70th percentile are considered another group (overhead right hand). For data set 6, the 25th and 75th percentiles are considered. For Kaplan-Meier analysis, the time to event is distant metastases-free survival in data set 5 (see Figure 18e) and death from breast cancer in data set 6 (see Figure 18f). In data set 5 (Table 20), we made again surprising observations, although most results are not statistically significant given the small sample size of only 64 patients. Unexpectedly, patients with positive lymph node involvement tend to be concentrated towards the right ($P$ =0.03; Wilcoxon rank-sum test). For the patients at or above the 90% percentile (i.e., six patients at the far right of Figure 17a), five did not develop metastases (median time to follow-up of 7.2 years; range, 5.2-10.7 - see Table 21). All six patients have a tumor larger than 2 cm, and three tumors are ER-negative.

**Table 20.** Correlation with clinical risk factors and genomic signatures in data set 5 (Minn *et al.*, 2005) (*n* = 64). The *P*-value for the comparison between the lower 30th and the upper 70th percentile (19 vs. 45 patients) is based on Fisher's exact test; the *P*-value for the overall distribution is based on Wilcoxon rank sum test for binary covariates and Kruskal-Wallis test for covariates with more than two values, and Welch's *t*-test for comparing the distributions of continuous values (age in years and tumor size) in the lower 30th percentile and upper 70th percentile. All tests are two-sided and without adjustments for multiple testing, *p* < 0.05 is considered statistically significant and shown in bold face. Median time to follow-up refers only to patients without metastases.

| Covariate | At or below 30% | Above 30% | *P*-value (lower 30% vs. upper 70%) | P-value (overall distribution) |
|---|---|---|---|---|
| Metastases | 11 mets vs. 8 no mets (median time to fol low up of 6.6 years, range, 4.4-10.8) | 11 mets vs. 34 no mets {median time to follow up of 7.2 years, range, 3.8-10.7) | **0.020 (Fisher's) 0.008 (log-rank)\*** | 0.076 |
| Tumor size ($\leq$ 2cm vs. > 2cm) | 4 tumors $\leq$ 2cm vs. 15 tumors > 2cm | 7 tumors $\leq$ 2cm vs. 38 tumors > 2cm | 0.719 | 0.533 |
| Tumor size (diameter in cm) | - | - | 0.895 | - |
| Positive lymph nodes {0 or 1 or 2 or 3) | - | - | - | 0.171 |
| Positive lymph nodes (0 vs. 1 or 2 or 3) | 10 LNN-0 vs. 9 LNN-1/2/3 | 18 LNN-0 vs. 27 LNN-1/2/3 | 0.415 | 0.031 |
| Age ($\leq$ 40 years vs. > 40 years) | 4 patients $\leq$ 40 years vs. 15 > 40 years | 5 patients $\leq$ 40 years vs. 40 > 40 years | 0.432 | 0.524 |
| Age (in years) | - | - | 0.231 | - |
| ER (positive vs. negative) | 8 ER+ vs. 11 ER- | 26 ER+ vs. 19 ER- | 0.284 | 0.783 |

(continued)

| Covariate | At or below 30% | Above 30% | P-value (lower 30% vs. upper 70%) | P-value (overall distribution) |
|---|---|---|---|---|
| PR (positive vs. negative) | 7 PR+vs. 12 PR- | 17 PR+ 28 PR- | 1.0 | 0.819 |
| Intrinsic subtypes (normal, ERBB2+, basal, luminal) | - | - | - | 0.219 |
| ERBB2 (positive vs. others) | 2 ERBB2+ vs. 17 others | 6 ERBB2+ vs. 39 others | 1.0 | 0.296 |
| Basal subtype (basal-like vs. others) | 6 basal-like vs. 13 others | 17 basal-like vs. 28 others | 0.778 | 0.615 |
| Wound-response (activated vs. quiescent) | 13 activated vs. 6 quiescent | 19 activated vs. 26 quiescent | 0.099 | 0.230 |
| Hypoxia-response (high vs. low) | 19 high vs. 0 low | 45 high vs. 0 low | 1.0 | 1.0 |
| 70-gene signature (poor vs. good) | 9 poor vs. 10 good | 13 poor vs. 32 good | 0.249 | 0.475 |
| 48-gene signature (lung mets. vs. no lung mets.) | 6 LM vs. 13 no LM | 3 LM vs. 42 no LM | 0.016 | 0.076 |

*Log-rank $p$-values from Kaplan-Meier analysis are also reported, cf. Fig. 8e, main manuscript. For the comparison of the lower $25^{th}$ (i.e., 16 patients) and the upper $75^{th}$ percentiles (i.e., 48 patients), we obtain $P = 0.07$ (Fisher's exact test) and $P = 0.03$ (log-rank test).

**Table 21.** Risk factors for patients at or above the 90% vs. below 90% percentile in data set 5 (6 vs. 58 patients). $P$-values are based on two-sided Fisher's exact test without corrections for multiple testing. For the time to distant metastases, an additional $P$-value is reported based on Kaplan-Meier analysis (log-rank test). Median time to follow-up refers only to patients without metastases.

| Covariate | At or above 90% | Below 90% | P-value |
|---|---|---|---|
| Metastases | 1 mets vs. 5 no mets (median time to follow-up of 7.2 years; range, 5.2-10.7) | 21 mets vs. 37 no mets. (median time to follow-up of 5.8 years; range, 0.7-10.8) | 0.655 (Fisher's) |
| | | | 0.368 (log-rank) |
| Tumor size | 6 tumors > 2cm vs. 0 tumors ≤ 2 cm | 48 tumors > 2 cm vs. 10 tumors ≤ 2 cm | 0.578 |
| Positive lymph nodes | 4 LNN+ vs. 2 LNN- | 32 LNN + vs. 26 LNN- | 0.688 |
| Age | 0 patients ≤ 40 years vs. 6 patients > 40 years | 9 patients ≤ 40 years vs. 49 patients > 40 years | 0.582 |
| ER | 3 ER- vs. 3 ER+ | 31 ER- vs. 27 ER+ | 1.0 |
| PR | 3 PR- vs. 3 PR+ | 37 PR- vs. 21 P R+ | 0.664 |
| ERBB2 | 1 ERBB2+ vs. 5 ERBB2- | 7 ERBB2+ vs. 51 ERBB2- | 0.567 |
| Basal-like | 0 basal-like vs. 6 non-basal-like | 23 basal-like vs. 35 non-basal-like | 0.080 |
| Wound-response | 3 activated vs. 3 quiescent | 29 activated vs. 29 quiescent | 1.0 |
| Hypoxia-response | 6 high vs. 0 low | 58 high vs. 0 low | 1.0 |
| 70-gene signature | 2 poor vs. 4 good | 20 poor vs. 38 good | 1.0 |
| 48-gene signature | 1 LM vs. 5 no LM | 8 LM. vs. 50 no LM | 1.0 |

In data set 6 (Figure 17b; endpoint: time to death from breast cancer), we observed that the tandem score correlates with the wound-response signature (see Table 22), as patients with an activated wound-response are concentrated in the lower 25% percentile ($P = 0.03$; Fisher's exact test). However, we failed to observe any remarkable association between the other risk factors and the tandem score. Patients at or below the lower 25th percentile have a 2.7-fold increased risk of dying from breast cancer, compared to the remaining patients, but this difference is only marginally significant ($P = 0.049$, log-rank test; cf. Fig. 8f). When we considered death from breast cancer as primary endpoint in data set 5 3 (cf. Fig. 6a), we made a similar observation. Here, patients in the high-risk group have a 1.8-fold increased risk (95%-Cl, 0.86-3.84), but the difference is not significant with $P = 0.12$ (log-rank test). Clearly, the endpoints 'metastases' and 'death' are positively correlated, but not equivalent, which could explain why we observed only a weak association between the tandem signature and time to death from breast cancer.

**Table 22.** Correlation with clinical risk factors and genomic signatures in data set 6 (Miller *et al.,* 2005) ($n = 149$). The P-value for the comparison between the lower 25th and the upper 75th percentile (37 vs. 112) is based on Fisher's exact test; the P-value for the overall distribution is based on Wilcoxon rank sum test for binary covariates and Kruskal-Wallis test for covariates with more than two values, and Welch's t-test for comparing the distributions of continuous values (age and tumor size) in the lower 25th percentile and upper 75th percentile. All tests are two-sided and without adjustments for multiple testing, p < 0.05 is considered statistically significant and shown in bold face. Median time to follow-up refers only to patients without event.

| Covariate | | At or below 25% | Above 25% | *P*-value (lower 25% vs. upper 75%) | *P*-value (overall distribution) |
|---|---|---|---|---|---|
| Event (death from breast cancer) | | 9 events vs. 18 no event (median time to follow up of 10.6 years, range, 3.0-12.4) | 13 events vs. 99 no event (median time to follow up of 10.7 years, range, 0.9-12.8) | 0.067 ($P = 0.049$, log-rank test)* | 0.200 |
| Grade (1, 2, or 3) | | - | - | - | 0.716 |
| | Grade 3 vs. grade 1 or 2 | 8 tumors grade 3 vs. 29 tumors grade 1 or 2 | 14 tumors grade 3 vs. 97 tumors grade 1 or 2 (1 tumor grade unknown) | 0.191 | 0.99 |
| Tumor size ($\leq$ 2cm vs. > 2cm) | | 20 tumors $\leq$ 2cm vs. 17 > 2cm | 65 tumors $\leq$ 2cm vs. 47 > 2cm | 0.705 | 0.508 |
| | Tumor size (diameter in cm) | - | - | 0.190 | - |
| Age ($\leq$ 40 years vs. > 40 years) | | 5 patients $\leq$ 40 years vs. 32 patients > 40 years | 6 patients $\leq$ 40 years vs. 106 patients > 40 years | 0.142 | 0.291 |
| | Age (in years) | | | 0.193 | - |
| ER (positive vs. negative) | | 31 ER+ vs. 5 ER- (1 unknown) | 96 ER+ vs. 14 ER- {2 unknown) | 1.0 | 0.732 |
| PR (positive vs. negative) | | 28 PR+ vs. 9 PR- | 90 PR+ vs. 22 PR- | 0.641 | 0.592 |
| Intrinsic subtypes {normal, ERBB2+, basal, luminal) | | - | - | - | 0.451 |
| | ERBB2 (positive vs. others) | 4 ERBB2+ vs. 33 others | 11 ERBB2+ vs. 101 others | 1.0 | 0.764 |
| | Basal subtype (basal-like vs. others) | 8 basal-like vs. 29 others | 27 basal-like vs. 85 others | 1.0 | 0.827 |

(continued)

| Covariate | At or below 25% | Above 25% | P-value (lower 25% vs. upper 75%) | P-value (overall distribution) |
|---|---|---|---|---|
| Wound-response (activated vs. quiescent) | 20 activated vs. 17 quiescent | 37 activated vs. 75 quiescent | 0.031 | 0.267 |
| Hypoxia-response (high vs. low) | 37 high vs. 0 low | 112 high vs. 0 low | 1.0 | 1.0 |
| 70-gene signature (poor vs. good) | 10 poor vs. 27 good | 27 poor vs. 85 good | 0.827 | 0.360 |
| 48-gene signature (lung mets. vs. no lung mets.) | 2 LM vs. 35 no LM | 2 LM vs. 110 no LM | 0.257 | 0.783 |
| * Log-rank p-values from Kaplan-Meier analysis are also reported, cf. Fig.8f, main manuscript. | | | | |

**Example 13. Multivariate Cox models for time to event**

The previous Examples revealed that (*i*) the tandem score is largely independent of established risk factors and (*ii*) the predictions based on this score frequently contradict the prognoses based on these factors, and often correctly so. Therefore, we performed a multivariate analysis using Cox proportional hazards regression models. In short, a multivariate Cox model combines multiple risk factors into one prediction model.

In data set 1 (Table 23), the tandem score is associated with the smallest multivariate Cox *p*-value of $1.5 \times 10^{-8}$ (hazard of 3.13; 95%-CI, 2.11-4.65). The partial effect of the tandem score is 44.33%, and greater than the effect of all other factors combined.

**Table 23.** Multivariate Cox model for data set 1 (*n* = 286). HR: hazard ratio for time to event (distant metastases-free survival); partial effect: gain (loss) in prognostic power (in percent of the explained deviance) when the covariate is included (omitted) into (from) a model containing all remaining covariates. *P*-values < 0.05 are considered statistically significant and shown in bold face.

| Covariate | P-value | HR (95%-CI) | Partial effect [%] |
|---|---|---|---|
| ER (positive vs. negative) | 0.23 | 1.34 (0.83-2.16) | 2.20 |
| ERBB2 (positive vs. negative) | 0.50 | 0.84 (0.50-1.40) | 0.71 |
| Wound-response (activated vs. quiescent) | 0.46 | 1.23 (0.71-2.13) | 0.83 |
| Hypoxia-response (high vs. low) | 0.11 | 1.38 (0.93-2.05) | 3.87 |
| 70-gene signature (poor vs. good) | $7.0 \times 10^{-5}$ | 2.51 (1.60-3.96) | 24.50 |
| 48-gene signature (lung mets. vs. no lung mets.) | 0.012 | 1.64 (1.12-2.42) | 9.50 |
| Tandem-signature (poor vs. good) | $1.5 \times 10^{-8}$ | 3.13 (2.11-4.65) | 44.33 |

In data set 2 (Table 24), the tandem score is also the most significant factor with *P* = 0.01 (hazard of 4.20; 95%-CI, 1.54-11.43; partial effect of 29.96%). Again, the partial effect of the tandem score is the highest. However, we note that the tandem score was derived using data sets 1 and 2; therefore, these results overestimate the true prognostic power, and the effects observed in the independent validation sets are more relevant.

**Table 24.** Multivariate Cox model for data set 2 (*n* = 104; 21 cases discarded due to missing values). HR: hazard ratio for time to event (distant metastases-free survival); partial effect: gain (loss) in prognostic power (in percent of the explained deviance) when the covariate is included (omitted) into (from) a model containing all remaining covariates. *P*-values < 0.05 are considered statistically significant and shown in bold face.

| Covariate | *P*-value | HR (95%-CI) | Partial effect [%] |
|---|---|---|---|
| Tumor size (≤ 2cm vs. > 2cm) | 0.02 | 3.15 (1.20-8.28) | 22.01 |
| Age (≤ 40 years vs. > 40 years) | 0.35 | 0.51 (0.12-2.08) | 3.95 |
| ER (positive vs. negative) | 0.36 | 1.61 (0.59-4.42) | 3.49 |
| Grade (poorly diff. vs. intermediate or well diff.) | 0.15 | 0.43 (0.14-1.37) | 8.87 |
| ERBB2 (positive vs. negative) | 0.58 | 1.46 (0.39-5.44) | 1.22 |
| Wound-response (activated vs. quiescent) | 0.31 | 0.50 (0.13-1.89) | 4.16 |
| Hypoxia-response (high vs. low) | 0.72 | 0.84 (0.32-2.19) | 0.52 |
| 70-gene signature (poor vs. good) | 0.28 | 2.01 (0.57-7.10) | 4.88 |
| 48-gene signature (lung mets. vs. no lung mets.) | 0.94 | 1.03 (0.41-2.59) | 0.02 |
| Tandem-signature (poor vs. good) | 0.01 | 4.20 (1.54-11.43) | 29.96 |

In data set 3 (Table 25), the predictions based on the 70-gene signature are the most important factor (*P* = 0.004; hazard of 3.89; 95%-CI, 1.56-9.74; partial effect of 25.09%). Here, the tandem score is not significant with *P* = 0.15, hazard of 1.58 (95%-CI, 0.85-2.95; partial effect of 5.26%). This can be explained by the fact that data set 3 contains a subset of samples from which the 70-gene signature was originally derived; hence, the results were expected to be biased towards the 70-gene signature.

**Table 25.** Multivariate Cox model for data set 3 (*n* = 141). HR: hazard ratio for time to event (distant metastases-free survival); partial effect: gain (loss) in prognostic power (in percent of the explained deviance) when the covariate is included (omitted) into (from) a model containing all remaining covariates. *P*-values < 0.05 are considered statistically significant and shown in bold face.

| Covariate | *P*-value | HR (95%-CI) | Partial effect [%] |
|---|---|---|---|
| Tumor size (≤ 2cm vs. > 2cm) | 0.09 | 1.90 (0.90-4.01) | 7.98 |
| Age (≤ 40 years vs. > 40 years) | 0.24 | 1.44 (0.78-2.63) | 3.52 |
| ER (positive vs. negative) | 0.18 | 1.64 (0.79-3.42) | 4.69 |
| Grade (poorly diff. vs. intermediate or well diff.) | 0.94 | 1.04 (0.44-2.46) | 0.02 |
| ERBB2 (positive vs. negative) | 0.12 | 1.80 (0.85-3.80) | 5.92 |
| St. Gallen (chemotherapy vs. no chemotherapy) | 0.96 | 0.97 (0.28-3.37) | 0.01 |
| NIH risk (high vs. intermediate or low) | 0.59 | 0.73 (0.23-2.34) | 0.75 |
| Wound-response (activated vs. quiescent) | 0.10 | 1.91 (0.89-4.10) | 7.19 |
| Hypoxia-response (high vs. low) | 0.27 | 1.45 (0.75-2.81) | 3.33 |

(continued)

| Covariate | P-value | HR (95%-CI) | Partial effect [%] |
|---|---|---|---|
| 70-gene signature (poor vs. good) | 0.004 | 3.89 (1.56-9.74) | 25.09 |
| 48-gene signature (lung mets. vs. no lung mets.) | 0.15 | 0.63 (0.33-1.19) | 5.36 |
| Tandem-signature (poor vs. good) | 0.15 | 1.58 (0.85-2.95) | 5.26 |

In data set 4 (Table 26), the tandem score is by far the most relevant factor with $P = 6.1 \times 10^{-4}$ (hazard of 3.10; 95%-CI, 1.62-5.92; partial effect of 48.74%). Here, the tandem score provided more information than all other risk factors combined.

Table 26. Multivariate Cox model for data set 4 ($n = 200$). HR: hazard ratio for time to event (distant metastases-free survival); partial effect: gain (loss) in prognostic power (in percent of the explained deviance) when the covariate is included (omitted) into (from) a model containing all remaining covariates. P-values < 0.05 are considered statistically significant and shown in bold face.

| Covariate | P-value | HR (95%-CI) | Partial effect [%] |
|---|---|---|---|
| Grade 3 vs. not grade 3 | 0.07 | 2.07 (0.94-4.55) | 13.24 |
| Tumor size ($\leq$ 2cm vs. > 2cm) | 0.21 | 1.49 (0.80-2.79) | 6.75 |
| ER (positive vs. negative) | 0.56 | 1.28 (0.56-2.93) | 1.50 |
| ERBB2 (positive vs. negative) | 0.10 | 2.19 (0.85-5.63) | 10.09 |
| Wound-response (activated vs. quiescent) | 0.47 | 1.72 (0.40-7.46) | 2.58 |
| Hypoxia-response (high vs. low) * | 0.76 | 0.90 (0.43-1.84) | 0.42 |
| 70-gene signature (poor vs. good) | 0.85 | 1.07 (0.52-2.24) | 0.16 |
| 48-gene signature (lung mets. vs. no lung mets.) | 0.34 | 2.08 (0.47-9.32) | 3.34 |
| Tandem-signature {poor vs. good) | $6.1 \times 10^{-4}$ | 3.10 (1.62-5.92) | 48.74 |

In data set 5 (Table 27), the tandem score is again the most informative factor with $P = 0.003$ (hazard of 4.94; 95%-CI, 1.70-14.35; partial effect of 38.18%).

Table 27. Multivariate Cox model for data set 5 ($n = 64$). HR: hazard ratio for time to event (distant metastases-free survival); partial effect: gain (loss) in prognostic power (in percent) when the covariate is included (omitted) into (from) a model containing all remaining covariates. P-values < 0.05 are considered statistically significant and shown in bold face.

| Covariate | P-value | HR (95%-CI) | Partial effect [%] |
|---|---|---|---|
| Tumor size ($\leq$ 2cm vs. > 2cm) | 0.90 | 1.07 (0.36-321) | 0.07 |
| Positive lymph nodes (0 vs. 1 or 2 or 3) | 0.41 | 1.56 (0.54-4.49) | 3.04 |
| Age ($\leq$ 40 years vs. > 40 years) | 0.048 | 0.11 (0.01-099) | 29.42 |
| ER (positive vs. negative) | 0.24 | 0.38 (0.08-191) | 6.51 |
| PR (positive vs. negative) | 0.79 | 0.82 (0.19-3.48) | 0.33 |
| ERBB2 (positive vs. negative) | 0.43 | 0.40 (0.04-3.85) | 3.39 |

(continued)

| Covariate | *P*-value | HR (95%-CI) | Partial effect [%] |
|---|---|---|---|
| Wound-response (activated vs. quiescent) | 0.048 | 0.13 (0.02-099) | 21.19 |
| 70-gene signature (poor vs. good) | 0.21 | 3.38 (0.50-22.76) | 8.22 |
| 48-gene signature (lung mets. vs. no lung mets.) | 0.62 | 1.38 (0.38-5.03) | 1.07 |
| Tandem-signature (poor vs. good) | 0.003 | 4.94 (1.70-14.35) | 38.18 |

In data set 6 (Table 28; endpoint: time to death from breast cancer), the tandem score is not a significant factor ($P$ = 0.14); here, tumor size and ER status provide the most information. This confirms our observation that the tandem signature is a predictor for development of metastases.

**Table 28.** Multivariate Cox model for data set 6 ($n$ = 145, four cases omitted due to missing values). HR: hazard ratio for time to event (death of breast cancer); partial effect: gain (loss) in prognostic power (in percent of the explained deviance) when the covariate is included (omitted) into (from) a model containing all remaining covariates. *P*-values < 0.05 are considered statistically significant and shown in bold face.

| Covariate | *P*-value | HR (95%-CI) | Partial effect [%] |
|---|---|---|---|
| Grade 3 vs. not grade 3 | 0.096 | 2.94 (0.83-10.45) | 10.38 |
| Tumor size ($\leq$ 2cm vs. > 2cm) | 0.018 | 3.51 (1.25-9.87) | 23.11 |
| Age ($\leq$ 40 years vs. > 40 years) | 0.180 | 2.41 (0.67-8.64) | 6.33 |
| ER (positive vs. negative) | 0.014 | 8.96 (1.56-51.48) | 25.27 |
| PR (positive vs. negative) | 0.063 | 0.26 (0.06-1.08) | 12.11 |
| ERBB2 (positive vs. negative) | 0.870 | 1.12 (0.18-453) | 0.10 |
| Wound-response (activated vs. quiescent) | 0.710 | 0.77 (0.19-3.14) | 0.53 |
| Hypoxia-response (high vs. low) | 0.170 | 1.94 (0.75-5.01) | 6.63 |
| 70-gene signature (poor vs. good) | 0.900 | 1.10 (0.28-4.28) | 0.07 |
| Tandem-signature (poor vs. good) | 0.140 | 2.02 (0.80-5.08) | 8.13 |

The isolation of a hyperinvasive population of cells from the characteristically weakly invasive MCF7 breast epithelial cancer cells strongly supports the hypothesis that the proclivity for metastases originates in the primary lesion. The hyperinvasive cells were clonally selected and expanded *in vitro* solely based on their propensity to invade, and they concomitantly showed characteristics of an epithelial to mesenchymal transition and a decreased adhesion to extracellular matrix components.

The wound scrape assays demonstrated the increased motility of the hyperinvasive cells. The mesenchymal appearance of the MCF7-I6 cells suggests a more motile phenotype with filapodia-like structures. Vimentin is one of the key genes involved in cell shape maintenance and is highly expressed in mesenchymal cells. Motility is dependent on the regulated formation and dissolution of focal adhesions of which paxillin (2.0-fold overexpressed in MCF7-6; $P$ = 0.001) is heavily involved and therefore its up-regulation is likely to contribute to increased turnover of these complexes, thereby stimulating migration. Therefore, the increased expression of vimentin and paxillin, coupled with the partial rearrangement of the cytoskeleton, offer an explanation on the increased motility of the MCF7-I6 cells.

We observed a significant down-regulation of interferon-induced genes in the aggressive MCF7-I6 cells. The down-regulation of interferon- and immune-responsive genes results in down-regulation of antigen processing and presentation, leading to reduced immunogenicity and camouflage of the tumor cell. Several members of the major histocompatibility complex are down regulated in the hyperinvasive cells, suggesting a means by which these cells could evade an immune response. Further down-regulation of pro-apoptotic genes such as FAS (TNF receptor superfamily, member 6) and the OAS (oligoadenylate synthetase) family encourage tumor formation. The anti-tumorigenic activities of the interferons mainly act through the JAK/STAT pathway. Since the expression of the JAK family members were largely unaltered between the two cell populations, STAT1 is likely a key player in this process. STAT1 is a transcriptional activator known to regulate the immune response and have anti-proliferative, pro-apoptotic and cell viability functions. The concurrent down-regulation of interferon-responsive genes on isolation of invasive cells suggests that the process of invasion requires a diminished interferon response.

Interestingly, the down-cassette (SET C) of the 63-gene set of the present invention ("tandem signature", SET A) contains a significant ($P$ = 1.74 x $10^{-15}$, hypergeometric test with Benjamini and Hochberg's adjustment for multiple testing, FDR < 0.05) number of immune-response related genes (20 of 36; 56%), and genes (11 of 36; 31 %) involved in antigen processing and presentation 15 ($P$ = 1.12 x $10^{-15}$). Taken together, these results are consistent with an immune selection and might represent further evidence that immunoediting is the seventh hallmark of cancer. By matching the differentially expressed genes from the *in vitro* analysis with genes that are prognostic for the development of metastases *in vivo,* we selected a novel and unique panel of invasion-mediating genes, consisting of a down- and an up-cassette. Tumors that show a low expression of the genes in the downcassette and a concomitant high expression of the genes in the up-cassette tend to metastasize significantly earlier than tumors that do not.

In our analysis, we observed a substantial number of patients across four multi-center studies who had a relatively good clinical outcome - despite poor prognoses based on established clinical risk factors or other prognostic signatures. In contrast, some of these patients would obtain a good prognosis based on the expression of the tandem signature. Therefore, the tandem signature may represent a useful complement to conventional risk factors and previously reported gene signatures, and perhaps with the potential to spare toxic adjuvant systemic therapy.

**Correlation Table**

Table 10. Correlating the unique probe set identifier, the gene to which the probe set is capable of hybridising, the GenBank accession number, the Genbank version number, and a reference made thereto, each of which is incorporated herein by reference.

| Probe ID | Gene Symbol | GenBank Accession Number | Version Number | Reference |
|---|---|---|---|---|
| 217478_s_at | HLA-DMA | X76775 | X76775.1 GI:512468 | Radley, E. et al., J. Biol. Chem. 269 (29), 18834-18838 (1994) |
| 208306_x_at | HLA-DRB4 | NM_021983 XM_940103 | NM_021983.4 GI:52630343 | Lacap, P.A. et al., AIDS 22 (9), 1029-1038 (2008) |
| 215193_x_at | HLA-DRB1 | AJ297586 | AJ297586.2 GI:15387628 | |
| 204670_x_at | HLA-DRB5 | NM_002125 | NM_002125.3 GI:26665892 | Lacap, P.A. et al., AIDS 22 (9), 1029-1038 (2008) |
| 209312_x_at | HLA-DRB1 | U65585 | U65585.1 GI:5478215 | Martinez-Quiles, N. et al., Tissue Antigens 49 (6), 658-661 (1997) |
| 209687_at | CXCL12 | U19495 | U19495.1 GI:1754834 | |
| 218999_at | FLJ11000 | NM_018295 | NM_018295.2 GI:111607481 | Scherer, S.W. et al., Science 300 (5620), 767-772 (2003) |

(continued)

| Probe ID | Gene Symbol | GenBank Accession Number | Version Number | Reference |
|---|---|---|---|---|
| 204490_s_at | CD44 | M24915 | M24915.1 GI:180196 | Stamenkovic, I.. et al., Cell 56 (6), 1057-1062 (1989) |
| 209835_x_at | CD44 | BC004372 | BC004372.1 GI:13325117 | Strausberg, R.L et al., Proc. Natl. Acad. Sci. U.S.A. 99 (26), 16899-16903 (2002) |
| 212014_x_at | CD44 | AI493245 | gi: 4394248 | |
| 212063_at | CD44 | BE903880 | Gi: 10395551 | |
| 203666_at | CXCL12 | NM_000609 | NM_000609.4 GI:76563934 | Yoshitake, N. et al., Br. J. Cancer 98 (10), 1682-1689 (2008) |
| 204780_s_at | FAS | AA164751 | gi: 1740929 | Hillier, L et al., Genome Res. 6 (9): 807-828 1996 |
| 216231_s_at | B2M | AW188940 | gi: 6463376 | |
| 214459_x_at | HLA-C | M12679 | M12679.1 GI:187911 | Szots, H. et al., Proc. Natl. Acad. Sci. U.S.A. 83 (5), 1428-1432 (1986) |
| 203768_s_at | STS | AU138166 | gi: 10999687 | Kimura, K. et al., Genome Res. 16 (1): 55-65 2006 |
| 221491_x_at | HLA-DRB1 | AA807056 | gi: 2876632 | |
| 202687_s_at | TNFSF10 | U57059 | U57059.1 GI:1336207 | |
| 202688_at | TNFSF10 | NM_003810 | NM_003810.2 GI:23510439 | Kim, M et al., Cancer Res. 68 (9), 3440-3449 (2008) |
| 204781_s_at | FAS | NM_000043 | NM_000043.3 GI:23510419 | Fountoulakis, S. et al., Eur. J. Endocrinol. 158 (6), 853-859 (2008) |
| 216252_x_at | FAS | Z70519 | Z70519.1 GI:1418817 | Papoff, G. et al., J. Immunol. 156 (12), 4622-4630 (1996) |
| 211799_x_at | HLA-C | U62824 | U62824.1 GI:1575443 | Wells, R.S. et al., Immunogenetics 46 (3), 173-180 (1997) |
| 221675_s_at | CHPT1 | AF195624 | AF195624.1 GI:9502012 | Henneberry, A.L. et al., J. Biol. Chem. 275 (38), 29808-29815 (2000) |
| 211911_x_at | HLA-B | L07950 | L07950.1 GI:307236 | Rodriguez, S.G. et al., Hum. Immunol. 37 (3), 192-194 (1993) |

(continued)

| Probe ID | Gene Symbol | GenBank Accession Number | Version Number | Reference |
|----------|-------------|--------------------------|----------------|-----------|
| 208812_x_at | HLA-C | BC004489 | BC004489.2 GI:39644689 | Strausberg, R.L. et al., Proc. Natl. Acad. Sci. U.S.A. 99 (26), 16899-16903 (2002) |
| 211528_x_at | HLA-G | M90685 | M90685.1 GI:184211 | Ishitani, A. et al., Proc. Natl. Acad. Sci. U.S.A. (1992) |
| 211529_x_at | HLA-G | M90684 | M90684.1 GI:188467 | Ishitani, A. et al., Proc. Natl. Acad. Sci. U.S.A. (1992) |
| 214022_s_at | IFITM1 | AA749101 | gi: 2789059 | Goto, Y. et al., FEBS Lett. 580 (7), 1833-1838 (2006) |
| 217933_s_at | LAP3 | NM_015907 | NM_015907.2 GI:41393560 | |
| 206346_at | PRLR | NM_000949 | NM_000949.2 GI:40254435 | Plotnikov, A. et al., Cancer Res. 68 (5), 1354-1361 (2008) |
| 209761_s_at | SP110 | AA969194 | gi: 3144374 | |
| 210070_s_at | CPT1B | U62733 | U62733.1 GI:1762532 | Britton, C.H. et al., Genomics 40 (1), 209-211 (1997) |
| 218429_s_at | FLJ11286 | NM_018381 | NM_018381.2 GI:154350197 | Ota, T. et al., Nat. Genet. 36 (1), 40-45 (2004) |
| 215313_x_at | HLA-A | AA573862 | gi: 2348377 | |
| 204806_x_at | HLA-F | NM_018950 | NM_018950.2 GI:149158697 | Burfoot, R.K. et al., Tissue Antigens 71 (1), 42-50 (2008) |
| 212203_x_at | IFITM3 | BF338947 | gi: 11285367 | |
| 201752_s_at | ADD3 | AI763123 | gi: 5178790 | |
| 210538_s_at | BIRC3 | U37546 | U37546.1 GI:1145290 | Uren, A.G. et al., Proc. Natl. Acad. Sci. U.S.A. 93 (10), 4974-4978 (1996) |
| 53720_at | FLJ11286 | AI862559 | gi: 5526666 | |
| 216526_x_at | HLA-C | AK024836 | AK024836.1 GI:10437242 | |
| 221875_x_at | HLA-F | AW514210 | gi: 7152378 | |
| 33304_at | ISG20 | U88964 | U88964.1 GI:2062679 | |
| 204279_at | PSMB9 | NM_002800 | NM_002800.4 GI:73747923 | Deshpande, A. et al., J. Infect. Dis. 197 (3), 371-381 (2008) |

(continued)

| Probe ID | Gene Symbol | GenBank Accession Number | Version Number | Reference |
|---|---|---|---|---|
| 201427_s_at | SEPP1 | NM_005410 | NM_005410.2 GI:62530390 | Peters, U. et al., Cancer Epidemiol. Biomarkers Prev. 17 (5), 1144-1154 (2008) |
| 208392_x_at | SP110 | NM_004510 | NM_004510.3 GI:190343007 | Cliffe, S.T. et al., Prenat. Diagn. 27 (7), 674-676 (2007) |
| 203147_s_at | TRIM14 | BE962483 | gi: 11765431 | |
| 205068_s_at | ARHGAP26 | BE671084 | gi: 10031625 | |
| 217523_at | CD44 | AV700298 | gi: 10302269 | Xu, X. et al., Proc. Natl. Acad. Sci. U.S.A. 98 (26): 15089-15094 2001 |
| 213932_x_at | HLA-A | AI923492 | Gi: 5659456 | |
| 221978_at | HLA-F | BE138825 | gi: 8601325 | |
| 200923_at | LGALS3BP | NM_005567 | NM_005567.2 GI:6006016 | Lee, Y.J. et al., Clin. Exp. Rheumatol. 25 (4 SUPPL 45), S41-S45 (2007) |
| 203788_s_at | SEMA3C | AI962897 | gi: 5755610 | |
| 202863_at | SP100 | NM_003113 | NM_003113.3 GI:122939209 | Takahashi, K. et al., Mol. Biol. Cell 18 (5), 1701-1709 (2007) |
| 202307_s_at | TAP1 | NM_000593 | NM_000593.5 GI:53759115 | Soundravally, R. et al., Scand. J. Immunol. 67 (6), 618-625 (2008) |
| 200927_s_at | RAB14 | AA919115 | gi: 3059005 | |

Table 11. Correlating the unique probe set identifier, the gene to which the probe set is capable of hybridising, the GenBank accession number, the Genbank version number, and a reference made thereto, each of which is incorporated herein by reference.

| Probe ID | Gene Symbol | GenBank Accession Number | Version Number | Reference |
|---|---|---|---|---|
| 204540_at | EEF1A2 | NM_001958 XR_017886 | NM_001958.2 GI:25453470 | Grassi,G. et al., Biochimie 89 (12), 1544-1552 (2007) |
| 207996_s_at | C18ORF1 | NM_004338 | NM_004338.2 GI:51093712 | Yoshikawa,T. et al., Genomics 47 (2), 246-257 (1998) |
| 202806_at | DBN1 | NM_004395 | NM_004395.3 GI:166362725 | Olsen,J.V. et al., Cell 127 (3), 635-648 (2006) |

(continued)

| Probe ID | Gene Symbol | GenBank Accession Number | Version Number | Reference |
|---|---|---|---|---|
| 202912_at | ADM | NM_001124 | NM_001124.1 GI:4501944 | Uzan,B. et al., J. Cell. Physiol. 215(1), 122-128 (2008) |
| 211823_s_at | PXN | D86862 | D86862.1 GI:1912054 | Mazaki,Y. et al., J. Biol. Chem. 272 (11), 7437-7444 (1997) |
| 219250_s_at | FLRT3 | NM_013281 | NM_013281.2 GI:38202220 | Deloukas,P. et al., Nature 414 (6866), 865-871 (2001) |
| 202219_at | SLC6A8 | NM_005629 | NM_005629.2 GI:183979976 | Anselm,I.A. et al., Neurology 70 (18), 1642-1644 (2008) |
| 203180_at | ALDH1A3 | NM_000693 | NM_000693.2 GI:153266821 | Rexer,B.N. et al., Cancer Res. 61 (19), 7065-7070 (2001) |
| 209682_at | CBLB | U26710 | U26710.1 GI:862406 | Keane,M.M. et al., Oncogene 10 (12), 2367-2377 (1995) |
| 212977_at | CMKOR1 | AI817041 | gi: 5436120 | |
| 205258_at | INHBB | NM_002193 | NM_002193.2 GI:154813203 | Purdue,M.P. et al., Cancer Res. 68 (8), 3043-3048 (2008) |
| 209099_x_at | JAG1 | U73936 | U73936.1 GI:1695273 | Lindsell,C.E. et al., Cell 80 (6), 909-917 (1995) |
| 216268_s_at | JAG1 | U77914 | U77914.1 GI:1684889 | Lindsell,C.E. et al., Cell 80 (6), 909-917 (1995) |
| 200771_at | LAMC1 | NM_002293 | NM_002293.3 GI:145309325 | Jakobsson,L. et al., FASEB J. 22 (5), 1530-1539 (2008) |
| 201398_s_at | TRAM1 | BC000687 | BC000687.2 GI:33990663 | Strausberg,R.L., Proc. Natl. Acad. Sci. U.S.A. 99 (26), 16899-16903 (2002) |
| 201294_s_at | WSB1 | N24643 | gi: 1138793 | |
| 209122_at | ADFP | BC005127 | BC005127.2 GI:33873146 | Strausberg,R.L. et al., Proc. Natl. Acad. Sci. U.S.A. 99 (26), 16899-16903 (2002) |
| 211946_s_at | BAT2D1 | AL096857 | AL096857.1 GI:5541862 | |
| 214820_at | BRWD1 | AJ002572 | AJ002572.1 GI:2959924 | Vidal-Taboada,J.M. et al., Biochem. Biophys. Res. Commun. 243 (2), 572-578 (1998) |

(continued)

| Probe ID | Gene Symbol | GenBank Accession Number | Version Number | Reference |
|---|---|---|---|---|
| 217025_s_at | DBN1 | AL110225 | AL110225.1 GI:5817161 | |
| 32137_at | JAG2 | Y14330 | Y14330.1 GI:2765401 | |
| 212364_at | MYO1B | BF432550 | gi: 11444700 | |
| 210854_x_at | SLC6A8 | U17986 | U17986.1 GI:602433 | Barnwell,L.F. et al., Gene 159 (2), 287-288 (1995) |
| 212739_s_at | NME4 | AL523860 | gi: 45699124 | |
| 203505_at | ABCA1 | AF285167 | AF285167.1 GI:9755158 | |
| 39248_at | AQP3 | N74607 | gi: 1231892 | |
| 221480_at | HNRPD | BG 180941 | gi: 12687644 | |
| 213222_at | PLCB1 | AL049593 | AL049593.10 GI:10443476 | |
| 201296_s_at | WSB1 | NM_015626 | NM_015626.8 GI:58331181 | Choi,D.W. et al., J. Biol. Chem. 283 (8), 4682-4689 (2008) |
| 211944_at | BAT2D1 | BE729523 | gi: 10143515 | |
| 207029_at | KITLG | NM_000899 | NM_000899.3 GI:59939901 | Kasamatsu,S. et al., J. Invest. Dermatol. 128 (7), 1763-1772 (2008) |
| 217875_s_at | TMEPAI | NM_020182 | NM_020182.3 GI:40317614 | Richter,E. et al., Epigenetics 2 (2), 100-109 (2007) |

**References**

Anselm IA, Coulter DL, Darras BT. Cardiac manifestations in a child with a novel mutation in creatine transporter gene SLC6A8. Neurology. 2008 Apr 29;70(18):1642-4.

Barnwell LF, Chaudhuri G, Townsel JG. Cloning and sequencing of a cDNA encoding a novel member of the human brain GABA/noradrenaline neurotransmitter transporter family. Gene. 1995 Jul 4;159(2):287-8.

Britton CH, et al. Fine chromosome mapping of the genes for human liver and muscle carnitine palmitoyltransferase I (CPT1A and CPT1B). Genomics. 1997 Feb 15;40(1):209-11.

Burfoot RK, et al. SNP mapping and candidate gene sequencing in the class I region of the HLA complex: searching for multiple sclerosis susceptibility genes in Tasmanians. Tissue Antigens. 2008 Jan;71(1):42-50.

Chang H.Y., et al (2005). Robustness, scalability, and integration of a wound-response gene expression signature in predicting breast cancer survival. Proc. Natl. Acad. Sci. USA 102(10):3738-43.

Chi, J.T., et al. Gene expression programs in response to hypoxia: cell type specificity and prognostic significance in human cancers. PLoS Med. 3(3):e47 (2006).

Choi DW et al. Ubiquitination and degradation of homeodomain-interacting protein kinase 2 by WD40 repeat/SOCS box protein WSB-1. J Biol Chem. 2008 Feb 22;283(8):4682-9.

Cliffe ST, et al. The first prenatal diagnosis for veno-occlusive disease and immunodeficiency syndrome, an autosomal recessive condition associated with mutations in SP110. Prenat Diagn. 2007 Jul;27(7):674-6.

Deloukas P, et al. The DNA sequence and comparative analysis of human chromosome 20. Nature. 2001 Dec 20-27;414(6866):865-71.

Deshpande A et al. Variation in HLA class I antigen-processing genes and susceptibility to human papillomavirus type 16-associated cervical cancer. J Infect Dis. 2008 Feb 1;197(3):371-81.

Fountoulakis S, et al. Differential expression of Fas system apoptotic molecules in peripheral lymphocytes from patients with Graves' disease and Hashimoto's thyroiditis. Eur J Endocrinol. 2008 Jun;158(6):853-9.

Goldhirsch A, et al., Panel members. Meeting highlights: updated international expert consensus on the primary therapy of early breast cancer. J Clin Oncol. 2003;21:3357-3365

Goto Y, Hattori A, Ishii Y, Tsujimoto M. Reduced activity of the hypertension-associated Lys528Arg mutant of human adipocyte-derived leucine aminopeptidase (A-LAP)/ER-aminopeptidase-1. FEBS Lett. 2006 Mar 20;580(7):1833-8.

Grassi G, et al. The expression levels of the translational factors eEF1A 1/2 correlate with cell growth but not apoptosis in hepatocellular carcinoma cell lines with different differentiation grade. Biochimie. 2007 Dec;89(12): 1544-52.

Harris L, Fritsche H, Mennel R, et al. American Society of Clinical Oncology 2007 update of recommendations for the use of tumor markers in breast cancer. J. Clin. Oncol. 2007;25:5287-310.

Henneberry AL, Wistow G, McMaster CR. Cloning, genomic organization, and characterization of a human cholinephosphotransferase. J Biol Chem. 2000 Sep 22;275(38):29808-15.

Hess, K.R., et al. Pharmacogenomic predictor of sensitivity to preoperative chemotherapy with paclitaxel and fluorouracil, doxorubicin, and cyclophosphamide in breast cancer. J. Clin. Oncology 24(26), 4236-4244 (2006).

Ishitani A, Geraghty DE. Alternative splicing of HLA-G transcripts yields proteins with primary structures resembling both class I and class II antigens. Proc Natl Acad Sci U S A. 1992 May 1;89(9):3947-51.

Jakobsson L, et al. Laminin deposition is dispensable for vasculogenesis but regulates blood vessel diameter independent of flow. FASEB J. 2008 May;22(5):1530-9.

Kasamatsu S, et al. Production of the soluble form of KIT, s-KIT, abolishes stem cell factor-induced melanogenesis in human melanocytes. J Invest Dermatol. 2008 Jul;128(7):1763-72.

Keane MM, et al. Cloning and characterization of cbl-b: a SH3 binding protein with homology to the c-cbl proto-oncogene. Oncogene. 1995 Jun 15;10(12):2367-77.

Kim M, et al. TRAIL inactivates the mitotic checkpoint and potentiates death induced by microtubule-targeting agents in human cancer cells. Cancer Res. 2008 May 1;68(9):3440-9.

Kimura K, et al. Diversification of transcriptional modulation: large-scale identification and characterization of putative alternative promoters of human genes. Genome Res. 2006 Jan;16(1):55-65.

Lee YJ, et al. Serum galectin-3 and galectin-3 binding protein levels in Behçet's disease and their association with disease activity. Clin Exp Rheumatol. 2007 Jul-Aug;25(4 Suppl 45):S41-5.

Lindsell CE, et al. Jagged: a mammalian ligand that activates Notch1. Cell. 1995 Mar 24;80(6):909-17. Liu R., et al (2007) The prognostic role of a gene signature from tumorigenic breast-cancer cells. N. Engl. J. Med. 356(3):217-26.

Maere S., Heymans K., Kuiper M. (2005) BiNGO: A Cytoscape plugin to assess overrepresentation of Gene Ontology categories in biological networks. Bioinformatics 21:3448-49.

Martinez-Quiles N, et al. Description of two new HLA-DRB alleles (DRB1*0310 and DRB3*01012) found in a Spanish infant. Tissue Antigens. 1997 Jun;49(6):658-61.

Massagué J. (2007) Sorting out breast-cancer signatures. N. Engl. J. Med. 356(3)294-7.

Mazaki Y, Hashimoto S, Sabe H. Monocyte cells and cancer cells express novel paxillin isoforms with different binding properties to focal adhesion proteins. J Biol Chem. 1997 Mar 14;272(11):7437-44.

Minn A.J., et al (2005) Genes that mediate breast cancer metastasis to lung. Nature 436(7050):518-24.

Olsen JV, et al. Global, in vivo, and site-specific phosphorylation dynamics in signaling networks. Cell. 2006 Nov 3; 127(3):635-48.

Ota T, et al. Complete sequencing and characterization of 21,243 full-length human cDNAs. Nat Genet. 2004 Jan; 36(1):40-5.

Papoff G, et al. An N-terminal domain shared by Fas/Apo-1 (CD95) soluble variants prevents cell death in vitro. J Immunol. 1996 Jun 15;156(12):4622-30.

Peters U, et al. Variation in the selenoenzyme genes and risk of advanced distal colorectal adenoma. Cancer Epidemiol Biomarkers Prev. 2008 May;17(5):1144-54.

Plotnikov A, et al. Oncogene-mediated inhibition of glycogen synthase kinase 3 beta impairs degradation of prolactin receptor. Cancer Res. 2008 Mar 1;68(5):1354-61.

Purdue MP, et al. Genetic variation in the inhibin pathway and risk of testicular germ cell tumors. Cancer Res. 2008 Apr 15;68(8):3043-8.

Radley E, et al. Genomic organization of HLA-DMA and HLA-DMB. Comparison of the gene organization of all six class II families in the human major histocompatibility complex. J Biol Chem. 1994 Jul 22;269(29):18834-8.

Rexer BN, Zheng WL, Ong DE. Retinoic acid biosynthesis by normal human breast epithelium is via aldehyde dehydrogenase 6, absent in MCF-7 cells. Cancer Res. 2001 Oct 1;61(19):7065-70.

Richter E, et al. A role for DNA methylation in regulating the growth suppressor PMEPA1 gene in prostate cancer. Epigenetics. 2007 Apr-Jun;2(2):100-9.

Rodriguez SG, Johnson AH, Hurley CK. Molecular characterization of HLA-B71 from an African American individual. Hum Immunol. 1993 Jul;37(3):192-4.

Scherer SW, et al (2007) Molecular definition of breast tumor heterogeneity. Cancer Cell 11(3):259-73.

Simon R. (2008) The use of genomics in clinical trial design. Clin Cancer Res. 14(19):5984-93.

Sørlie T., et al (2001) Gene expression patterns of breast carcinomas distinguish tumor subclasses with clinical implications. Proc. Natl. Acad. Sci. USA 98(19):10869-74.

Sotiriou C., et al (2006) Gene expression profiling in breast cancer: understanding the molecular basis of histologic grade to improve prognosis. J. Natl. Cancer Inst. 98(4):262-72.

Soundravally R, Hoti SL. Polymorphisms of the TAP 1 and 2 gene may influence clinical outcome of primary dengue viral infection. Scand J Immunol. 2008 Jun;67(6):618-25.

Stamenkovic I, Amiot M, Pesando JM, Seed B. A lymphocyte molecule implicated in lymph node homing is a member of the cartilage link protein family. Cell. 1989 Mar 24;56(6):1057-62.

Strausberg RL, et al. Mammalian Gene Collection Program Team. Generation and initial analysis of more than 15,000 full-length human and mouse cDNA sequences. Proc Natl Acad Sci U S A. 2002 Dec 24;99(26):16899-903.

Szöts H, et al. Complete sequence of HLA-B27 cDNA identified through the characterization of structural markers unique to the HLA-A, -B, and -C allelic series. Proc Natl Acad Sci U S A. 1986 Mar;83(5):1428-32.

Takahashi K, et al. Dynamic regulation of p53 subnuclear localization and senescence by MORC3. Mol Biol Cell. 2007 May;18(5):1701-9.

Uren AG, et al. Cloning and expression of apoptosis inhibitory protein homologs that function to inhibit apoptosis and/or bind tumor necrosis factor receptor-associated factors. Proc Natl Acad Sci U S A. 1996 May 14;93(10):4974-8.

Uzan B, et al. Adrenomedullin is anti-apoptotic in osteoblasts through CGRP1 receptors and MEK-ERK pathway. J Cell Physiol. 2008 Apr;215(1):122-8.

van't Veer L.J., et al (2002) Gene expression profiling predicts clinical outcome of breast cancer. Nature 415(6871): 530-6.

Vidal-Taboada JM, et al. High resolution physical mapping and identification of transcribed sequences in the Down syndrome region-2. Biochem Biophys Res Commun. 1998 Feb 13;243(2):572-8.

Wang, Y., et al. (2005). Gene-expression profiles to predict distant metastasis of lymph-node-negative primary breast cancer. Lancet 365(9460):671-9.

Wells RS, et al. Cw*1701 defines a divergent african HLA-C allelic lineage. Immunogenetics. 1997;46(3):173-80.

Xu XR, et al. Insight into hepatocellular carcinogenesis at transcriptome level by comparing gene expression profiles of hepatocellular carcinoma with those of corresponding noncancerous liver. Proc Natl Acad Sci U S A. 2001 Dec 18;98(26):15089-94.

Xu, X., et al. IFN-gamma induces cell growth inhibition by Fas-mediated apoptosis: requirement of STAT1 protein for up-regulation of Fas and FasL expression. Cancer Res. 58, 2832-2837 (1998).

Yoshikawa T, et al. Multiple transcriptional variants and RNA editing in C18orf1, a novel gene with LDLRA and transmembrane domains on 18p11.2. Genomics. 1998 Jan 15;47(2):246-57.

Yoshitake N, et al. Expression of SDF-1 alpha and nuclear CXCR4 predicts lymph node metastasis in colorectal cancer. Br J Cancer. 2008 May 20;98(10):1682-9.

## Claims

1. Use of an expression level of a gene set for the identification of breast cancer likely to progress to an invasive phenotype, the gene set consisting of the genes ABCA1, ADD3, ADFP, ADM, ALDH1A3, AQP3, ARHGAP26, B2M, BAT2D1, BIRC3, BRWD1, C18ORF1, CBLB, CD44, CHKB, CHPT1, CMKOR1, CXCL12, DBN1, EEF1A2, FAS, FLJ11000, FLJ11286, FLRT3, HLA-A, HLA-B, HLA-C, HLA-DMA, HLA-DRB1, HLA-DRB4, HLA-DRB5, HLA-F, HLA-G, HNRPD, IFITM1, IFITM3, INHBB, ISG20, JAG1, JAG2, KITLG, LAMC1, LAP3, LGALS3BP, MYO1B, NME4, PLCB1, PRLR, PSMB9, PXN, RAB14, SEMA3C, SEPP1, SLC6A8, SP100, SP110, STS, TAP1, TMEPAI, TNFSF10, TRAM1, TRIM14, and WSB1.

2. A method of stratifying animals, optionally patients, further optionally human patients, into cohorts, the method comprising the steps of:

   a. determining an expression level of a gene set, the gene set consisting of the genes ABCA1, ADD3, ADFP, ADM, ALDH1A3, AQP3, ARHGAP26, B2M, BAT2D1, BIRC3, BRWD1, C18ORF1, CBLB, CD44, CHKB, CHPT1, CMKOR1, CXCL12, DBN1, EEF1A2, FAS, FLJ11000, FLJ11286, FLRT3, HLA-A, HLA-B, HLA-C, HLA-DMA, HLA-DRB1, HLA-DRB4, HLA-DRB5, HLA-F, HLA-G, HNRPD, IFITM1, IFITM3, INHBB, ISG20, JAG1, JAG2, KITLG, LAMC1, LAP3, LGALS3BP, MYO1B, NME4, PLCB1, PRLR, PSMB9, PXN, RAB14, SEMA3C, SEPP1, SLC6A8, SP100, SP110, STS, TAP1, TMEPAI, TNFSF10, TRAM1, TRIM14, and WSB1;

b. identifying breast cancer likely to progress to an invasive phenotype based on the expression level of the genes selected; and

c. stratifying animals into cohorts based on the likelihood to progress to the invasive phenotype.

3. A method according to Claim 2, wherein the determining step further comprises the step of comparing the expression level of each gene to a normal control.

4. Use according to Claim 1 or a method according to Claim 2 or 3, wherein the gene set is divided into at least two subsets.

5. Use or a method according to Claim 4, wherein a first subset consists of the gene set having an expression level in a disease setting relatively higher to the normal, and a second subset consists of the gene set having an expression level in a disease setting relatively lower to the normal.

6. Use or a method according to Claim 4 or 5, wherein the first subset consists of the genes ABCA1, ADFP, ADM, ALDH1A3, AQP3, BAT2D1, BRWD1, C18ORF1, CBLB, CMKOR1, DBN1, EEF1A2, FLRT3, HNRPD, INHBB, JAG1, JAG2, KITLG, LAMC1, MYO1B, NME4, PLCB1, PXN, SLC6A8, TMEPAI, TRAM1, and WSB1.

7. Use or a method according to any one of Claims 4-6, wherein the second subset consists of the genes ADD3, ARHGAP26, B2M, BIRC3, CD44, CHKB, CHPT1, CXCL12, FAS, FLJ11000, FLJ11286, HLA-A, HLA-B, HLA-C, HLA-DMA, HLA-DRB1, HLA-DRB4, HLA-DRB5, HLA-F, HLA-G, IFITM1, IFITM3, ISG20, LAP3, LGALS3BP, PRLR, PSMB9, RAB14, SEMA3C, SEPP1, SP100, SP110, STS, TAP1, TNFSF10, and TRIM14.

8. A method according to any of Claims 4-7, wherein the identifying step involves comparing the gene expression profile of the genes from the first subset, and the gene expression profile of the genes from the second subset.

9. An array for expression profiling, the array consisting of polynucleotides, and complementary sequences thereof, that specifically hybridise to the genes ABCA1, ADD3, ADFP, ADM, ALDH1A3, AQP3, ARHGAP26, B2M, BAT2D1, BIRC3, BRWD1, C18ORF1, CBLB, CD44, CHKB, CHPT1, CMKOR1, CXCL12, DBN1, EEF1A2, FAS, FLJ11000, FLJ11286, FLRT3, HLA-A, HLA-B, HLA-C, HLA-DMA, HLA-DRB1, HLA-DRB4, HLA-DRB5, HLA-F, HLA-G, HNRPD, IFITM1, IFITM3, INHBB, ISG20, JAG1, JAG2, KITLG, LAMC1, LAP3, LGALS3BP, MYO1B, NME4, PLCB1, PRLR, PSMB9, PXN, RAB14, SEMA3C, SEPP1, SLC6A8, SP100, SP110, STS, TAP1, TMEPAI, TNFSF10, TRAM1, TRIM14, and WSB1.

10. An array according to Claim 9, wherein the gene set is divided into at least two subsets.

11. An array according to Claim 10, wherein the first subset consists of the genes ABCA1, ADFP, ADM, ALDH1A3, AQP3, BAT2D1, BRWD1, C18ORF1, CBLB, CMKOR1, DBN1, EEF1A2, FLRT3, HNRPD, INHBB, JAG1, JAG2, KITLG, LAMC1, MYO1B, NME4, PLCB1, PXN, SLC6A8, TMEPAI, TRAM1, and WSB1.

12. An array according to Claim 10 or 11, wherein the second subset consists of the genes ADD3, ARHGAP26, B2M, BIRC3, CD44, CHKB, CHPT1, CXCL12, FAS, FLJ11000, FLJ11286, HLA-A, HLA-B, HLA-C, HLA-DMA, HLA-DRB1, HLA-DRB4, HLA-DRB5, HLA-F, HLA-G, IFITM1, IFITM3, ISG20, LAP3, LGALS3BP, PRLR, PSMB9, RAB14, SEMA3C, SEPP1, SP100, SP110, STS, TAP1, TNFSF10, and TRIM14.

**Patentansprüche**

1. Verwendung eines Expressionsniveaus einer Gengruppe für die Identifizierung von Brustkrebs, der sich wahrscheinlich zu einem invasiven Phänotyp entwickelt, wobei die Gengruppe aus folgenden Genen besteht: ABCA1, ADD3, ADFP, ADM, ALDH1A3, AQP3, ARHGAP26, B2M, BAT2D1, BIRC3, BRWD1, C18ORF1, CBLB, CD44, CHKB, CHPT1, CMKOR1, CXCL12, DBN1, EEF1A2, FAS, FLJ11000, FLJ11286, FLRT3, HLA-A, HLA-B, HLA-C, HLA-DMA, HLA-DRB1, HLA-DRB4, HLA-DRB5, HLA-F, HLA-G, HNRPD, IFITM1, IFITM3, INHBB, ISG20, JAG1, JAG2, KITLG, LAMC1, LAP3, LGALS3BP, MYO1B, NME4, PLCB1, PRLR, PSMB9, PXN, RAB14, SEMA3C, SEPP1, SLC6A8, SP100, SP110, STS, TAP1, TMEPAI, TNFSF10, TRAM1, TRIM14 und WSB1.

2. Verfahren zur Stratifizierung von Tieren, optional Patienten, weiter optional menschlichen Patienten, in Kohorten, wobei das Verfahren die folgenden Schritte umfasst:

    a. Bestimmen eines Expressionsniveaus einer Gengruppe, wobei die Gengruppe aus den folgenden Genen besteht: ABCA1, ADD3, ADFP, ADM, ALDH1A3, AQP3, ARHGAP26, B2M, BAT2D1, BIRC3, BRWD1, C18ORF1, CBLB, CD44, CHKB, CHPT1, CMKOR1, CXCL12, DBN1, EEF1A2, FAS, FLJ11000, FLJ11286, FLRT3, HLA-A, HLA-B, HLA-C, HLA-DMA, HLA-DRB1, HLA-DRB4, HLA-DRB5, HLA-F, HLA-G, HNRPD, IFITM1, IFITM3, INHBB, ISG20, JAG1, JAG2, KITLG, LAMC1, LAP3, LGALS3BP, MYO1B, NME4, PLCB1, PRLR, PSMB9, PXN, RAB14, SEMA3C, SEPP1, SLC6A8, SP100, SP110, STS, TAP1, TMEPAI, TNFSF10, TRAM1, TRIM14 und WSB1;
    b. Identifizieren von Brustkrebs, der sich wahrscheinlich zu einem invasiven Phänotyp entwickelt, auf der Grundlage des Expressionsniveaus der ausgewählten Gene; und
    c. Stratifizieren von Tieren in Kohorten auf der Grundlage der Wahrscheinlichkeit der Weiterentwicklung zu dem invasiven Phänotyp.

3. Verfahren nach Anspruch 2, worin der Bestimmungsschritt weiter den Schritt des Vergleichens des Expressionsniveaus von jedem Gen mit einer normalen Kontrolle umfasst.

4. Verwendung nach Anspruch 1 oder ein Verfahren nach Anspruch 2 oder 3, worin die Gengruppe in mindestens zwei Untergruppen eingeteilt ist.

5. Verwendung oder ein Verfahren nach Anspruch 4, worin eine erste Untergruppe aus der Gengruppe besteht, die ein Expressionsniveau in einer Krankheitssituation aufweist, das höher im Vergleich zum normalen ist, und eine zweite Untergruppe aus der Gengruppe besteht, die ein Expressionsniveau in einer Krankheitssituation aufweist, das niedriger im Vergleich zum normalen ist.

6. Verwendung oder ein Verfahren nach Anspruch 4 oder 5, worin die erste Untergruppe aus den folgenden Genen besteht: ABCA1, ADFP, ADM, ALDH1A3, AQP3, BAT2D1, BRWD1, C18ORF1, CBLB, CMKOR1, DBN1, EEF1A2, FLRT3, HNRPD, INHBB, JAG1, JAG2, KITLG, LAMC1, MYO1B, NME4, PLCB1, PXN, SLC6A8, TMEPAI, TRAM1 und WSB1.

7. Verwendung oder ein Verfahren nach einem der Ansprüche 4-6, worin die zweite Untergruppe aus den folgenden Genen besteht: ADD3, ARHGAP26, B2M, BIRC3, CD44, CHKB, CHPT1, CXCL12, FAS, FLJ11000, FLJ11286, HLA-A, HLA-B, HLA-C, HLA-DMA, HLA-DRB1, HLA-DRB4, HLA-DRB5, HLA-F, HLA-G, IFITM1, IFITM3, ISG20, LAP3, LGALS3BP, PRLR, PSMB9, RAB14, SEMA3C, SEPP1, SP100, SP110, STS, TAP1, TNFSF10 und TRIM14.

8. Verfahren nach einem der Ansprüche 4-7, worin der Identifizierungsschritt das Vergleichen des Genexpressionsprofils der Gene aus der ersten Untergruppe und des Genexpressionsprofils der Gene aus der zweiten Untergruppe einschließt.

9. Array zur Erstellung von Expressionsprofilen, wobei das Array aus Polynukleotiden und komplementären Sequenzen davon besteht, die spezifisch mit den folgenden Genen hybridisieren: ABCA1, ADD3, ADFP, ADM, ALDH1A3, AQP3, ARHGAP26, B2M, BAT2D1, BIRC3, BRWD1, C18ORF1, CBLB, CD44, CHKB, CHPT1, CMKOR1, CXCL12, DBN1, EEF1A2, FAS, FLJ11000, FLJ11286, FLRT3, HLA-A, HLA-B, HLA-C, HLA-DMA, HLA-DRB1, HLA-DRB4, HLA-DRB5, HLA-F, HLA-G, HNRPD, IFITM1, IFITM3, INHBB, ISG20, JAG1, JAG2, KITLG, LAMC1, LAP3, LGALS3BP, MYO1B, NME4, PLCB1, PRLR, PSMB9, PXN, RAB14, SEMA3C, SEPP1, SLC6A8, SP100, SP110, STS, TAP1, TMEPAI, TNFSF10, TRAM1, TRIM14 und WSB1.

10. Array nach Anspruch 9, worin die Gengruppe in mindestens zwei Untergruppen eingeteilt ist.

11. Array nach Anspruch 10, worin die erste Untergruppe aus den folgenden Genen besteht: ABCA1, ADFP, ADM, ALDH1A3, AQP3, BAT2D1, BRWD1, C18ORF1, CBLB, CMKOR1, DBN1, EEF1A2, FLRT3, HNRPD, INHBB, JAG1, JAG2, KITLG, LAMC1, MYO1B, NME4, PLCB1, PXN, SLC6A8, TMEPAI, TRAM1 und WSB1.

12. Array nach Anspruch 10 oder 11, worin die zweite Untergruppe aus den folgenden Genen besteht: ADD3, ARHGAP26, B2M, BIRC3, CD44, CHKB, CHPT1, CXCL12, FAS, FLJ11000, FLJ11286, HLA-A, HLA-B, HLA-C, HLA-DMA, HLA-DRB1, HLA-DRB4, HLA-DRB5, HLA-F, HLA-G, IFITM1, IFITM3, ISG20, LAP3, LGALS3BP, PRLR, PSMB9, RAB14, SEMA3C, SEPP1, SP100, SP110, STS, TAP1, TNFSF10 und TRIM14.

**Revendications**

1. Utilisation d'un taux d'expression d'un set de gènes pour l'identification d'un cancer du sein susceptible de progresser vers un phénotype invasif, le set de gènes étant constitué des gènes ABCA1, ADD3, ADFP, ADM, ALDH1A3, AQP3, ARHGAP26, B2M, BAT2D1, BIRC3, BRWD1, C18ORF1, CBLB, CD44, CHKB, CHPT1, CMKOR1, CXCL12, DBN1, EEF1A2, FAS, FLJ11000, FLJ11286, FLRT3, HLA-A, HLA-B, HLA-C, HLA-DMA, HLA-DRB1, HLA-DRB4, HLA-DRB5, HLA-F, HLA-G, HNRPD, IFITM1, IFITM3, INHBB, ISG20, JAG1, JAG2, KITLG, LAMC1, LAP3, LGALS3BP, MYO1B, NME4, PLCB1, PRLR, PSMB9, PXN, RAB14, SEMA3C, SEPP1, SLC6A8, SP100, SP110, STS, TAP1, TMEPAI, TNFSF10, TRAM1, TRIM14, et WSB1.

2. Méthode de stratification d'animaux, éventuellement de patients, de même éventuellement de patients humains, en cohortes, la méthode comprenant les étapes consistant à :

   a. déterminer un taux d'expression d'un set de gènes, le set de gènes étant constitué des gènes ABCA1, ADD3, ADFP, ADM, ALDH1A3, AQP3, ARHGAP26, B2M, BAT2D1, BIRC3, BRWD1, C18ORF1, CBLB, CD44, CHKB, CHPT1, CMKOR1, CXCL12, DBN1, EEF1A2, FAS, FLJ11000, FLJ11286, FLRT3, HLA-A, HLA-B, HLA-C, HLA-DMA, HLA-DRB1, HLA-DRB4, HLA-DRB5, HLA-F, HLA-G, HNRPD, IFITM1, IFITM3, INHBB, ISG20, JAG1, JAG2, KITLG, LAMC1, LAP3, LGALS3BP, MYO1B, NME4, PLCB1, PRLR, PSMB9, PXN, RAB14, SEMA3C, SEPP1, SLC6A8, SP100, SP110, STS, TAP1, TMEPAI, TNFSF10, TRAM1, TRIM14, et WSB1 ;
   b. identifier le cancer du sein susceptible de progresser vers un phénotype invasif sur la base du taux d'expression des gènes sélectionnés ; et
   c. stratifier les animaux en cohortes sur la base de la probabilité d'une progression vers le phénotype invasif.

3. Méthode selon la revendication 2, dans laquelle l'étape de détermination comprend en outre consistant à comparer le taux d'expression de chaque gène avec un témoin normal.

4. Utilisation selon la revendication 1 ou méthode selon la revendication 2 ou 3, dans laquelle le set de gènes est divisé en au moins deux sous-sets.

5. Utilisation ou méthode selon la revendication 4, dans laquelle un premier sous-set est constitué du set de gènes ayant un taux d'expression dans un cadre pathologique relativement supérieur à la normale, et un deuxième sous-set est constitué du set de gènes ayant un taux d'expression dans un cadre pathologique relativement inférieur à la normale.

6. Utilisation ou méthode selon la revendication 4 ou 5, dans laquelle le premier sous-set est constitué des gènes ABCA1, ADFP, ADM, ALDH1A3, AQP3, BAT2D1, BRWD1, C18ORF1, CBLB, CMKOR1, DBN1, EEF1A2, FLRT3, HNRPD, INHBB, JAG1, JAG2, KITLG, LAMC1, MYO1B, NME4, PLCB1, PXN, SLC6A8, TMEPAI, TRAM1, et WSB1.

7. Utilisation ou méthode selon l'une quelconque des revendications 4-6, dans laquelle le deuxième sous-set est constitué des gènes ADD3, ARHGAP26, B2M, BIRC3, CD44, CHKB, CHPT1, CXCL12, FAS, FLJ11000, FLJ11286, HLA-A, HLA-B, HLA-C, HLA-DMA, HLA-DRB1, HLA-DRB4, HLA-DRB5, HLA-F, HLA-G, IFITM1, IFITM3, ISG20, LAP3, LGALS3BP, PRLR, PSMB9, RAB14, SEMA3C, SEPP1, SP100, SP110, STS, TAP1, TNFSF10, et TRIM14.

8. Méthode selon l'une quelconque des revendications 4-7, dans laquelle l'étape d'identification met en jeu la comparaison du profil d'expression génétique des gènes issus du premier sous-set avec le profil d'expression génétique des gènes issus du deuxième sous-set.

9. Puce de profilage d'expression, la puce étant constituée de polynucléotides, et de séquences complémentaires de ceux-ci, s'hybridant spécifiquement aux gènes ABCA1, ADD3, ADFP, ADM, ALDH1A3, AQP3, ARHGAP26, B2M, BAT2D1, BIRC3, BRWD1, C18ORF1, CBLB, CD44, CHKB, CHPT1, CMKOR1, CXCL12, DBN1, EEF1A2, FAS, FLJ11000, FLJ11286, FLRT3, HLA-A, HLA-B, HLA-C, HLA-DMA, HLA-DRB1, HLA-DRB4, HLA-DRB5, HLA-F, HLA-G, HNRPD, IFITM1, IFITM3, INHBB, ISG20, JAG1, JAG2, KITLG, LAMC1, LAP3, LGALS3BP, MYO1B, NME4, PLCB1, PRLR, PSMB9, PXN, RAB14, SEMA3C, SEPP1, SLC6A8, SP100, SP110, STS, TAP1, TMEPAI, TNFSF10, TRAM1, TRIM14, et WSB1.

10. Puce selon la revendication 9, dans laquelle le set de gènes est divisé en au moins deux sous-sets.

11. Puce selon la revendication 10, dans laquelle le premier sous-set est constitué des gènes ABCA1, ADFP, ADM, ALDH1A3, AQP3, BAT2D1, BRWD1, C18ORF1, CBLB, CMKOR1, DBN1, EEF1A2, FLRT3, HNRPD, INHBB, JAG1, JAG2, KITLG, LAMC1, MYO1B, NME4, PLCB1, PXN, SLC6A8, TMEPAI, TRAM1, et WSB1.

12. Puce selon la revendication 10 ou 11, dans laquelle le deuxième sous-set est constitué des gènes ADD3, ARHGAP26, B2M, BIRC3, CD44, CHKB, CHPT1, CXCL12, FAS, FLJ11000, FLJ11286, HLA-A, HLA-B, HLA-C, HLA-DMA, HLA-DRB1, HLA-DRB4, HLA-DRB5, HLA-F, HLA-G, IFITM1, IFITM3, ISG20, LAP3, LGALS3BP, PRLR, PSMB9, RAB14, SEMA3C, SEPP1, SP100, SP110, STS, TAP1, TNFSF10, et TRIM14.

**Figure 1A**

Data set 2:125 patients

Bottom 75%     Top 25% of patients

Figure 1A

Figure 1A

Figure 1B

Figure 2

Figure 3

Figure 4

Figure 5

Figure 6

Figure 7

Figure 8

Figure 9

Figure 10

Figure 11

Figure 12

Figure 13

Figure 14

Analysis of differential gene
expression in
MCF7-I0 and MCF7-I6

54675 probe sets

Filter #1:
Absolute fold change >= 2
and no absent call in either
MCF7-I0 or MCF7-I6 ?

430 genes

Filter #2:
Keep only genes
contained in all data sets

289 genes

Learning:
Univariate Cox proportional hazards
regression with bootstrapping
using data sets 1 and 2

Test:
Apply tandem signature to data sets
3, 4, 5 and 6

Filter #3:
Bootstrapped estimate of Cox p-
value smaller than 0.15 in either
data set 1 or 2 or both ?

158 genes

Apply:

Order samples based on
average expression in down-cassette
minus average expression in up-cassette.

Filter #4:
Cox coefficient negative in both
data sets 1 and 2 and probe set
under-expressed in MCF7-I6 ? OR:
Cox coefficient positive in both
data sets 1 and 2 and probe set
over-expressed in MCF7-I6 ?

63 genes

Tandem signature consisting of
down-cassette (55 probe sets) and
up-cassette (33 probes sets)

EP 2 370 595 B1

Figure 15A

Figure 15B

Figure 16A

Data set 4:200 patients

Figure 16B

Data set 5:64 patients

Top 30% of patients

Color Key

Low High

**Figure 17A**

Down-cassette

Up-cassette

ER Pos■, Neg☐

Mets ■,No mets☐

Positive LN (0▨, 1▨, 2☐, 3■ )

Tumor size (≤2cm■,>2cm ☐ )

Intrinsic subtypes
(normal▨, ERBB2+■, basal▨, luminal▨)

Wound-response (activated■, quiescent ☐)

70-genes (poor■, good☐ )

48-genes (lung mets ■, no lung mets☐)

Figure 17B

Color Key

Low — High

Data set 6: 149 patients

Top 25% of patients

Down-cassette

Up-cassette

ER Pos■, Neg☐

Event ■, No Event ☐

Tumor size (≤2cm■, >2cm☐)

Grade (1■, 2☐, 3, ⊠)

Intrinsic subtypes
(normal▨, ERBB2+■, basal▨, luminal▨)

Wound-response (activated ■, quiescent☐)

70-genes (poor■, good☐)

48-genes (lung mets■, no lung mets☐)

Figure 18

EP 2 370 595 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1961825 A

**Non-patent literature cited in the description**

- **HARRIS et al.** *American Society of Clinical Oncology,* 2007
- *J. Clin. Oncol.,* 2007, vol. 25, 5287-310
- **SIMON R.** The use of genomics in clinical trial design. *Clin Cancer Res.,* 2008, vol. 14 (19), 5984-93
- **HESS K.R. ; ANDERSON K. ; SYMMANS W.F. et al.** Pharmacogenomic predictor of sensitivity to preoperative chemotherapy with paclitaxel and fluorouracil, doxorubicin, and cyclophosphamide in breast cancer. *J Clin. Oncology,* 2006, vol. 24 (26), 4236-44
- **MINN, A. J. et al.** Genes that mediate breast cancer metastasis to lung. *Nature,* 2005, vol. 436 (7050), 518-524
- **RADLEY, E. et al.** *J. Biol. Chem.,* 1994, vol. 269 (29), 18834-18838
- **LACAP, P.A. et al.** *AIDS,* 2008, vol. 22 (9), 1029-1038
- **MARTINEZ-QUILES, N. et al.** *Tissue Antigens,* 1997, vol. 49 (6), 658-661
- **SCHERER, S.W. et al.** *Science,* 2003, vol. 300 (5620), 767-772
- **STAMENKOVIC, I. et al.** *Cell,* 1989, vol. 56 (6), 1057-1062
- **STRAUSBERG, R.L et al.** *Proc. Natl. Acad. Sci. U.S.A.,* 2002, vol. 99 (26), 16899-16903
- **YOSHITAKE, N. et al.** *Br. J. Cancer,* 2008, vol. 98 (10), 1682-1689
- **HILLIER, L et al.** *Genome Res.,* 1996, vol. 6 (9), 807-828
- **SZOTS, H. et al.** *Proc. Natl. Acad. Sci. U.S.A.,* 1986, vol. 83 (5), 1428-1432
- **KIMURA, K. et al.** *Genome Res.,* 2006, vol. 16 (1), 55-65
- **KIM, M et al.** *Cancer Res.,* 2008, vol. 68 (9), 3440-3449
- **FOUNTOULAKIS, S. et al.** *Eur. J. Endocrinol.,* 2008, vol. 158 (6), 853-859
- **PAPOFF, G. et al.** *J. Immunol.,* 1996, vol. 156 (12), 4622-4630
- **WELLS, R.S. et al.** *Immunogenetics,* 1997, vol. 46 (3), 173-180
- **HENNEBERRY, A.L. et al.** *J. Biol. Chem.,* 2000, vol. 275 (38), 29808-29815
- **RODRIGUEZ, S.G. et al.** *Hum. Immunol.,* 1993, vol. 37 (3), 192-194
- **STRAUSBERG, R.L. et al.** *Proc. Natl. Acad. Sci. U.S.A.,* 2002, vol. 99 (26), 16899-16903
- **ISHITANI, A. et al.** *Proc. Natl. Acad. Sci. U.S.A.,* 1992
- **GOTO, Y. et al.** *FEBS Lett.,* 2006, vol. 580 (7), 1833-1838
- **PLOTNIKOV, A. et al.** *Cancer Res.,* 2008, vol. 68 (5), 1354-1361
- **BRITTON, C.H et al.** *Genomics,* 1997, vol. 40 (1), 209-211
- **OTA, T. et al.** *Nat. Genet.,* 2004, vol. 36 (1), 40-45
- **BURFOOT, R.K. et al.** *Tissue Antigens,* 2008, vol. 71 (1), 42-50
- **UREN, A.G. et al.** *Proc. Natl. Acad. Sci. U.S.A.,* 1996, vol. 93 (10), 4974-4978
- **DESHPANDE, A. et al.** *J. Infect. Dis.,* 2008, vol. 197 (3), 371-381
- **PETERS, U. et al.** *Cancer Epidemiol. Biomarkers Prev.,* 2008, vol. 17 (5), 1144-1154
- **CLIFFE, S.T. et al.** *Prenat. Diagn.,* 2007, vol. 27 (7), 674-676
- **XU, X. et al.** *Proc. Natl. Acad. Sci. U.S.A.,* 2001, vol. 98 (26), 15089-15094
- **LEE, Y.J. et al.** *Clin. Exp. Rheumatol.,* 2007, vol. 25 (4), S41-S45
- **TAKAHASHI, K. et al.** *Mol. Biol. Cell,* 2007, vol. 18 (5), 1701-1709
- **SOUNDRAVALLY, R. et al.** *Scand. J. Immunol.,* 2008, vol. 67 (6), 618-625
- **GRASSI, G. et al.** *Biochimie,* 2007, vol. 89 (12), 1544-1552
- **YOSHIKAWA, T. et al.** *Genomics,* 1998, vol. 47 (2), 246-257
- **OLSEN, J.V. et al.** *Cell,* 2006, vol. 127 (3), 635-648
- **UZAN, B. et al.** *J. Cell. Physiol.,* 2008, vol. 215 (1), 122-128
- **MAZAKI, Y. et al.** *J. Biol. Chem.,* 1997, vol. 272 (11), 7437-7444

- **DELOUKAS,P. et al.** *Nature,* 2001, vol. 414 (6866), 865-871
- **ANSELM,I.A. et al.** *Neurology,* 2008, vol. 70 (18), 1642-1644
- **REXER,B.N. et al.** *Cancer Res.,* 2001, vol. 61 (19), 7065-7070
- **KEANE,M.M. et al.** *Oncogene,* 1995, vol. 10 (12), 2367-2377
- **PURDUE,M.P. et al.** *Cancer Res.,* 2008, vol. 68 (8), 3043-3048
- **LINDSELL,C.E. et al.** *Cell,* 1995, vol. 80 (6), 909-917
- **JAKOBSSON,L. et al.** *FASEB J.,* 2008, vol. 22 (5), 1530-1539
- **STRAUSBERG,R.L.** *Proc. Natl. Acad. Sci. U.S.A.,* 2002, vol. 99 (26), 16899-16903
- **STRAUSBERG,R.L. et al.** *Proc. Natl. Acad. Sci. U.S.A.,* 2002, vol. 99 (26), 16899-16903
- **VIDAL-TABOADA,J.M. et al.** *Biochem. Biophys. Res. Commun,* 1998, vol. 243 (2), 572-578
- **BARNWELL,L.F. et al.** *Gene,* 1995, vol. 159 (2), 287-288
- **CHOI,D.W. et al.** *J. Biol. Chem.,* 2008, vol. 283 (8), 4682-4689
- **KASAMATSU,S. et al.** *J. Invest. Dermatol.,* 2008, vol. 128 (7), 1763-1772
- **RICHTER,E. et al.** *Epigenetics,* 2007, vol. 2 (2), 100-109
- **ANSELM IA ; COULTER DL ; DARRAS BT.** Cardiac manifestations in a child with a novel mutation in creatine transporter gene SLC6A8. *Neurology.,* 29 April 2008, vol. 70 (18), 1642-4
- **BARNWELL LF ; CHAUDHURI G ; TOWNSEL JG.** Cloning and sequencing of a cDNA encoding a novel member of the human brain GABA/noradrenaline neurotransmitter transporter family. *Gene,* 04 July 1995, vol. 159 (2), 287-8
- **BRITTON CH et al.** Fine chromosome mapping of the genes for human liver and muscle carnitine palmitoyltransferase I (CPT1A and CPT1B. *Genomics.,* 15 February 1997, vol. 40 (1), 209-11
- **BURFOOT RK et al.** SNP mapping and candidate gene sequencing in the class I region of the HLA complex: searching for multiple sclerosis susceptibility genes in Tasmanians. *Tissue Antigens,* January 2008, vol. 71 (1), 42-50
- **CHANG H.Y. et al.** Robustness, scalability, and integration of a wound-response gene expression signature in predicting breast cancer survival. *Proc. Natl. Acad. Sci. USA,* 2005, vol. 102 (10), 3738-43
- **CHI, J.T. et al.** Gene expression programs in response to hypoxia: cell type specificity and prognostic significance in human cancers. *PLoS Med.,* 2006, vol. 3 (3), e47
- **CHOI DW et al.** Ubiquitination and degradation of homeodomain-interacting protein kinase 2 by WD40 repeat/SOCS box protein WSB-1. *J Biol Chem.,* 22 February 2008, vol. 283 (8), 4682-9
- **CLIFFE ST et al.** The first prenatal diagnosis for veno-occlusive disease and immunodeficiency syndrome, an autosomal recessive condition associated with mutations in SP110. *Prenat Diagn.,* July 2007, vol. 27 (7), 674-6
- **DELOUKAS P et al.** The DNA sequence and comparative analysis of human chromosome 20. *Nature,* 20 December 2001, vol. 414 (6866), 865-71
- **DESHPANDE A et al.** Variation in HLA class I antigen-processing genes and susceptibility to human papillomavirus type 16-associated cervical cancer. *J Infect Dis.,* 01 February 2008, vol. 197 (3), 371-81
- **FOUNTOULAKIS S et al.** Differential expression of Fas system apoptotic molecules in peripheral lymphocytes from patients with Graves' disease and Hashimoto's thyroiditis. *Eur J Endocrinol.,* June 2008, vol. 158 (6), 853-9
- **GOLDHIRSCH A et al.** Panel members. Meeting highlights: updated international expert consensus on the primary therapy of early breast cancer. *J Clin Oncol.,* 2003, vol. 21, 3357-3365
- **GOTO Y ; HATTORI A ; ISHII Y ; TSUJIMOTO M.** Reduced activity of the hypertension-associated Lys528Arg mutant of human adipocyte-derived leucine aminopeptidase (A-LAP)/ER-aminopeptidase-1. *FEBS Lett.,* 20 March 2006, vol. 580 (7), 1833-8
- **GRASSI G et al.** The expression levels of the translational factors eEF1A 1/2 correlate with cell growth but not apoptosis in hepatocellular carcinoma cell lines with different differentiation grade. *Biochimie,* December 2007, vol. 89 (12), 1544-52
- **HARRIS L ; FRITSCHE H ; MENNEL R et al.** American Society of Clinical Oncology 2007 update of recommendations for the use of tumor markers in breast cancer. *J. Clin. Oncol.,* 2007, vol. 25, 5287-310
- **HENNEBERRY AL ; WISTOW G ; MCMASTER CR.** Cloning, genomic organization, and characterization of a human cholinephosphotransferase. *J Biol Chem.,* 22 September 2000, vol. 275 (38), 29808-15
- **HESS, K.R. et al.** Pharmacogenomic predictor of sensitivity to preoperative chemotherapy with paclitaxel and fluorouracil, doxorubicin, and cyclophosphamide in breast cancer. *J. Clin. Oncology,* 2006, vol. 24 (26), 4236-4244
- **ISHITANI A ; GERAGHTY DE.** Alternative splicing of HLA-G transcripts yields proteins with primary structures resembling both class I and class II antigens. *Proc Natl Acad Sci U S A.,* 01 May 1992, vol. 89 (9), 3947-51
- **JAKOBSSON L et al.** Laminin deposition is dispensable for vasculogenesis but regulates blood vessel diameter independent of flow. *FASEB J.,* May 2008, vol. 22 (5), 1530-9

- **KASAMATSU S et al.** Production of the soluble form of KIT, s-KIT, abolishes stem cell factor-induced melanogenesis in human melanocytes. *J Invest Dermatol,* July 2008, vol. 128 (7), 1763-72
- **KEANE MM et al.** Cloning and characterization of cbl-b: a SH3 binding protein with homology to the c-cbl proto-oncogene. *Oncogene,* 15 June 1995, vol. 10 (12), 2367-77
- **KIM M et al.** TRAIL inactivates the mitotic checkpoint and potentiates death induced by microtubule-targeting agents in human cancer cells. *Cancer Res.,* 01 May 2008, vol. 68 (9), 3440-9
- **KIMURA K et al.** Diversification of transcriptional modulation: large-scale identification and characterization of putative alternative promoters of human genes. *Genome Res.,* January 2006, vol. 16 (1), 55-65
- **LEE YJ et al.** Serum galectin-3 and galectin-3 binding protein levels in Behçet's disease and their association with disease activity. *Clin Exp Rheumatol.,* July 2007, vol. 25 (4), 41-5
- **LINDSELL CE et al.** Jagged: a mammalian ligand that activates Notch1. *Cell,* 24 March 1995, vol. 80 (6), 909-17
- **LIU R. et al.** The prognostic role of a gene signature from tumorigenic breast-cancer cells. *N. Engl. J. Med.,* 2007, vol. 356 (3), 217-26
- **MAERE S. ; HEYMANS K. ; KUIPER M.** BiNGO: A Cytoscape plugin to assess overrepresentation of Gene Ontology categories in biological networks. *Bioinformatics,* 2005, vol. 21, 3448-49
- **MARTINEZ-QUILES N et al.** Description of two new HLA-DRB alleles (DRB1*0310 and DRB3*01012) found in a Spanish infant. *Tissue Antigens,* June 1997, vol. 49 (6), 658-61
- **MASSAGUÉ J.** Sorting out breast-cancer signatures. *N. Engl. J. Med,* 2007, vol. 356 (3), 294-7
- **MAZAKI Y ; HASHIMOTO S ; SABE H.** Monocyte cells and cancer cells express novel paxillin isoforms with different binding properties to focal adhesion proteins. *J Biol Chem.,* 14 March 1997, vol. 272 (11), 7437-44
- **MINN A.J. et al.** Genes that mediate breast cancer metastasis to lung. *Nature,* 2005, vol. 436 (7050), 518-24
- **OLSEN JV et al.** Global, in vivo, and site-specific phosphorylation dynamics in signaling networks. *Cell,* 03 November 2006, vol. 127 (3), 635-48
- **OTA T et al.** Complete sequencing and characterization of 21,243 full-length human cDNAs. *Nat Genet,* January 2004, vol. 36 (1), 40-5
- **PAPOFF G et al.** An N-terminal domain shared by Fas/Apo-1 (CD95) soluble variants prevents cell death in vitro. *J Immunol.,* 15 June 1996, vol. 156 (12), 4622-30
- **PETERS U et al.** Variation in the selenoenzyme genes and risk of advanced distal colorectal adenoma. *Cancer Epidemiol Biomarkers Prev.,* May 2008, vol. 17 (5), 1144-54
- **PLOTNIKOV A et al.** Oncogene-mediated inhibition of glycogen synthase kinase 3 beta impairs degradation of prolactin receptor. *Cancer Res.,* 01 March 2008, vol. 68 (5), 1354-61
- **PURDUE MP et al.** Genetic variation in the inhibin pathway and risk of testicular germ cell tumors. *Cancer Res.,* 15 April 2008, vol. 68 (8), 3043-8
- **RADLEY E et al.** Genomic organization of HLA-DMA and HLA-DMB. Comparison of the gene organization of all six class II families in the human major histocompatibility complex. *J Biol Chem.,* 22 July 1994, vol. 269 (29), 18834-8
- **REXER BN ; ZHENG WL ; ONG DE.** Retinoic acid biosynthesis by normal human breast epithelium is via aldehyde dehydrogenase 6, absent in MCF-7 cells. *Cancer Res.,* 01 October 2001, vol. 61 (19), 7065-70
- **RICHTER E et al.** A role for DNA methylation in regulating the growth suppressor PMEPA1 gene in prostate cancer. *Epigenetics,* April 2007, vol. 2 (2), 100-9
- **RODRIGUEZ SG ; JOHNSON AH ; HURLEY CK.** Molecular characterization of HLA-B71 from an African American individual. *Hum Immunol.,* July 1993, vol. 37 (3), 192-4
- **SCHERER SW et al.** Molecular definition of breast tumor heterogeneity. *Cancer Cell,* 2007, vol. 11 (3), 259-73
- **SØRLIE T. et al.** Gene expression patterns of breast carcinomas distinguish tumor subclasses with clinical implications. *Proc. Natl. Acad. Sci. USA,* 2001, vol. 98 (19), 10869-74
- **SOTIRIOU C. et al.** Gene expression profiling in breast cancer: understanding the molecular basis of histologic grade to improve prognosis. *J. Natl. Cancer Inst,* 2006, vol. 98 (4), 262-72
- **SOUNDRAVALLY R ; HOTI SL.** Polymorphisms of the TAP 1 and 2 gene may influence clinical outcome of primary dengue viral infection. *Scand J Immunol.,* June 2008, vol. 67 (6), 618-25
- **STAMENKOVIC I ; AMIOT M ; PESANDO JM ; SEED B.** A lymphocyte molecule implicated in lymph node homing is a member of the cartilage link protein family. *Cell,* 24 March 1989, vol. 56 (6), 1057-62
- **STRAUSBERG RL et al.** Mammalian Gene Collection Program Team. Generation and initial analysis of more than 15,000 full-length human and mouse cDNA sequences. *Proc Natl Acad Sci U S A.,* 24 December 2002, vol. 99 (26), 16899-903

- **SZÖTS H et al.** Complete sequence of HLA-B27 cDNA identified through the characterization of structural markers unique to the HLA-A, -B, and -C allelic series. *Proc Natl Acad Sci U S A.,* March 1986, vol. 83 (5), 1428-32
- **TAKAHASHI K et al.** Dynamic regulation of p53 subnuclear localization and senescence by MORC3. *Mol Biol Cell,* May 2007, vol. 18 (5), 1701-9
- **UREN AG et al.** Cloning and expression of apoptosis inhibitory protein homologs that function to inhibit apoptosis and/or bind tumor necrosis factor receptor-associated factors. *Proc Natl Acad Sci U S A.,* 14 May 1996, vol. 93 (10), 4974-8
- **UZAN B et al.** Adrenomedullin is anti-apoptotic in osteoblasts through CGRP1 receptors and MEK-ERK pathway. *J Cell Physiol.,* April 2008, vol. 215 (1), 122-8
- **VAN'T VEER L.J. et al.** Gene expression profiling predicts clinical outcome of breast cancer. *Nature,* 2002, vol. 415 (6871), 530-6
- **VIDAL-TABOADA JM et al.** High resolution physical mapping and identification of transcribed sequences in the Down syndrome region-2. *Biochem Biophys Res Commun,* 13 February 1998, vol. 243 (2), 572-8
- **WANG, Y. et al.** Gene-expression profiles to predict distant metastasis of lymph-node-negative primary breast cancer. *Lancet,* 2005, vol. 365 (9460), 671-9
- **WELLS RS et al.** Cw*1701 defines a divergent african HLA-C allelic lineage. *Immunogenetics,* 1997, vol. 46 (3), 173-80
- **XU XR et al.** Insight into hepatocellular carcinogenesis at transcriptome level by comparing gene expression profiles of hepatocellular carcinoma with those of corresponding noncancerous liver. *Proc Natl Acad Sci U S A.,* 18 December 2001, vol. 98 (26), 15089-94
- **XU, X. et al.** IFN-gamma induces cell growth inhibition by Fas-mediated apoptosis: requirement of STAT1 protein for up-regulation of Fas and FasL expression. *Cancer Res.,* 1998, vol. 58, 2832-2837
- **YOSHIKAWA T et al.** Multiple transcriptional variants and RNA editing in C18orf1, a novel gene with LDLRA and transmembrane domains on 18p11.2. *Genomics,* 15 January 1998, vol. 47 (2), 246-57
- **YOSHITAKE N et al.** Expression of SDF-1 alpha and nuclear CXCR4 predicts lymph node metastasis in colorectal cancer. *Br J Cancer,* 20 May 2008, vol. 98 (10), 1682-9